# EUROPEAN PATENT SPECIFICATION

(11) **EP 3 038 633 B1**
(45) Date of publication and mention of the grant of the patent: **14.10.2020**
(21) Application number: 14765883.5
(22) Date of filing: 28.08.2014
(51) Int. Cl.: A61K 38/00, C07K 14/47

(54) **STABLE POLYPEPTIDES BINDING TO HUMAN COMPLEMENT C5**
AN HUMANES KOMPLEMENT C5 BINDENDE STABILE POLYPEPTIDE
POLYPEPTIDES STABLES SE LIANT AU C5 DU COMPLÉMENT HUMAIN

(30) Priority: 28.08.2013 SE 1350986
(43) Date of publication of application: 06.07.2016
(73) Proprietor: IPC Research, LLC, New Castle, Delaware 19808-1674 (US)
(72) Inventor: NILSSON, Joakim, 182 52 Danderyd (SE); NORDLING, Erik, 182 35 Danderyd (SE); STRÖMBERG, Patrik, 191 34 Sollentuna (SE)
(74) Representative: Soames, Candida Jane
(86) International application number: PCT/EP2014/068282
(87) International publication number: WO 2015/028558

(56) References cited:
- WO-A1-2005/075507
- WO-A1-2009/077175
- WO-A1-2013/126006
- WO-A2-2012/004384
- US-A1- 2005 090 448
- STROMBERG PATRIK ET AL: "Development of Affibody (R) C5 inhibitors for versatile and efficient therapeutic targeting of the terminal complement pathway", MOLECULAR IMMUNOLOGY, vol. 61, no. 2, Sp. Iss. SI, October 2014 (2014-10), page 256, XP002733197, & 25TH INTERNATIONAL COMPLEMENT WORKSHOP; RIO DE JANEIRO, BRAZIL; SEPTEMBER 14 -18, 2014 ISSN: 0161-5890

## Description

### TECHNICAL FIELD

The present invention relates to polypeptides that bind to human complement component 5 (C5) and to the use of such polypeptides in therapy.

### BACKGROUND ART

The complement protein C5 is a central component of the complement system; a key part of the innate immune system. The complement system is an intricate immune surveillance system with numerous tasks in tightly controlled, diverse processes. It functions as a first line host defense system against infection by other organisms, and also in discriminating healthy host tissues from cellular debris and apoptotic and necrotic cells. Furthermore, it is involved in clearance of immune complexes, regulation of the adaptive immune response, promotion of tissue regeneration, angiogenesis, mobilization of stem cells and development of the central nervous system (Woodruff et al. Mol Immunol 2011, 48 (14):1631-1642); Ricklin et al. Nat Immunol 2010, 11(9):785-795). Any trigger, for example erroneous or unrestricted activation or insufficient regulation, that disturbs the fine balance of complement activation and regulation may lead to pathologic conditions including self-attack of the host's cells leading to extensive tissue damage.

The complement system consists of about 30 proteins. There are three pathways to initiate complement; the classical pathway that employs C1q to recognize immune complexes on the surface of cells; the lectin pathway that is initiated when mannose-binding lectin (MBL) recognizes certain sugars; and the alternative pathway that is initiated spontaneously by hydrolysis of complement factor 3 (C3), a process suppressed by certain mammalian cell surface molecules not present on invading pathogens. The alternative pathway also acts as an amplification loop for the complement system. All three pathways converge at the level of C3. Cleavage of C3 into C3a and C3b leads to the formation of a convertase that in turn cleaves complement factor 5 (C5) into C5a and C5b. C5a is a very potent attractant of various immune cells while C5b oligomerizes with C6-9 to form a pore known as the membrane attack complex (MAC) or sometimes the terminal complement complex (TCC). Activation of the complement system leads to a number of mechanisms with the purpose of neutralizing the pathogen; formation of MAC on the surface of a cell such as an invading bacteria leads to lysis, deposition of C3 and C4 cleavage products C3b and C4b aids opsonization leading to phagocytosis of the pathogen by macrophages and anaphylatoxins such as C3a and C5a attracts monocytes and neutrophils to the site of activation, up-regulates surface markers leading to increased immunologic susceptibility and to the release of cytokines.

C5 is a 190-kDa glycoprotein comprised of 2 disulfide-linked polypeptide chains, alpha and beta, with a molecular mass of 115 and 75 kDa, respectively (Tack et al. Biochem 1979, 18:1490-1497). Haviland et al. (J Immun 1991, 146: 362-368) constructed the complete cDNA sequence of human complement pro-C5, which is predicted to encode a 1,676-amino acid pro-molecule that contains an 18-amino acid leader peptide and a 4-amino acid linker separating the beta and alpha chains (SEQ ID NO: 251). Since C5 is common to all pathways of complement activation, blocking C5 will stop the progression of the cascade regardless of the stimuli and thereby prevent the deleterious properties of terminal complement activation while leaving the immunoprotective and immunoregulatory functions of the proximal complement cascade intact.

The complement system's key role in the defense against pathogens in general makes it an interesting target for pharmaceutical intervention. This is emphasized by the fact that many mutations or impaired regulation of complement is involved in various diseases and conditions. These include increased susceptibility to auto-immune diseases such as systemic lupus erythematosis (SLE) where deposition of immune complexes triggers the classical pathway (Manderson et al. Annu Rev Immunol 2004, 22:431-456). In addition, mutations of the complement proteins C1-C5 often result in SLE or SLE like symptoms. Other autoimmune diseases with a strong involvement of the complement system are rheumatoid arthritis (RA) where immune complexes may activate complement in the RA joint, Sjögren's syndrome, dermatomyositis and other autoantibody driven diseases such as Guillain-Barré syndrome (GBS), Fisher syndrome (Kaida et al. J. Neuroimmun 2010, 223:5-12), different types of vasculitis, systemic sclerosis, anti-glomerular basement membrane (anti-GBM) and anti-phospholipid syndrome (APS) (Chen et al. J Autoimmun 2010, 34:J276-J286). Furthermore, complement inhibition have been proven effective in animal models of such different conditions as periodontitis (Abe et al. J Immunol 2012, 189:5442-5448), wound healing (Cazender et al. Clin Dev Immunol 2012, on-line publication), tumor growth (Markiewski et al. Nat Immunol 2008, 9:1225-1235) and diseases of the eye such as uveitis and age-related macular degeneration (AMD) (Copland et al. Clin Exp Immunol 2009, 159:303-314).

Antibodies targeted to human complement C5 are known from, e.g., WO 95/29697; WO 02/30985; and WO 2004/007553. Eculizumab (Soliris™) is a humanized monoclonal antibody directed against protein C5 and prevents cleavage of C5 into C5a and C5b. Eculizumab has been shown to be effective in treating paroxysmal nocturnal hemoglobinuria (PNH), a rare and sometimes life threatening disease of the blood characterized by intravascular hemolytic anemia, thrombophilia and bone marrow failure, and is approved for this indication. Eculizumab was also recently approved by the FDA for treatment of atypical hemolytic syndrome (aHUS), a rare but life threatening disease caused by loss of control of the alternative complement pathway leading to over-activation manifested as thrombotic microangiopathy (TMA) leading to constant risk of damage to vital organs such as kidney, heart and the brain. In aHUS, transplantation of the damaged organ only temporarily helps the patient as the liver continues to produce the mutated form of controlling protein (most often complement factor H or other proteins of the alternative pathway). A related disease with a transient acute pathophysiology is HUS caused by infection of Shiga toxin positive *E. coli* (STEC-HUS) and there are promising clinical data suggesting efficacy also for this condition (Lapeyraque et al, N Engl J Med 2011, 364:2561-2563). Finally, the C5 blocking antibody Eculizumab has proven efficacious in preventing antibody mediated rejection (AMR) in recipients of highly mismatched kidneys (Stegall, M. D. et al. Am J Transplant 2011, 11:2405-2413), and in treating autoimmune neuropathies such as neuromyelitis optica and myasthenia gravis (Pittock et al. Lancet Neurol 2013, 12:554-562; Howard et al. Muscle Nerve 2013, 48:76-84).

Apart from full length antibodies, single-chain variable fragments (scFV), minibodies and aptamers targeting C5 are described in literature. These C5 inhibitors may bind to different sites (epitopes) on the C5 molecule and may have different modes of action. For example, whereas Eculizumab interacts with C5 at some distance of the convertase cleavage site, the minibody Mubodina® interacts with the cleavage site of C5. The C5 inhibitory protein *Ornithodoros moubata* Complement Inhibitor (OmCI, Nunn, M. A. et al. J Immunol 2005, 174:2084-2091) from soft tick *Ornithodoros moubata* has been hypothesized to bind to the distal end of the CUB-C5d-MG8 superdomain, which is close to the convertase cleavage site (Fredslund et al. Nat Immunol 2008, 9 (7):753-760). In contrast to the three proteins mentioned above inhibiting cleavage of C5, the monoclonal antibody TNX-558 binds to a C5a epitope present both on intact C5 and released C5a without inhibiting the cleavage of C5. (Fung et al. Clin Exp Immunol 2003, 133 (2):160-169).

C5 binding polypeptides, comprising a C5 binding motif, are disclosed in the International Patent Application No. PCT/SE2013/050139, published as WO 2013/126006. In particular, WO 2013/126006 discloses a C5 binding motif, *BM,* consisting of the amino acid sequence
EX₂X₃X₄A X₆X₇EID X₁₁LPNL X₁₆X₁₇X₁₈QW X₂₁AFIX₂₅ X₂₆LX₂₈D,
wherein, independently of each other,
X₂ is selected from H, Q, S, T and V;
X₃ is selected from I, L, M and V;
X₄ is selected from A, D, E, H, K, L, N, Q, R, S, T and Y;
X₆ is selected from N and W;
X₇ is selected from A, D, E, H, N, Q, R, S and T;
X₁₁ is selected from A, E, G, H, K, L, Q, R, S, T and Y;
X₁₆ is selected from N and T;
X₁₇ is selected from I, L and V;
X₁₈ is selected from A, D, E, H, K, N, Q, R, S and T;
X₂₁ is selected from I, L and V;
X₂₅ is selected from D, E, G, H, N, S and T;
X₂₆ is selected from K and S; and
X₂₈ is selected from A, D, E, H, N, Q, S, T and Y.

Examples of specific C5 binding motifs, as previously disclosed in WO 2013/126006, are shown as SEQ ID NO: 1-248 in the present patent application.

It is known from WO 2013/126006 that additional peptides or polypeptides may improve stabilization of C5 binding polypeptides. One example of such a polypeptide is the albumin binding domain (ABD) shown as SEQ ID NO: 250 in the present description. Other examples of suitable albumin binding domains are disclosed in WO 2009/016043 and WO 2012/004384. An ABD-extended polypeptide binds to serum albumin *in vivo,* and benefits from its longer half-life, which increases the net half-life of the polypeptide itself (see e.g. WO 91/01743).

The continued provision of agents with comparable C5 blocking activity remains a matter of substantial interest within the field. In particular, there is a continued need for molecules that prevent the terminal complement cascade as well as the formation of the pro-inflammatory molecule C5a. Of great interest is also a provision of uses of such molecules in the treatment of disease.

### BRIEF DESCRIPTION OF THE DRAWINGS

Figure 1 illustrates an SDS-PAGE gel wherein the bands represent the C5 binding compound PSI0242 (SEQ ID NO: 249) (0) prior to stability test; and (2w) after 2 weeks stability test.
Figure 2 is a chromatogram from reversed phase HPLC of PSI0242 (SEQ ID NO: 249) prior to stability test (solid line) and after 2 weeks stability test (dotted line).
Figure 3 illustrates an SDS-PAGE gel wherein the first lane contains SeeBlue 2P size marker and the bands represent (0) the initial samples; and (2w) the samples after 2 weeks stability test. Fig. 3A: SEQ ID NO: 249; Fig. 3B: SEQ ID NO: 261; Fig. 3C: SEQ ID NO: 262; Fig. 3D: SEQ ID NO: 264.
Figure 4 is a chromatogram from reversed phase HPLC of a C5 binding compound (SEQ ID NO: 253) prior to stability test (solid line) and after 2 weeks stability test (dotted line).
Figure 5 is a chromatogram from reversed phase HPLC of a C5 binding compound (SEQ ID NO: 264) prior to stability test (solid line) and after 2 weeks stability test (dotted line).

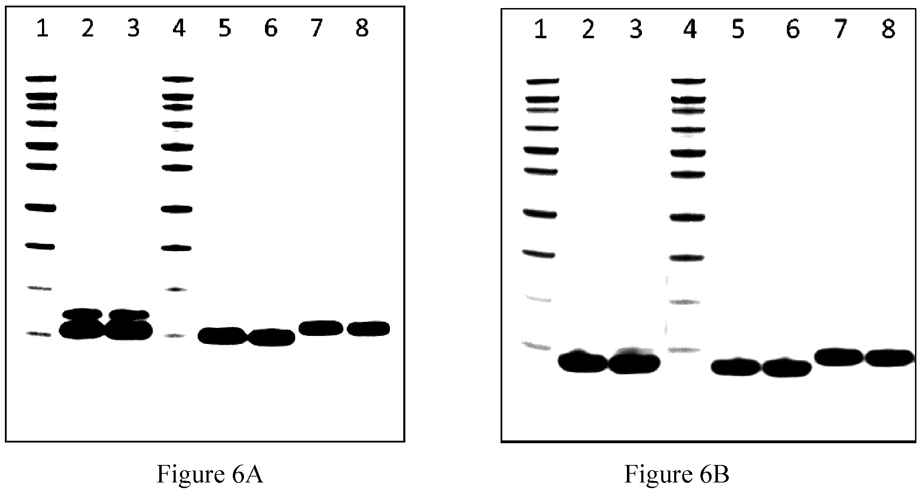

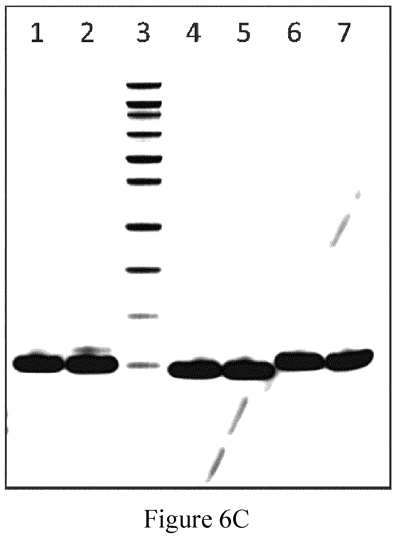

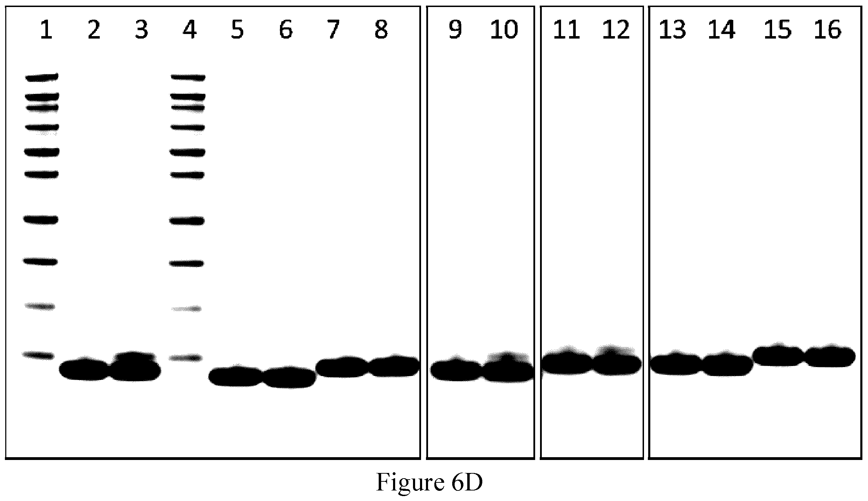

Figure 6A-D show images of SDS-PAGE gels comparing original and modified polypeptide variants (0) before and (2w) after 2 weeks stability test. The molecular size marker (Mw) was Novex® Sharp Pre-stained Protein Standard (216, 160, 110, 80, 60, 50, 40, 30, 20, 15, 10, 3.5 kDa). Fig. 6A shows a gel of HER2 binding polypeptides wherein the lanes show lane 1: Mw, lane 2: (0) Z02891 (SEQ ID NO: 272), lane 3: (2w) Z02891 (SEQ ID NO: 272), lane 4: Mw, lane 5: (0) Z17341 (SEQ ID NO: 273), lane 6: (2w) Z17341 (SEQ ID NO: 273), lane 7: (0) Z17342 (SEQ ID NO: 274), lane 8: (2w) Z17342 (SEQ ID NO: 274). Fig. 6B is a gel of PDGF-Rβ binding polypeptides wherein the lanes show: lane 1: Mw, lane 2: (0) Z15805 (SEQ ID NO: 275), lane 3: (2w) Z15805 (SEQ ID NO: 275), lane 4: Mw, lane 5: (0) Z17343 (SEQ ID NO: 276), lane 6: (2w) Z17343 (SEQ ID NO: 276), lane 7: (0) Z17344 (SEQ ID NO: 277), lane 8: (2w) Z17344 (SEQ ID NO: 277). Fig. 6C shows a gel of FcRn binding polypeptides wherein the lanes show: lane 1: (0) Z10103 (SEQ ID NO: 278), lane 2: (2w) Z10103 (SEQ ID NO: 278), lane 3: Mw, lane 4: (0) Z17347 (SEQ ID NO: 279), lane 5: (2w) Z17347 (SEQ ID NO: 279), lane 6: (0) Z17348 (SEQ ID NO: 280), lane 7: (2w) Z17348 (SEQ ID NO: 280). The diagonal bands seen in Figure 6C is an artefact resulting from an imprint from a second gel stained in the same container. Fig. 6D is a gel of CAIX binding polypeptides wherein the lanes show lane 1:Mw, lane 2: (0) Z09782 (SEQ ID NO: 281), lane 3: (2w) Z09782 (SEQ ID NO: 281), lane 4: Mw, lane 5: (0) Z17351 (SEQ ID NO: 282), lane 6: (2w) Z17351 (SEQ ID NO: 282), lane 7: (0) Z17352 (SEQ ID NO: 283), lane 8: (2w) Z17352 (SEQ ID NO: 283); lane 9: (0) Z17355 (SEQ ID NO: 284), lane 10: (2w) Z17355 (SEQ ID NO: 284), lane 11: (0) Z17357 (SEQ ID NO: 285), lane 12: (2w) Z17357 (SEQ ID NO: 285), lane 13: (0) Z17359 (SEQ ID NO: 286), lane 14: (2w) Z17359 (SEQ ID NO: 286), lane 15: (0) Z17360 (SEQ ID NO: 287), lane 16: (2w) Z17360 (SEQ ID NO: 287).
Figure 7 is a table showing the amino acid sequences of:
   - examples of C5 binding motifs (SEQ ID NO: 1-248);
   - the C5 binding compound designated PSI0242 (SEQ NO: 249);
   - an albumin binding domain (SEQ ID NO: 250);
   - the Swiss-Prot entry P01031 of human C5 (SEQ ID NO:251) wherein the α-chain corresponds to amino acid residues 678-1676 and the β-chain corresponds to amino acid residues 19-673;
   - examples of modified C5 binding polypeptides (SEQ ID NO: 260, 265-267).
   - examples of modified C5 binding compounds (SEQ ID NO: 252-259, 261-264, 268-270).
   - examples of polypeptide variants with binding affinity for other target molecules (SEQ ID NO: 272, 275, 278, 281)
   - examples of stability improved polypeptide variants with binding affinity for other targets with (SEQ ID NO: 273-274, 276-277, 279-280, 282-287).

### DISCLOSURE OF THE INVENTION

It has surprisingly been found that C5 binding polypeptides and compounds, wherein the amino acid sequences have been modified in specific positions, have improved stability when compared to previously known C5 binding polypeptides and compounds.

Consequently, this invention provides a polypeptide capable of binding human complement component 5 (C5), said polypeptide comprising the amino acid sequence:
[*BM*]-[L2]-QSX₄₂X₄₃LLX₄₆EAKKLX₅₂X₅₃X₅₄Q
wherein, independently of each other,
[*BM*] is a C5 binding motif comprising the amino acid sequence
EX₉X₁₀X₁₁A X₁₃X₁₄EIDX₁₈LPNLX₂₃X₂₄X₂₅QWX₂₈AFIX₃₂X₃₃LX₃₅;
wherein, independently of each other,
X₉ is selected from H, Q, S, T and V;
X₁₀ is selected from I, L, M and V;
X₁₁ is selected from A, D, E, H, K, L, N, Q, R, S, T and Y;
X₁₃ is selected from N and W;
X₁₄ is selected from A, D, E, H, N, Q, R, S and T;
X₁₈ is selected from A, E, G, H, K, L, Q, R, S, T and Y;
X₂₃ is selected from N and T;
X₂₄ is selected from I, L and V;
X₂₅ is selected from A, D, E, H, K, N, Q, R, S and T;
X₂₈ is selected from I, L and V;
X₃₂ is selected from D, E, G, H, N, S and T;
X₃₃ is selected from K and S; and
X₃₅ is selected from A, D, E, H, N, Q, S, T and Y;
[L2] is selected from DDPS and RQPE;
X₄₂ is selected from A and S;
X₄₃ is selected from N and E;
X₄₆ is selected from A, S and C;
X₅₂ is selected from E, N and S;
X₅₃ is selected from D, E and S, provided that X₅₃ is not D when X₅₂ is N; and
X₅₄ is selected from A and S.

The inventors have surprisingly found that modification or substitution of amino acid residue(s) in certain position(s) of the amino acid sequence of the C5 binding polypeptides as described in WO 2013/126006 improves stability of the C5 binding polypeptides while biological activity, such as binding affinity for human complement component 5 (C5) and inhibition of complement pathway function, is essentially retained. Thus, the biological activity of the modified C5 binding polypeptides is comparable to the biological activity of the known C5 binding polypeptides. Stability testing of the C5 binding polypeptides of the present invention demonstrates that substitution in either X₅₂, from N to E or S, or X₅₃, from D to E or S, improves stability. It has moreover been found that specific amino acid substitution in [L2] independently may promote stability.

The terms "C5 binding" and "binding affinity for C5" as used in this specification refers to a property of a polypeptide which may be tested for example by the use of surface plasmon resonance technology, such as in a Biacore instrument (GE Healthcare). C5 binding affinity may e.g. be tested in an experiment in which C5 is immobilized on a sensor chip of a Biacore instrument, and the sample containing the polypeptide to be tested is passed over the chip. Alternatively, the polypeptide to be tested is immobilized on a sensor chip of the instrument, and a sample containing C5, or fragment thereof, is passed over the chip. The skilled person may then interpret the results obtained by such experiments to establish at least a qualitative measure of the binding of the polypeptide to C5. If a quantitative measure is desired, for example to determine the apparent equilibrium dissociation constant K_{D} for the interaction, surface plasmon resonance methods may also be used. Binding values may for example be defined in a Biacore 2000 instrument (GE Healthcare). C5 is immobilized on a sensor chip of the measurement, and samples of the polypeptide whose affinity is to be determined are prepared by serial dilution and injected over the chip. K_{D} values may then be calculated from the results using for example the 1:1 Langmuir binding model of the BIAevaluation software provided by the instrument manufacturer. The C5 or fragment thereof used in the K_{D} determination may for example comprise the amino acid sequence represented by SEQ ID NO: 251. Examples of how C5 binding affinity may be tested are given herein, see Example 3 and 5.

In a preferred form of the invention, said polypeptide comprises the amino acid sequence:
AEAKYAK-[*BM*]-[L2]-QSX₄₂X₄₃LLX₄₆EAKKLX₅₂X₅₃X₅₄QAP
wherein, independently of each other,
[*BM*], [L2], X₄₂, X₄₃, X₄₆, X₅₂, X₅₃ and X₅₄ are as defined above.

As previously disclosed in WO 2013/126006, the C5 binding polypeptide according to the invention may form part of a three-helix bundle protein domain. Said C5 binding motif *[BM]* essentially may form part of two alpha helices, with an interconnecting loop, within said three-helix bundle. A second interconnecting loop, referred to herein as [L2], connects the C5 binding motif to the third alpha helix, referred to as the "Backbone".

In one embodiment, the C5 binding motif *[BM]* is essentially as disclosed in WO 2013/126006. However, according to the present invention the C5 binding motif preferably consists of 28, rather than 29, amino acids, and may in addition carry further amino acid substitutions.

In a preferred embodiment, the C5 binding motif *[BM]* is essentially as disclosed in WO 2013/126006. Said *[BM]* is accordingly a polypeptide comprising the amino acid sequence:
EX₉X₁₀X₁₁A X₁₃X₁₄EIDX₁₈LPNLX₂₃X₂₄X₂₅QWX₂₈AFIX₃₂X₃₃LX₃₅;
wherein, independently of each other,
X₉ is selected from H, Q, S, T and V;
X₁₀ is selected from I, L, M and V;
X₁₁ is selected from A, D, E, H, K, L, N, Q, R, S, T and Y;
X₁₃ is selected from N and W;
X₁₄ is selected from A, D, E, H, N, Q, R, S and T;
X₁₈ is selected from A, E, G, H, K, L, Q, R, S, T and Y;
X₂₃ is selected from N and T;
X₂₄ is selected from I, L and V;
X₂₅ is selected from A, D, E, H, K, N, Q, R, S and T;
X₂₈ is selected from I, L and V;
X₃₂ is selected from D, E, G, H, N, S and T;
X₃₃ is selected from K and S;
X₃₅ is selected from A, D, E, H, N, Q, S, T and Y.

In a further preferred aspect, [*BM*] comprises or consists of an amino acid sequence selected from the group consisting of positions 1-28 in SEQ ID NOS: 1-248. More preferably, [*BM*] comprises or consists of the amino acid sequence shown as positions 1-28 in SEQ ID NO: 1.

In a further aspect, the C5 binding polypeptide according to the invention comprises the amino acid sequence: wherein, independently of each other,
X₉ is selected from H, Q, S, T and V;
X₁₀ is selected from I, L, M and V;
X₁₁ is selected from A, D, E, H, K, L, N, Q, R, S, T and Y;
X₁₃ is selected from N and W;
X₁₄ is selected from A, D, E, H, N, Q, R, S and T;
X₁₈ is selected from A, E, G, H, K, L, Q, R, S, T and Y;
X₂₃ is selected from N and T;
X₂₄ is selected from I, L and V;
X₂₅ is selected from A, D, E, H, K, N, Q, R, S and T;
X₂₈ is selected from I, L and V;
X₃₂ is selected from D, E, G, H, N, S and T;
X₃₃ is selected from K and S;
X₃₅ is selected from A, D, E, H, N, Q, S, T and Y;
[L2] is selected from DDPS and RQPE;
X₄₂ is selected from A and S;
X₄₃ is selected from N and E;
X₄₆ is selected from A, S and C;
X₅₂ is selected from E, N and S;
X₅₃ is selected from D, E and S, provided that X₅₃ is not D when X₅₂ is N; and
X₅₄ is selected from A and S.

In preferred forms of the invention, at least one of the following eighteen, optionally nineteen, conditions is fulfilled:
X₉ is V,
X₁₀ is L,
X₁₁ is E,
X₁₃ is W,
X₁₄ is D,
optionally X₁₇ is D,
X₁₈ is R,
X₂₃ is T,
X₂₄ is I,
X₂₅ is E,
X₂₈ is L,
X₃₂ is N,
X₃₃ is K,
X₃₅ is D,
[L2] is DDPS,
X₄₂ is S,
X₄₃ is E,
X₄₆ is S,
X₅₄ is S.

More preferably, at least two, three, four, five, six, seven, eight, nine, ten, eleven, twelve, thirteen, fourteen, fifteen, sixteen, seventeen, eighteen or nineteen of the above conditions are fulfilled.

In an embodiment, X₅₂ and X₅₃ are independently selected from E and S. Preferably, (a) X₅₂ is S and X₅₃ is E, or (b) X₅₂ is E and X₅₃ is S.

In an embodiment, X₅₂ is S and X₅₃ is D.

In another embodiment, X₅₂ is N and X₅₃ is E.

In a further aspect, the polypeptide according to the invention comprises the amino acid sequence shown as SEQ ID NO: 260, SEQ ID NO: 265, SEQ ID NO: 266, or SEQ ID NO: 267.

In a further aspect, there is provided a compound capable of binding C5, said compound comprising:
a. at least one C5 binding polypeptide as defined above;
b. at least one albumin binding domain of streptococcal protein G, or a derivative thereof; and
c. optionally, at least one linking moiety for linking said at least one albumin binding domain or derivative thereof to the C or N terminal of said at least one C5 binding polypeptide.

Preferably, said albumin binding domain comprises the amino acid sequence shown as SEQ ID NO: 250.

Preferably, said linking moiety is a peptide comprising the amino acid sequence KVX₆₀GS, wherein X₆₀ is selected from D, E and A. When X₆₀ is D, a preferred compound comprises or consists of the amino acid sequence shown as SEQ ID NO: 253. When X₆₀ is E, preferred compounds comprise or consist of the amino acid sequence shown as SEQ ID NO: 261, SEQ ID NO: 263, SEQ ID NO: 264, SEQ ID NO: 269 or SEQ ID NO: 270. When X₆₀ is A, a preferred compound comprises or consists of the amino acid sequence shown as SEQ ID NO: 262. In the above listed amino acid sequences of the C5 binding compounds, amino acid residues 1-57 represent the amino acid sequence of a C5 binding polypeptide, residues 58-62 represent the amino acid sequence of a linker, and residues 63-108 represent the amino acid sequence of an albumin binding domain.

In an embodiment, the linking moiety is absent.

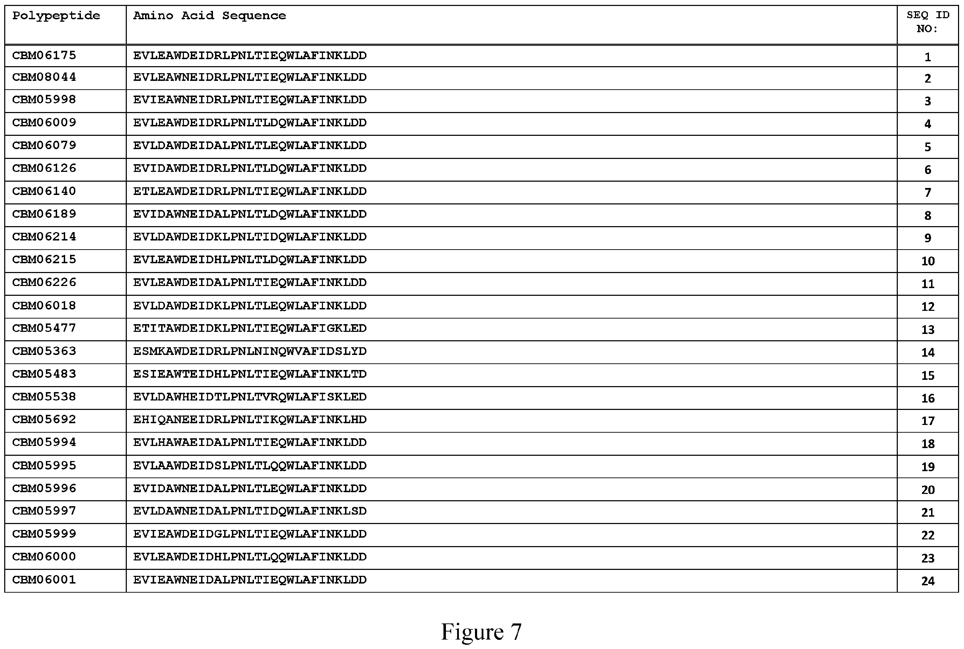

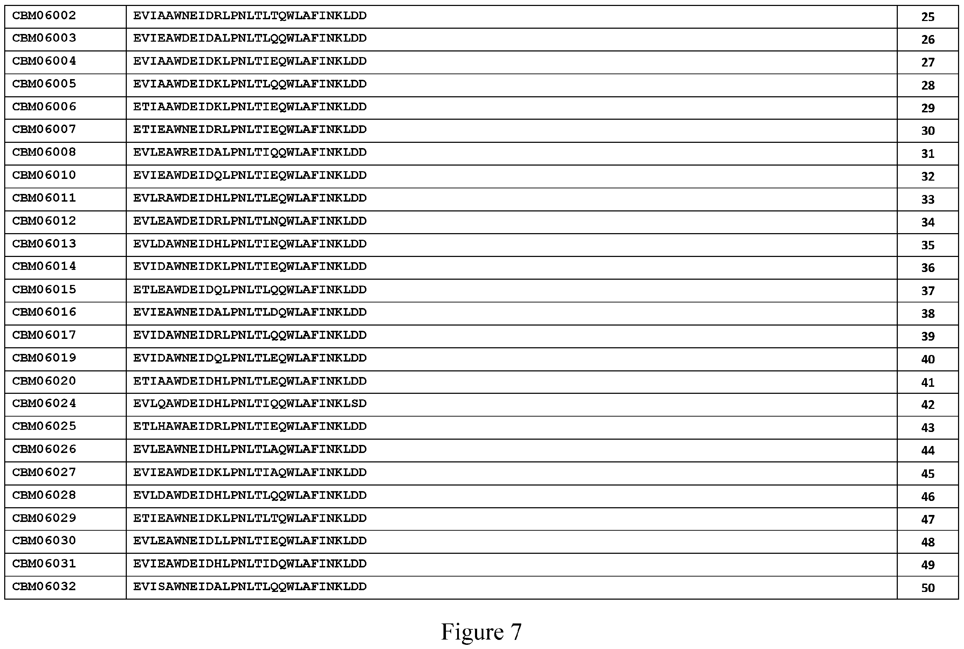

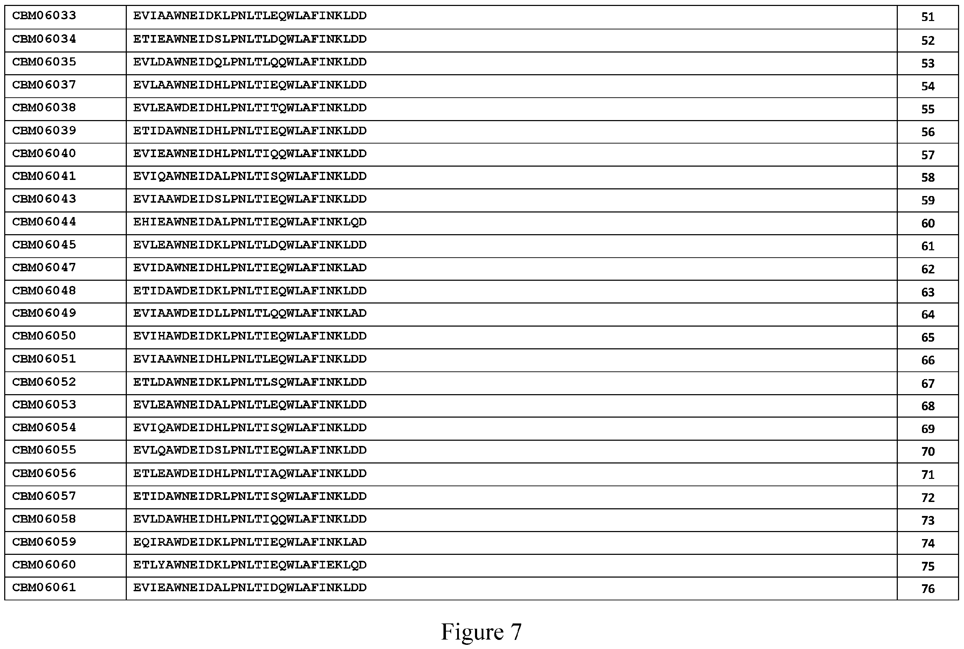

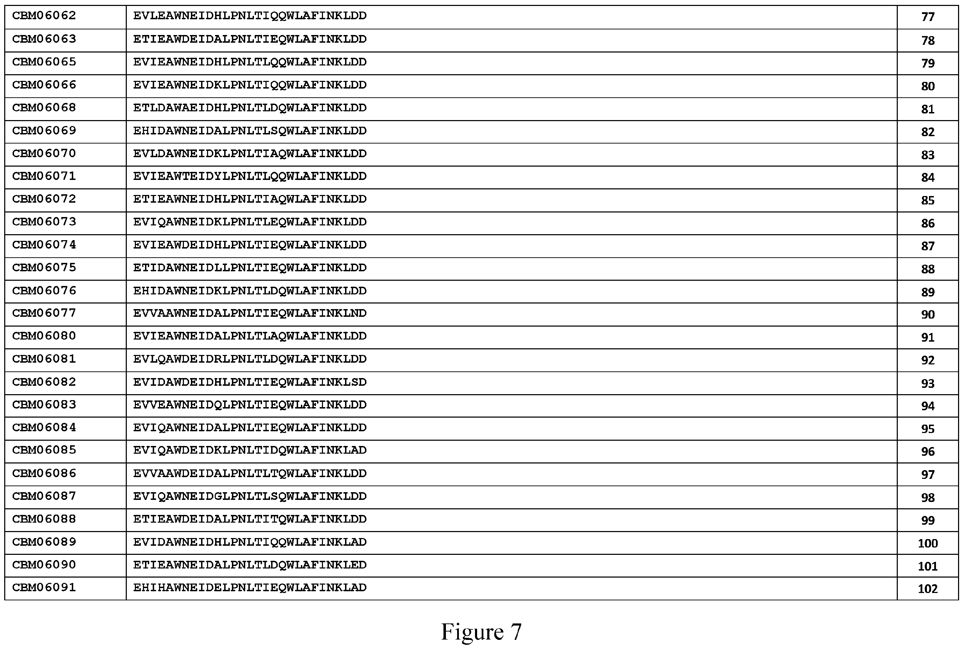

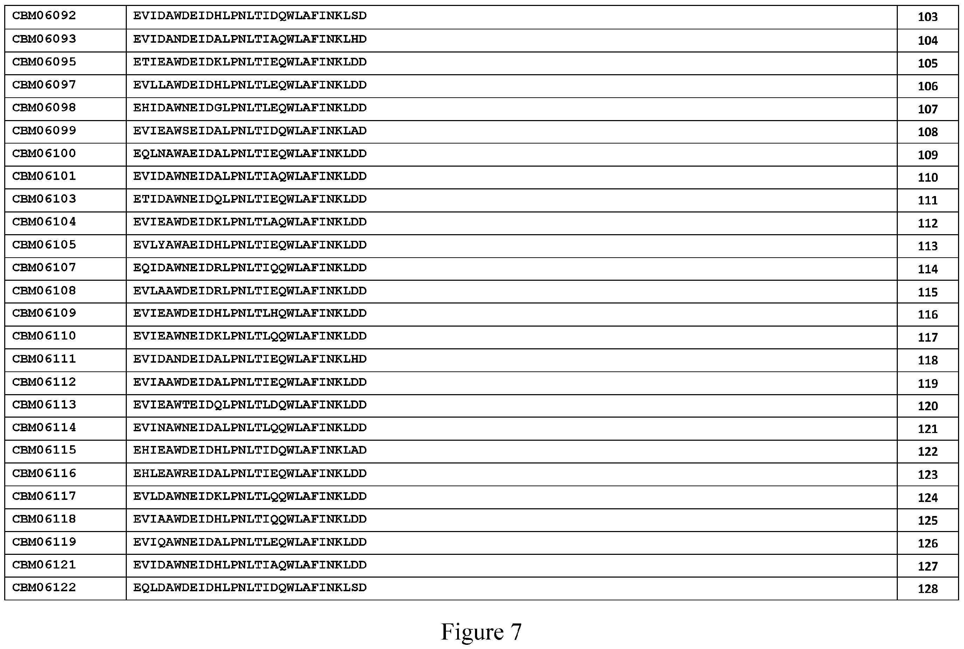

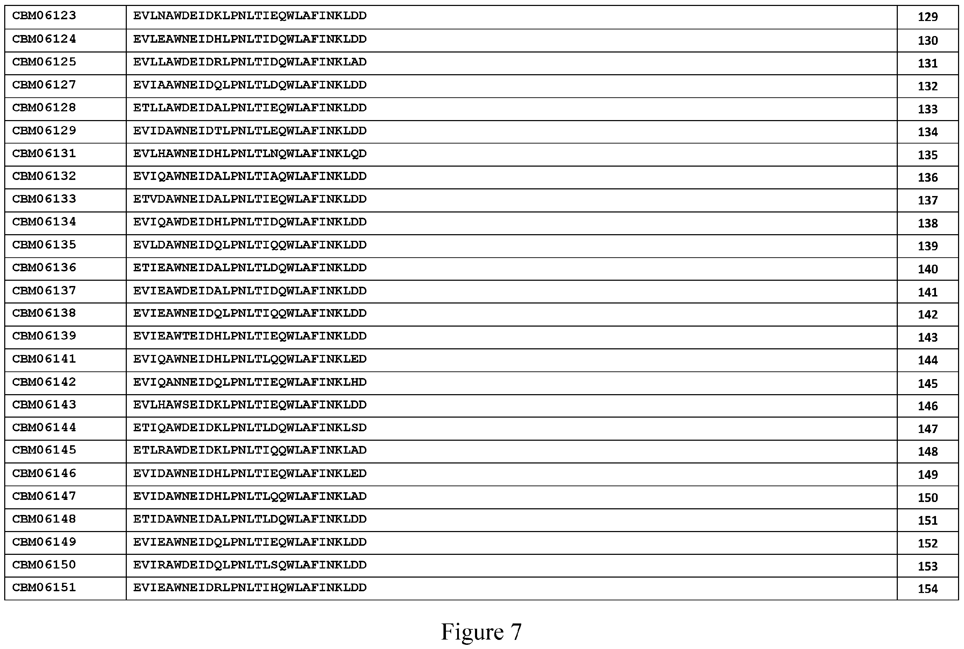

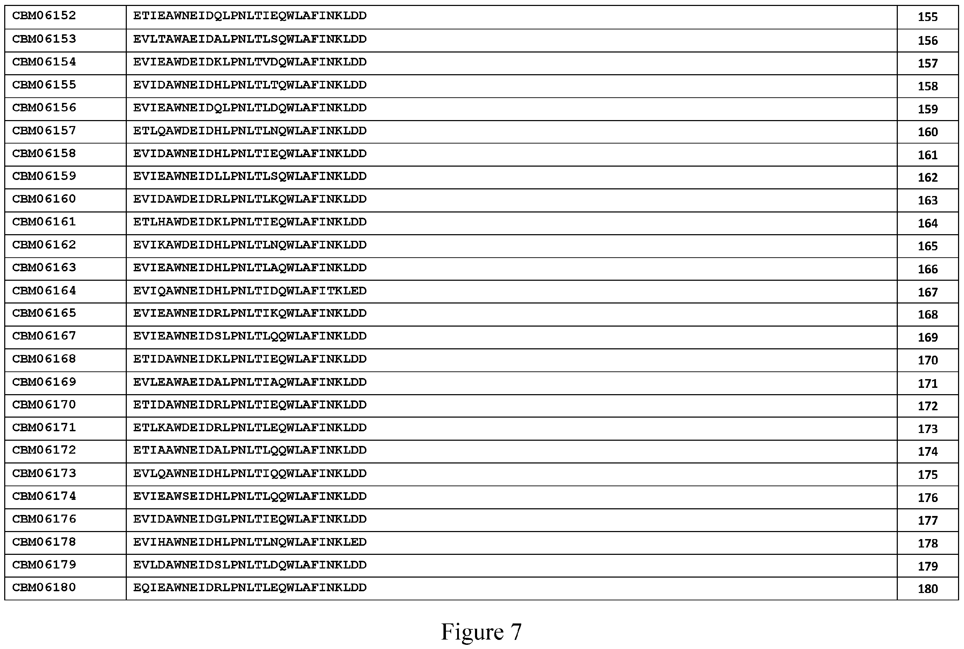

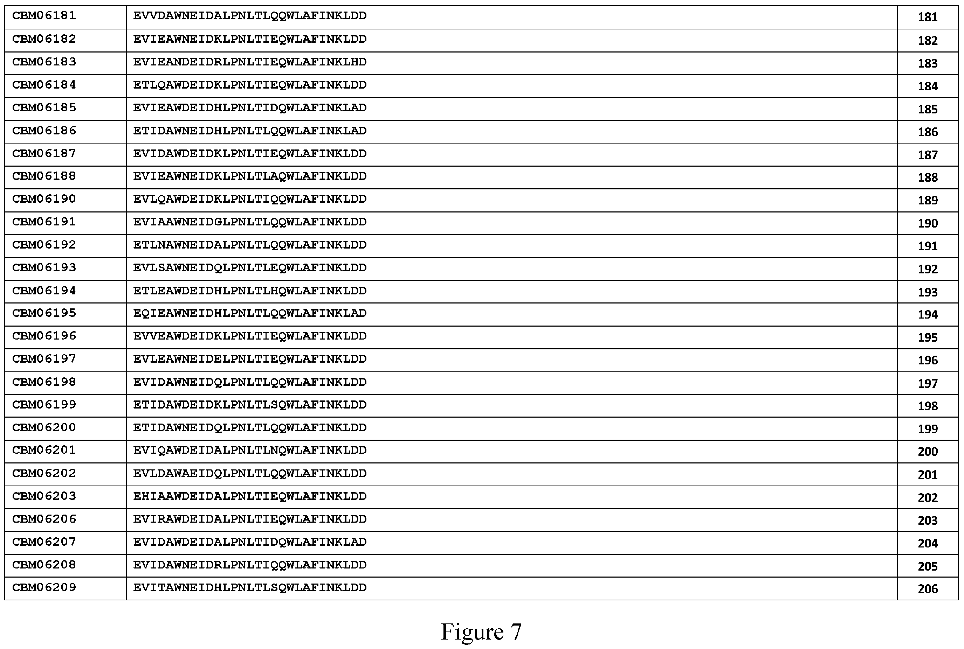

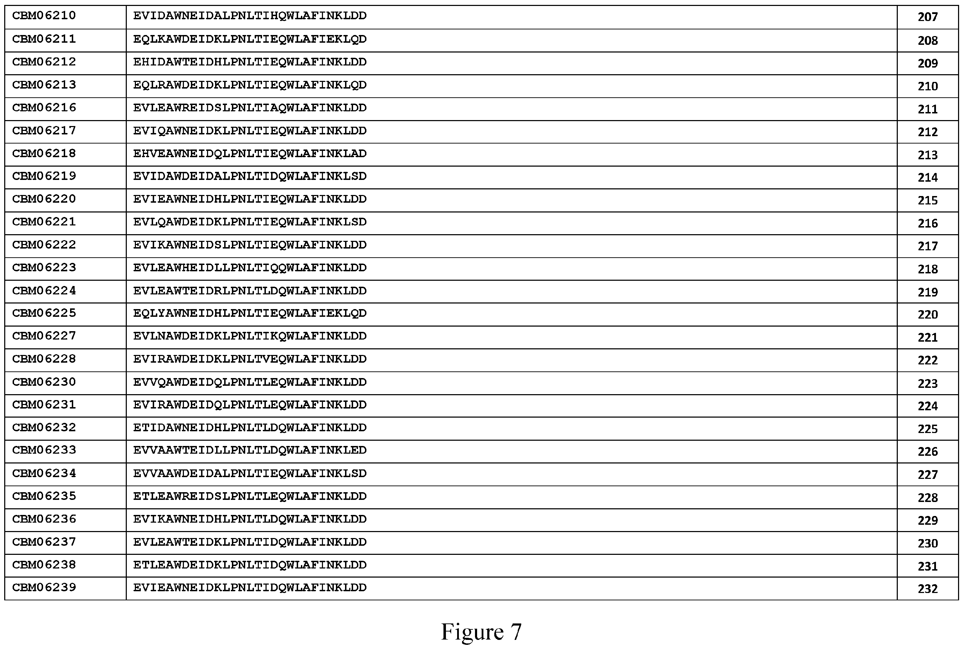

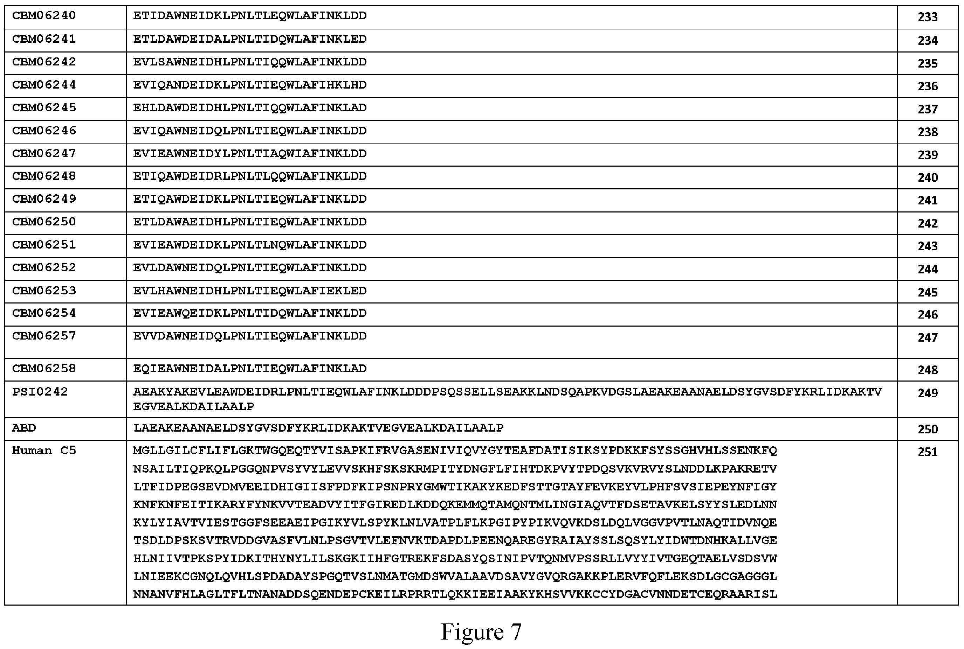

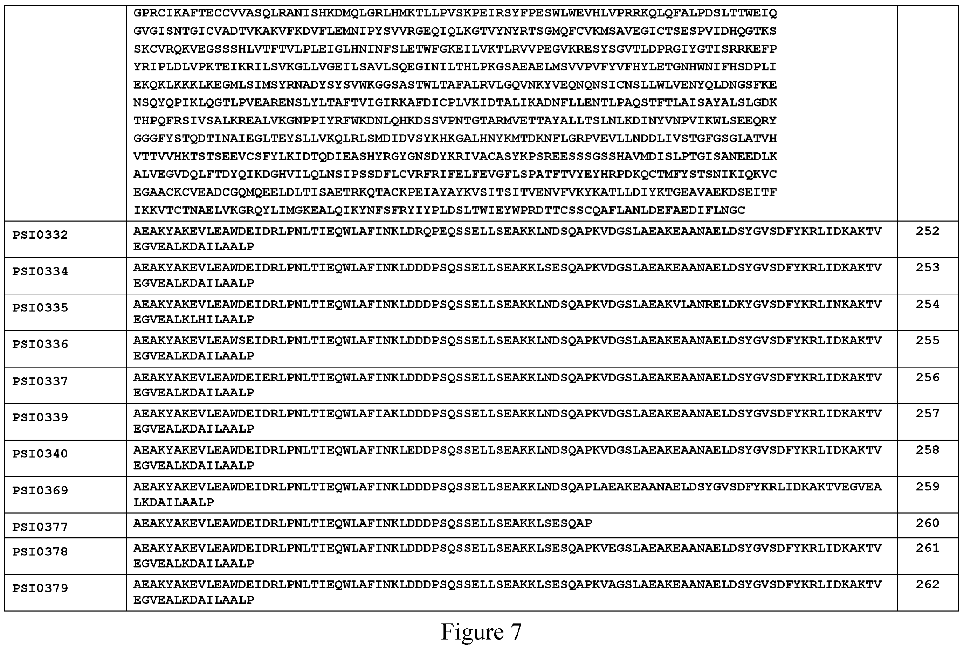

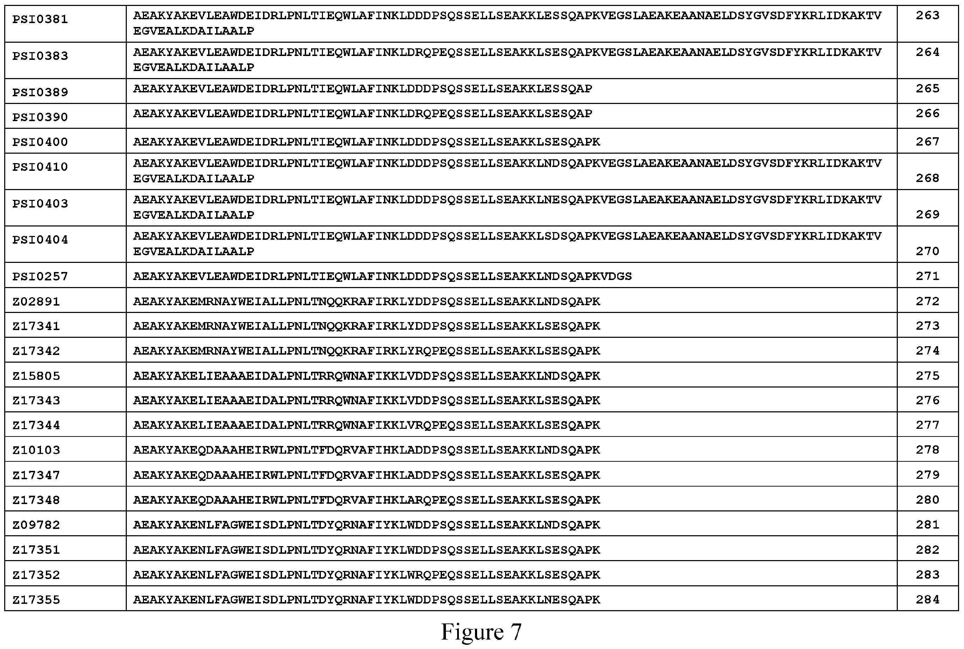

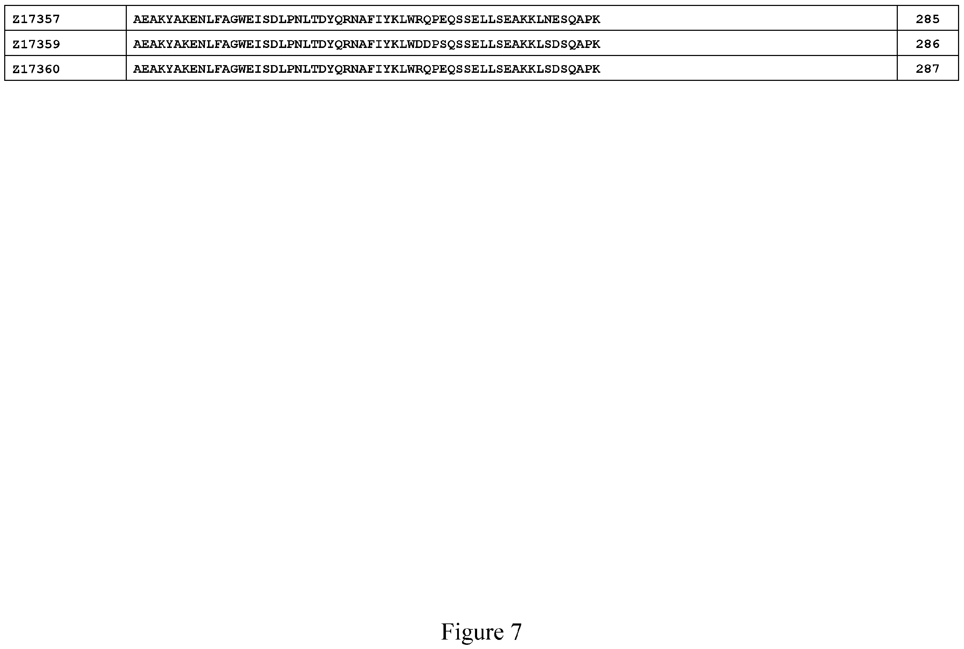

As discussed above, preferred C5 binding polypeptides according to the invention include those wherein X₅₂ and X₅₃ are independently selected from E and S. Specifically, compounds according to the invention can be derived from PSI0242 (SEQ ID NO: 249) but have modifications in at least one of positions 52, 53 and 60. For instance, as shown in Fig. 7 and the sequence listing, the preferred compound designated PSI0378 (SEQ ID NO: 261) carries the amino acid substitutions N52S, D53E and D60E; the preferred compound designated PSI0379 (SEQ ID NO: 262) carries the amino acid substitutions N52S, D53E and D60A; the preferred compound designated PSI0381 (SEQ ID NO: 263) carries the amino acid substitutions N52E, D53S and D60E; and the preferred compound designated PSI0383 (SEQ ID NO: 264) carries the amino acid substitutions N52S, D53E and D60E. Further, SEQ ID NO: 264 also carries substitutions in the loop [L2], namely D36R, D37Q and S39E. Moreover, the preferred compound designated PSI0403 (SEQ ID NO: 269) carries the amino acid substitutions D53E and D60E, and the preferred compound designated PSI0404 (SEQ ID NO: 270) carries the amino acid substitutions N52S and D60E.

As accounted for above, the inventors have surprisingly found that amino acid substitutions in certain positions of the amino acid sequence of the C5 binding polypeptides as described in WO 2013/126006 may improve stability. Such substitutions improve stability of the C5 binding compounds while biological activity, such as C5 binding capability and inhibition of hemolysis *in vitro,* is retained. Stability testing of the C5 binding compounds of the present invention demonstrate that for instance each of N52S (X₅₂) and D53E (X₅₃) (SEQ ID NO: 253) individually, as well as removal of D60 (X₆₀) (SEQ ID NO: 259 lacking linking moiety) improves stability. The combination of the substitutions N52S, D53E and D60E or D60A further improves the stability (SEQ ID NO: 261 and SEQ ID NO: 262). Each of the combined substitutions of N52S and D60E (SEQ ID NO: 270) and D53E and D60E (SEQ ID NO: 269) has similarly been found to improve stability. This indicates that each of the listed amino acid substitutions is involved in improving the stability of the polypeptide, and thus that each of these substitutions will provide further stabilized C5 binding polypeptides and compounds compared to previously known C5 binding polypeptides and compounds.

However, the skilled person will be able to identify polypeptides and/or compounds which have modifications in at least one of positions 52, 53 and 60, and/or in the loop [L2], but which also carry additional modifications like substitutions, small deletions, insertions or inversions, and nevertheless have substantially the disclosed biological activities and improved stability. Further, a C5 binding polypeptide and/or compound according to the invention could comprise further C terminal and/or N terminal amino acids that improve production, purification, stabilization *in vivo* or *in vitro,* coupling, or detection of the polypeptide.

It has been found that removal of D60 or an amino acid substitution in position 60 of SEQ ID NO: 249 alone improves stability of the C5 binding compounds of the invention compared to previously known C5 binding compounds. Preferably, the linking moiety is KVEGS (X₆₀=E) while X₅₂X₅₃ may be ND, and an example of a preferred compound carrying such a linking moiety is PSI0410 (SEQ ID NO: 268). In another preferred embodiment, D60 and the entire linking moiety is absent and an example of such a compound is the preferred compound designated PSI0369 (SEQ ID NO: 259).

Specific amino acid substitutions in the loop [L2] have been found to improve stability of the C5 binding compounds (e.g. SEQ ID NO: 252) of the invention compared to previously known C5 binding compounds.

Included in the invention are C5 binding polypeptides and compounds as described above, for use in therapy. In particular, the C5 binding polypeptides and compounds according to the invention are useful in methods for the treatment and/or prophylaxis of C5-related conditions, such as inflammatory diseases; autoimmune diseases; infectious diseases; cardiovascular diseases; neurodegenerative disorders; graft injury; eye diseases; kidney diseases; pulmonary diseases; haematological diseases such as paroxysmal nocturnal hemoglobinuria (PNH); allergic diseases and dermatological diseases.

In methods for treatment and/or prophylaxis, the said C5 binding polypeptide or compound can preferably be administered intravenously, subcutaneously, by inhalation, nasally, orally, intravitreally, or topically.

### EXAMPLES

### EXAMPLE 1: Stability test of known C5 inhibitor

The C5 binding compound designated PSI0242 (SEQ ID NO: 249) was formulated in 25 mM NaP /125 mM NaCl pH 7.0 and subjected to an accelerated stability study for 2 weeks at 37 °C. The stability was measured by the appearance of new variants after the stability testing by SDS-PAGE and Reversed Phase HPLC (RPC). In both analyses the initial sample and the one subjected to the stability study were run in parallel. For the SDS-PAGE, 7.5 µg protein was loaded into each well. The RPC was run on an Agilent 1100 HPLC using a Mobile Phase A consisting of 0.1 % trifluoroacetic acid (TFA) in water, and using a Mobile Phase B consisting of 0.1 % TFA / 45 % MeOH / 45 % isopropylamine (IPA) / 10 % water.

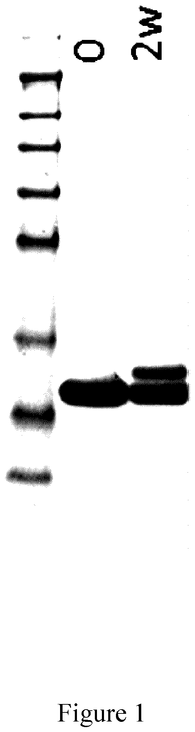

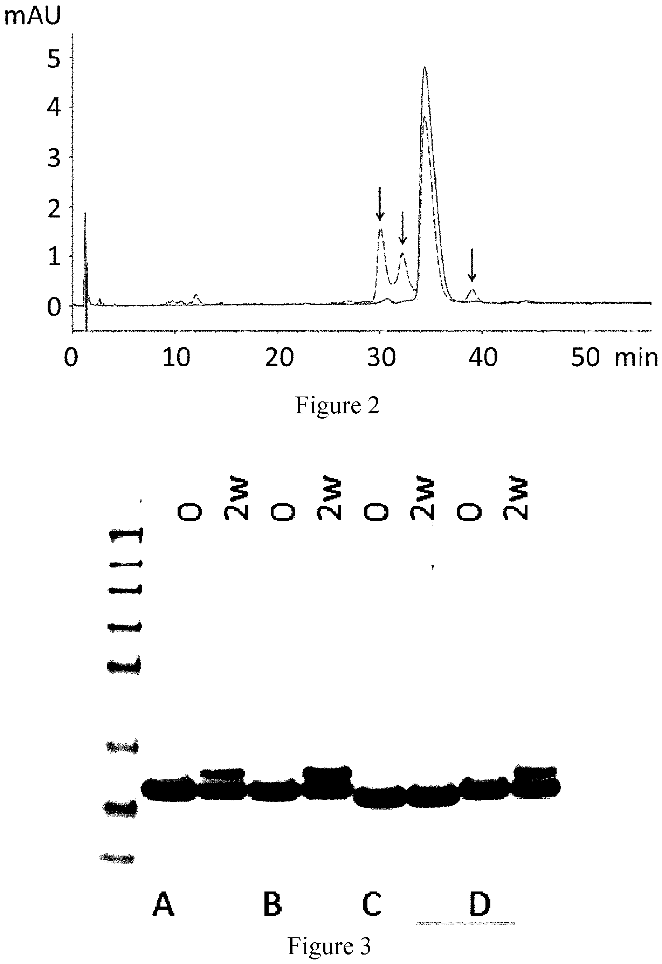

The results show that new forms of the protein are formed during incubation, these new forms visualized as bands in SDS-PAGE (Fig.1) and as new peaks in Reversed Phase HPLC (RPC) chromatograms (Fig. 2). In Fig. 2, the main peak after 2 weeks incubation corresponds to 57 % of the original protein sample.

Positions 1-60 in SEQ ID NO: 249 correspond to the polypeptide Z06175a, previously disclosed in WO 2013/126006 as SEQ ID NO: 753. PSI0242 (SEQ ID NO: 249) was produced essentially as disclosed in WO 2013/126006.

### EXAMPLE 2: Stability of modified C5 binding polypeptides and compounds

Modified C5 binding polypeptides and compounds were synthesized and purified essentially as described in WO 2013/126006.

Briefly, DNA encoding C5 binding Z variants was *E. coli* codon optimized and synthesized by GeneArt, GmbH. The synthetic genes representing the C5 binding Z variants were subcloned and expressed in *E. coli.*

Intracellularly expressed Z variants were purified using conventional chromatography methods. Homogenization and clarification was performed by sonication followed by centrifugation and filtration. Anion exchange chromatography was used as capture step. Further purification was obtained by hydrophobic interaction chromatography. The purifications were executed at acidic conditions (pH 5.5). Polishing and buffer exchange was performed by size exclusion chromatography.

The purified proteins were formulated in 25 mM NaP /125 mM NaCl pH 7.0 and subjected to an accelerated stability study for 2 weeks at 37 °C. The stability was measured by the appearance of new variants after the stability testing by SDS-PAGE and Reversed Phase HPLC (RPC). In both analyses the initial sample and the one subjected to the stability study were run in parallel. For the SDS-PAGE, 7.5 µg protein was loaded into each well. An example of a resulting gel is shown in Fig. 3.

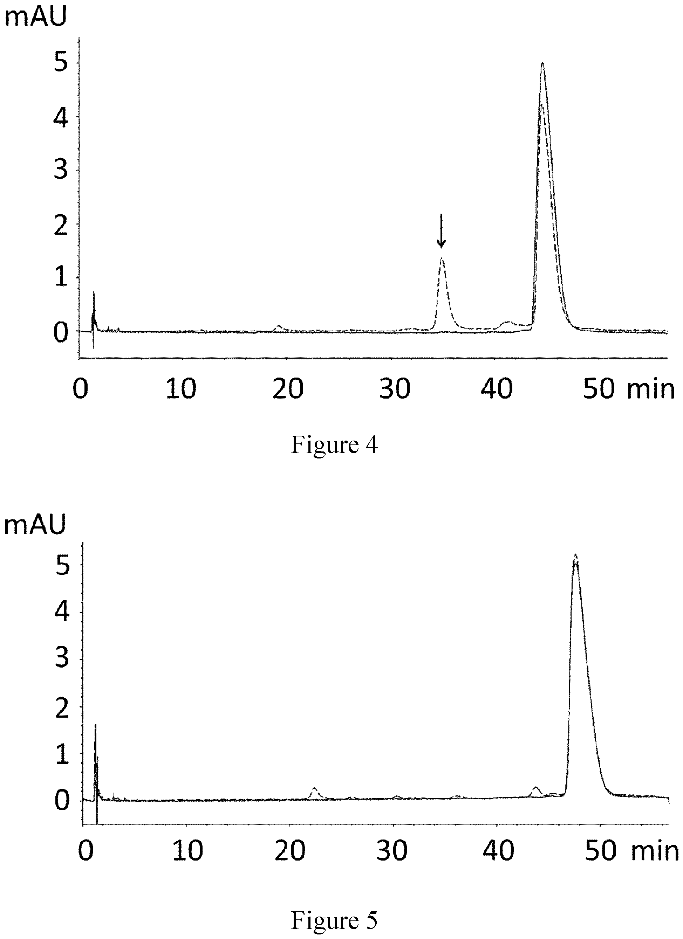

The RPC was run on an Agilent 1100 HPLC using a Mobile Phase A consisting of 0.1 % trifluoroacetic acid (TFA) in water, and a Mobile Phase B consisting of 0.1 % TFA / 45 % MeOH / 45 % isopropylamine (IPA) / 10 % water. An example of a resulting chromatogram is shown in Fig. 4 for SEQ ID NO: 253.

The results of the stability testing are summarized in Table I, below.

**TABLE I**

| SEQ ID NO: | Name | SDS-PAGE bands | RPC prepeaks | Main peak (% of total protein) | RPC post peaks |
|---|---|---|---|---|---|
| 249 | PSI0242 | 2 | 2 | 57 | 1 |
| 252 | PSI0332 | 2 | 1 | 57 | 1 |
| 253 | PSI0334 | 1 | 1 | 73 | 0 |
| 254 | PSI0335 | 2 | 2 | 57 | 1 |
| 255 | PSI0336 | 2 | 2 | 57 | 1 |
| 256 | PSI0337 | 2 | 2 | 57 | 1 |
| 257 | PSI0339 | 2 | 2 | 57 | 1 |
| 258 | PSI0340 | 2 | 2 | 67 | 1 |
| 259 | PSI0369 | 2 | 1 | 90 | 1 |
| 260 | PSI0377 | 1 | 0 | 77 | 0 |
| 261 | PSI0378 | 1 | 0 | 89 | 0 |
| 262 | PSI0379 | 1 | 0 | 88 | 0 |
| 263 | PSI0381 | 1 | 0 | 87 | 0 |
| 264 | PSI0383 | 1 | 0 | 91 | 0 |
| 267 | PSI0400 | 1 | 0 | 91 | 0 |
| 268 | PSI0410 | 1 | 1 | 72 | 1 |
| 269 | PSI0403 | 1 | 1 | 77 | 1 |
| 270 | PSI0404 | 1 | 1 | 88 | 0 |

It can be concluded from Table I that certain modified C5 binding polypeptides or compounds have improved properties, such as increased stability, when compared with PSI0242. Such improved C5 binding polypeptides or compounds include PSI0334 (SEQ ID NO: 253), PSI0340 (SEQ ID NO: 258), PSI0369 (SEQ ID NO: 259), PSI0377 (SEQ ID NO: 260), PSI0378 (SEQ ID NO: 261), PSI0379 (SEQ ID NO: 262), PSI0381 (SEQ ID NO: 263), PSI0383 (SEQ ID NO: 264), PSI0400 (SEQ ID NO: 267), PSI0410 (SEQ ID NO: 268), PSI0403 (SEQ ID NO: 269) and PSI0404 (SEQ ID NO: 270). In six of the mentioned variants (SEQ ID NO: 253, 260, 261, 262, 264 and 267), the amino acid residues in positions 52-53 have been substituted from ND (cf PSI0242) to SE. In SEQ ID NO: 263, the corresponding substitution is from ND to ES. In SEQ ID NO: 269 only the amino acid residue in position 53 has been substituted from D to E, while in SEQ ID NO: 270 the amino acid residue in position 52 has been substituted from N to S.

Further, PSI0378 (SEQ ID NO: 261), PSI0381 (SEQ ID NO: 263), PSI0383 (SEQ ID NO: 264), PSI0410 (SEQ ID NO: 268), PSI0403 (SEQ ID NO: 269) and PSI0404 (SEQ ID NO: 270) have in common an amino acid residue substitution from D to E in position 60.

The combined benefit of stability enhancing substitutions in position 52 or 53 and position 60 can be seen in Fig. 5, showing the chromatogram of PSI0383 (SEQ ID NO: 264). In PSI0379 (SEQ ID NO: 262) the substitution in position 60 is from D to A.

In PSI0369 (SEQ ID NO: 259) the linker moeity (including D60) is altogether removed, yielding a more stable C5 binding compound and indicating the influence of position 60 upon stability of the C5 binding compounds.

### EXAMPLE 3: Binding of modified compounds to human C5

Human serum albumin was immobilized to Amine Reactive 2^{nd} generation (AR2G) Dip and Read Biosensors (Pall Life sciences (ForteBio) Cat # 18-5092) by amine coupling. PSI0242 (SEQ ID NO: 249; 1 µM) and C5 binding compounds (1 µM) in read buffer (HBS-EP Buffer ready-to-use 200 ml, GE Healthcare #BR100188) were loaded, each onto a separate sensor with HSA, for 120 seconds followed by a base line recording for 60 seconds in read buffer before being subjected to human C5 (Quidel Cat # 403) in concentrations ranging from 0.79 nM to 25 nM in read buffer with a regeneration cycle and a base line recording between each concentration. Regeneration conditions for the sensors were 10 mM Glycine, pH 2 (three pulses with 30 seconds and running buffer for 60 seconds). Each spectrogram was reference subtracted against an analogous construct containing an albumin binding domain (SEQ ID NO: 250) but without the C5 binding capacity. The data were analyzed according to Langmuir 1:1 model using ForteBio Analysis 7.1 (Pall Life sciences (ForteBio) kinetics software).

The K_{D} of the interaction with C5 relative to PSI0242 (SEQ ID NO: 249) is shown in Table II. The K_{D} of PSI0242 varied from 1-3 nM in different runs.

The results in Table II indicate that C5 binding compounds according to the invention have a binding capacity to human C5 which is similar to that of the polypeptide PSI0242 (SEQ ID NO: 249) disclosed in WO 2013/126006.

**TABLE II**

| SEQ ID NO: | Name | Rel. K_{D} |
|---|---|---|
| 249 | PSI0242 | 1.0 |
| 253 | PSI0334 | 1.1 |
| 261 | PSI0378 | 1.3 |
| 263 | PSI0381 | 23 |
| 264 | PSI0383 | 2.1 |

### EXAMPLE 4: Stability of chemically synthesized C5 binding polypeptide

A chemically synthesized PSI0400 (SEQ ID NO: 267) was ordered from BACHEM AG. The stability of the polypeptide was tested according to the same methodology as in Example 2. The results of the stability testing are shown in Table III.

**TABLE III**

| SEQ ID NO: | Name | SDS-PAGE bands | RPC prepeaks | Main peak (% of total protein) | RPC post peaks |
|---|---|---|---|---|---|
| 267 | PSI0400 | 1 | 0 | 91 | 0 |

The stability of the PSI0400 was comparable to the polypeptides that were produced in *E.coli* in Example 2.

The integrity of the fold of PSI0400 (SEQ ID NO: 267) was compared to a recombinant C5 binding polypeptide (PSI0257, SEQ ID NO: 271), produced in accordance with the methods of Example 2, using far UV circular dichroism (CD) spectra.

The CD spectra were recorded by a J-720 CD spectropolarimeter (Jasco, Japan). The samples were diluted to 0.17 mg/ml protein concentration using Pi buffer (5 mM Na-K-PO₄, pH 7.0). A CD spectrum of Pi buffer was firstly recorded, then spectra were recorded for each of the samples and lastly for the Pi buffer again. As the two buffer spectra coincide, the firstly recorded spectrum was used as the buffer spectrum. The buffer spectrum was smoothened using the Savitzky-Golay procedure with convolution width of 25. The other spectra were smoothened according to the same procedure with a convolution width of 15. The smoothened buffer spectrum was then subtracted from each of the other smoothened spectra. The CDNN program was used to estimate the secondary structure content of the proteins and the resulting estimations are presented in Table IV. The results showed that neither the two amino acid substitutions at position 52 and 53 nor the polypeptide production by chemical synthesis influence the secondary structure content of the chemically synthesized polypeptide. The integrity of the secondary structure content was compared to the recombinantly produced PSI0257 (SEQ ID NO: 271).

**TABLE IV**

| | SEQ ID NO: 271 | SEQ ID NO: 267 |
|---|---|---|
| Helix | 63 % | 69% |
| Antiparallel | 3 % | 2% |
| Parallel | 3 % | 3% |
| Beta- Turn | 13 % | 12 % |
| Rndm. Coil | 13 % | 11 % |

### EXAMPLE 5: Binding of modified compounds and polypeptides to human C5

The binding affinity of the C5 binding compounds PSI0242 (SEQ ID NO: 249), PSI0340 (SEQ ID NO: 258), PSI0378 (SEQ ID NO: 261), and PSI0410 (SEQ ID NO: 268) and the C5 binding polypeptide PSI0400 (SEQ ID NO: 267) for human C5 was analyzed using a Biacore T200 instrument (GE Healthcare). Human C5 (A403, Quidel Corporation) was coupled to a CM5 sensor chip (900 RU) using amine coupling chemistry according to the manufacturer's protocol. The coupling was performed by injecting hC5 at a concentration of 7.5 µg/mL in 10 mM Na-acetate buffer pH=5 (GE Healthcare). The reference cell was treated with the same reagents but without injecting human C5. Binding of the C5 binders to immobilized hC5 was studied with the single cycle kinetics method, in which five concentrations of sample, typically 25, 12.5, 6.25, 3.12 and 1.56 nM in HBS-EP buffer (10 mM HEPES pH 7.4, 150 mM NaCl, 3 mM EDTA, 0.005% Surfactant P20, GE Healthcare) were injected one after the other at a flow rate of 30 µL/min at 25°C in the same cycle without regeneration between injections. Data from the reference cell were subtracted to compensate for bulk refractive index changes. In most cases, an injection of HBS-EP was also included as control so that the sensorgrams were double blanked. The surfaces were regenerated in HBS-EP buffer. Kinetic constants were calculated from the sensorgrams using the Langmuir 1:1 analyte model of the Biacore T200 Evaluation Software version 1.0.The resulting K_{D} values of the interactions are tabulated in the Table V.

**TABLE V**

| SEQ ID NO: | Name | K_{D} (nM) |
|---|---|---|
| 249 | PSI0242 | 1.3 |
| 258 | PSI0340 | 2.5 |
| 261 | PSI0378 | 2.1 |
| 267 | PSI0400 | 0.53 |
| 268 | PSI0410 | 1.3 |

The stability enhancing amino acid substitutions are not detrimental for the ability of the molecules to bind to C5 and thus do not influence their biological activities.

### EXAMPLE 6: Inhibition of hemolysis

For studies of classical complement pathway function and inhibition thereof by the C5 binding compounds PSI0378 (SEQ ID NO: 261) and PSI0410 (SEQ ID NO: 268), and C5 binding polypeptide PSI0400 (SEQ ID NO: 267), sheep erythrocytes were prepared from fresh sheep whole blood in Alsever's solution (Swedish National Veterinary Institute) and thereafter treated with rabbit anti-sheep erythrocyte antiserum (Sigma) to become antibody sensitized sheep erythrocyte (EA). The whole process was conducted under aseptic conditions. All other reagents were from commercial sources.

The *in vitro* assay was run in 96-well U-form microtiter plate by consecutive additions of a test protein, a complement serum and EA suspension. The final concentrations of all reagents, in a total reaction volume of 50 µL per well and at pH 7.3-7.4, were: 0.15 mM CaCl 2; 0.5 mM MgCl 2; 3 mM NaN 3; 138 mM NaCl; 0.1 % gelatin; 1.8 mM sodium barbital; 3.1 mM barbituric acid; 5 million EA; complement protein C5 serum at suitable dilution, and C5 binding compound or polypeptide at desired concentrations.

The C5 binding compounds and polypeptide were pre-incubated with the above described complement serum for 20 min on ice prior to starting the reaction by the addition of EA suspension. The hemolytic reaction was allowed to proceed at 37 °C during agitation for 45 min and was then optionally ended by addition of 100 µL ice-cold saline containing 0.02 % Tween 20. The cells were centrifuged to the bottom and the upper portion, corresponding to 100 µL supernatant, was transferred to a transparent microplate having half-area and flat-bottom wells. The reaction results were analyzed as optical density using a microtiter plate reader at a wavelength of 415 nm.

On all test occasions, a control sample (PSI0242, SEQ ID NO: 249) and vehicle were included in each plate to define values for uninhibited and fully inhibited reactions, respectively. These values were used to calculate the % inhibition of the complement hemolysis at any given sample concentration. The inhibitory potencies (IC 50-values) of tested C5 binding compounds and polypeptide were defined by applying the same assay in the presence of a controlled concentration of human C5 added to C5 depleted serum. For highly potent inhibitors (low nanomolar to sub-nanomolar), a final C5 concentration of the reaction mixture was controlled at 0.1 nM, which was optionally established by using C5 depleted or deficient sera. The results are presented below in Table VI.

**TABLE VI**

| SEQ ID NO: | Name | Potency (%) | IC 50 (nM) |
|---|---|---|---|
| 249 | PSI0242 | 100 | 0.47 |
| 261 | PSI0378 | 83 | 0.58 |
| 267 | PSI0400 | - | 4 |
| 268 | PSI0410 | 107 | 0.49 |

The results from the hemolysis assay show that the improved C5 binding compounds SEQ ID NO: 261 and 268 are comparable to the reference compound. The C5 binding polypeptide SEQ ID NO: 267 was functional in the assay but since it does not contain an albumin binding domain the results cannot be directly compared to the reference compound.

### EXAMPLE 7: Binding to human albumin

For assessment of C5 binding compounds binding affinity for albumin, a human albumin ELISA utilizing recombinant human albumin (coating) and commercially available antibodies (primary and detecting) purchased from Novozymes, Affibody AB and DakoCytomation, respectively, was used. A method standard prepared from PSI0242 (SEQ ID NO:249), comprising a C5 binding polypeptide and an albumin binding domain of streptococcal protein G, was used for quantification of samples. A 96-well microplate was coated with recombinant human albumin. The plate was then washed with phosphate buffered saline containing 0.05 % Tween 20 (PBST) and blocked for 1-2 hours with 1 % casein in PBS. After a plate wash, the standard, method controls, control sample and test samples are added to the plate. After incubation for 2 hours, unbound material was removed by a wash. A goat Anti-Affibody® IgG (Affibody AB, cat no. 20.1000.01.0005) was added to the wells and the plate was incubated for 1.5 hours to allow binding to the bound C5 binding compounds. After a wash, rabbit anti-goat IgG HRP was allowed to bind to the goat antibodies for 1 h. After a final wash, the amount of bound HRP was detected by addition of TMB substrate, which was converted to a blue product by the enzyme. Addition of 1 M hydrochloric acid after 30 minutes stopped the reaction and the color of the well contents changed from blue to yellow. The absorbance at 450 nm was measured photometrically, using the absorbance at 650 nm as a reference wavelength. The color intensity was proportional to the amount of PSI0242 (SEQ ID NO:249) and the sample concentrations were determined from the standard curve.

The C5 binding compounds comprising an albumin binding domain of streptococcal protein G proved capable of binding to human albumin and the data is presented in Table VII below.

**TABLE VII**

| SEQ ID NO: | Name | % of total protein content |
|---|---|---|
| 249 | PSI0242 | 103 |
| 261 | PSI0378 | 85 |
| 268 | PSI0410 | 150 |

The results from the assay showed that both of the investigated stability improved C5 binding compounds maintain their ability to bind human albumin.

### EXAMPLE 8: 3 month stability test of C5 binding polypeptides/compounds

The C5 binding polypeptides/compounds that showed an improved stability compared toPSI0242 in the 2 weeks stability test at 37 °C (Example 2) were subjected to a longer 3 month stability test at 37 °C. The setup of the stability test was as described in Example 2 and the evaluation of the stability was made by measuring the main peak of the chromatogram percentage of the total protein content by Reversed Phase HPLC (RPC), the RPC method was performed as described in example 2. The 2 weeks data from example 2 is included in Table VIII below to make the interpretation easier.

**TABLE VIII**

| SEQ ID NO: | Name | 2 weeks, 37 °C Main peak (% of total protein) | 3 months, 37 °C Main peak (% of total protein) |
|---|---|---|---|
| 253 | PSI0334 | 73 | 16 |
| 261 | PSI0378 | 89 | 59 |
| 262 | PSI0379 | 88 | 58 |
| 263 | PSI0381 | 87 | 46 |
| 264 | PSI0383 | 91 | 59 |
| 268 | PSI0410 | 72 | 16 |
| 269 | PSI0403 | 77 | 35 |
| 270 | PSI0404 | 88 | 46 |

C5 binding compounds with amino acid substitutions in position 52, 53 from ND to SE and a replacement in position 60 from D to E or A (SEQ ID NO: 261, 264, and 262) compared to PSI0242 have a higher proportion of protein in the original form after 3 months at 37 °C than PSI0242 (SEQ ID NO: 249) has after 2 weeks at the same conditions. The other compounds also displayed an increased stability.

### EXAMPLE 9: Stability of similarly modified polypeptides

Previously known polypeptide variants derived from protein Z (Grönwall et al. J Biotechnol 2007, 128:162-183) with binding affinity for other target molecules than C5 were similarly modified in specific positions of the amino acid sequence in order to improve stability. Selection and production of the original polypeptide variants with binding affinity for the human epidermal growth factor receptor 2 (HER2), the platelet-derived growth factor receptor beta (PDGF-Rβ), the neonatal Fc receptor (FcRn), and the carbonic anhydrase IX (CAIX) is disclosed in e.g. WO 2009/080810, WO 2009/077175, PCT/EP2014/055299, and WO 2014/096163. The stability improved polypeptide variants were produced by site-directed mutagenesis at selected positions of the amino acid sequence. The stability improving amino acid substitutions in the polypeptide variants Z02891 (SEQ ID NO: 272), targeting HER2; Z15805 (SEQ ID NO: 275), targeting PDGF-Rβ; Z10103 (SEQ ID NO: 278), targeting FcRn; and Z09782 (SEQ ID NO: 281), targeting CAIX, are specified below in Table IX. These stability improved polypeptide variants differ from the C5 binding polypeptides of the present invention for example in that they have a binding motif [*BM*] with binding affinity for HER2, PDGF-Rβ, FcRn, and CAIX.

All variants were cloned with an N-terminal 6 x Histidine-tag (His6) and the achieved constructs encoded polypeptides in the format MGSSHHHHHHLQ-[Z#####]. Mutations were introduced in the plasmids of the polypeptide variants using overlapping oligonucleotide primer pairs encoding the desired amino acid substitutions and by applying established molecular biology techniques. The correct plasmid sequences were verified by DNA sequencing.

*E coli* (strain T7E2) cells (GeneBridge) were transformed with plasmids containing the gene fragments encoding the original and the modified polypeptides. The cells were cultivated at 37 °C in TSB-YE medium supplemented with 50 µg/ml kanamycin and protein expression was subsequently induced by addition of IPTG. Pelleted cells were disrupted using a FastPrep®-24 homogenizer (Nordic Biolabs) and cell debris was removed by centrifugation. Each supernatant containing the polypeptide variant as a His6-tagged protein was purified by immobilized metal ion affinity chromatography (IMAC) using His GraviTrapTM columns (GE Healthcare) according to the manufacturers instructions. Purified polypeptide variants were buffer exchanged to phosphate-buffered saline (PBS; 1.47 mM KH2PO4, 8.1 mM Na2HPO4, 137 mM NaCl, 2.68 mM KCl, pH 7.4) using PD-10 desalting columns (GE Healthcare). The correct identity of each polypeptide was verified by SDS-PAGE and HPLC-MS.

**TABLE IX.**

| SEQ ID NO: | Name | Target | Amino acid substitutions | Original vs modified |
|---|---|---|---|---|
| 272 | Z02891 | HER2 | - | Original |
| 273 | Z17341 | HER2 | N52S, D53E | Modified |
| 274 | Z17342 | HER2 | D36R, D37Q, S39E, N52S, D53E | Modified |
| 275 | Z15805 | PDGF-Rβ | - | Original |
| 276 | Z17343 | PDGF-Rβ | N52S, D53E | Modified |
| 277 | Z17344 | PDGF-Rβ | D36R, D37Q, S39E, N52S, D53E | Modified |
| 278 | Z10103 | FcRn | - | Original |
| 279 | Z17347 | FcRn | N52S, D53E | Modified |
| 280 | Z17348 | FcRn | D36R, D37Q, S39E, N52S, D53E | Modified |
| 281 | Z09782 | CAIX | - | Original |
| 282 | Z17351 | CAIX | N52S, D53E | Modified |
| 283 | Z17352 | CAIX | D36R, D37Q, S39E, N52S, D53E | Modified |
| 284 | Z17355 | CAIX | D53E | Modified |
| 285 | Z17357 | CAIX | D36R, D37Q, S39E, D53E | Modified |
| 286 | Z17359 | CAIX | N52S | Modified |
| 287 | Z17360 | CAIX | D36R, D37Q, S39E, N52S | Modified |

Apart from the substitutions of one of (SEQ ID NO: 284-287) or both of (SEQ ID NO: 273-274, 276-277, 279-280, 282-283) N52 and D53, substitutions were also performed in the positions corresponding to the loop [L2]. Thus, in the polypeptide variants of SEQ ID NO: 274, 277, 280, 283, 285, and 287, [L2] is RQPE.

For carrying out the stability testing, the polypeptide variants, formulated in PBS pH 7.4, were diluted to 1 mg/ml and 200 µl aliquotes were incubated at 37 °C for 2 weeks. Samples collected prior to and after the stability test were analyzed by SDS-PAGE using 10% Bis-Tris NuPAGE gels (Invitrogen) and loading 5 µg protein into each well. Resulting Coomassie blue stained gels are shown in Fig. 6. The stability was assessed by the appearance of new variants after incubation at the elevated temperature and mutated variants were compared to respective original polypeptide.

All polypeptide variants with the modifications as outlined in Table IX showed improved stability compared to the respective original polypeptide in the sense that a second band just above the main band observed for samples of the original polypeptide was not visible in samples of the modified polypeptides with the substitution D53E and/or N52S, see Fig. 6. Polypeptides with the substitutions D53E and/or N52S combined with the substitutions D36R, D37Q and S39E showed similar profiles on the SDS-PAGE gel. The substitution D53E alone or in combination with the substitutions D36R, D37Q and S39E seemed to reduce the amount of the species with an alternative conformation observed as a second band on the SDS-PAGE gel, but could not completely prevent the formation of this species.

In addition, the binding capability of the modified polypeptide variants was tested. All polypeptide variants retained their binding affinity for their target after being modified (results not shown).

The results presented above for the polypeptide variants having binding affinity for other target molecules than C5 correspond well with the results presented for the C5 binding polypeptides and compounds of the present invention (see e.g. Example 2 and 4). Thus, the specific amino acid modifications as described herein appear to have a stabilizing effect irrespective of the amino acid sequence of the [*BM*]. The amino acid modifications or substitutions as described herein are thus considered to improve stability of all the C5 binding polypeptides and compounds as described herein and in WO 2013/126006.

## Claims

1. A polypeptide capable of binding human complement component 5 (C5), said polypeptide comprising the amino acid sequence:
[*BM*]-[L2]-QSX₄₂X₄₃LLX₄₆EAKKLX₅₂X₅₃X₅₄Q
wherein, independently of each other,
[*BM*] is a C5 binding motif comprising the amino acid sequence
EX₉X₁₀X₁₁A X₁₃X₁₄EIDX₁₈LPNLX₂₃X₂₄X₂₅QWX₂₈AFIX₃₂X₃₃LX₃₅;
wherein, independently of each other,
X₉ is selected from H, Q, S, T and V;
X₁₀ is selected from I, L, M and V;
X₁₁ is selected from A, D, E, H, K, L, N, Q, R, S, T and Y;
X₁₃ is selected from N and W;
X₁₄ is selected from A, D, E, H, N, Q, R, S and T;
X₁₈ is selected from A, E, G, H, K, L, Q, R, S, T and Y;
X₂₃ is selected from N and T;
X₂₄ is selected from I, L and V;
X₂₅ is selected from A, D, E, H, K, N, Q, R, S and T;
X₂₈ is selected from I, L and V;
X₃₂ is selected from D, E, G, H, N, S and T;
X₃₃ is selected from K and S; and
X₃₅ is selected from A, D, E, H, N, Q, S, T and Y;
[L2] is selected from DDPS and RQPE;
X₄₂ is selected from A and S;
X₄₃ is selected from N and E;
X₄₆ is selected from A, S and C;
X₅₂ is selected from E, N and S;
X₅₃ is selected from D, E and S, provided that X₅₃ is not D when X₅₂ is N; and
X₅₄ is selected from A and S.

2. The polypeptide according to claim 1, said polypeptide comprising the amino acid sequence:
AEAKYAK-[*BM*-[L2]-QSX₄₂X₄₃LLX₄₆EAKKLX₅₂X₅₃X₅₄QAP
wherein, independently of each other,
[*BM*], [L2], X₄₂, X₄₃, X₄₆, X₅₂, X₅₃ and X₅₄ are as defined in claim 1.

3. The polypeptide according to claim 1 or 2, wherein
X₅₂ and X₅₃ are independently selected from E and S;
X₅₂ is S and X₅₃ is E;
X₅₂ is E and X₅₃ is S;
X₅₂ is S and X₅₃ is D, or
X₅₂ is N and X₅₃ is E.

4. The polypeptide according to any one of claims 1 to 3 comprising the amino acid sequence: wherein, independently of each other,
X₉ is selected from H, Q, S, T and V;
X₁₀ is selected from I, L, M and V;
X₁₁ is selected from A, D, E, H, K, L, N, Q, R, S, T and Y;
X₁₃ is selected from N and W;
X₁₄ is selected from A, D, E, H, N, Q, R, S and T;
X₁₈ is selected from A, E, G, H, K, L, Q, R, S, T and Y;
X₂₃ is selected from N and T;
X₂₄ is selected from I, L and V;
X₂₅ is selected from A, D, E, H, K, N, Q, R, S and T;
X₂₈ is selected from I, L and V;
X₃₂ is selected from D, E, G, H, N, S and T;
X₃₃ is selected from K and S;
X₃₅ is selected from A, D, E, H, N, Q, S, T and Y;
[L2] is selected from DDPS and RQPE;
X₄₂ is selected from A and S;
X₄₃ is selected from N and E;
X₄₆ is selected from A, S and C;
X₅₂ is selected from E, N and S;
X₅₃ is selected from D, E and S, provided that X₅₃ is not D when X₅₂ is N; and
X₅₄ is selected from A and S.

5. The polypeptide according to any one of the claims 1 to 4, wherein at least one of the following conditions is fulfilled:
X₉ is V,
X₁₀ is L,
X₁₁ is E,
X₁₃ is W,
X₁₄ is D,
X₁₈ is R,
X₂₃ is T,
X₂₄ is I,
X₂₅ is E,
X₂₈ is L,
X₃₂ is N,
X₃₃ is K,
X₃₅ is D,
[L2] is DDPS,
X₄₂ is S,
X₄₃ is E,
X₄₆ is S,
X₅₄ is S.

6. The polypeptide according to any one of claims 1 to 5, wherein [*BM*] comprises an amino acid sequence selected from the group consisting of positions 1-28 in SEQ ID NOS: 1-248, preferably wherein [*BM*] comprises the amino acid sequence shown as positions 1-28 in SEQ ID NO: 1.

7. The polypeptide according to any one of claims 1 to 6, selected from a polypeptide comprising the amino acid sequence shown as SEQ ID NO: 260, SEQ ID NO: 265, SEQ ID NO: 266 and SEQ ID NO: 267.

8. A compound capable of binding C5, said compound comprising:
a. at least one C5 binding polypeptide according to any one of the preceding claims;
b. at least one albumin binding domain of streptococcal protein G; and
c. optionally, at least one linking moiety for linking said at least one albumin binding domain to the C or N terminal of said at least one C5 binding polypeptide, said linking moiety preferably being a peptide comprising the amino acid sequence KVX₆₀GS, wherein X₆₀ is selected from D, E and A.

9. The compound according to claim 8, wherein X₆₀ is E and said compound is a polypeptide comprising the amino acid sequence shown as SEQ ID NO: 261, SEQ ID NO: 263, SEQ ID NO: 264, SEQ ID NO: 269 or SEQ ID NO: 270.

10. The compound according to claim 8, wherein X₆₀ is A and said compound is a polypeptide comprising the amino acid sequence shown as SEQ ID NO: 262.

11. The C5 binding polypeptide according to any one of claims 1-7 or the C5 binding compound according to any one of claims 8-10 for use in therapy.

12. The C5 binding polypeptide according to any one of claims 1-7 or the C5 binding compound according to any one of claims 8-10 for use in a method for treatment and/or prophylaxis of a C5-related condition.

13. The C5 binding polypeptide for use according to claim 12 or the C5 binding compound for use according to claim 12, wherein said C5-related condition is a condition selected from inflammatory diseases; autoimmune diseases; infectious diseases; cardiovascular diseases; neurodegenerative disorders; graft injury; eye diseases; kidney diseases; pulmonary diseases; hematological diseases such as paroxysmal nocturnal hemoglobinuria (PNH); allergic diseases and dermatological diseases.

14. The C5 binding polypeptide for use according to any one of claims 11-13 or the C5 binding compound for use according to any one of claims 11-13, wherein said C5 binding polypeptide or compound is administered intravenously, subcutaneously, by inhalation, nasally, orally, intravitreally, or topically.

## Patentansprüche

1. Polypeptid, das in der Lage ist, menschliche Komplementkomponente 5 (C5) zu binden, das Polypeptid umfassend die Aminosäuresequenz:
[*BM*]-[L2]-QSX₄₂X₄₃LLX₄₆EAKKLX₅₂X₅₃X₅₄Q
wobei unabhängig voneinander
*[BM]* ein C5-Bindungsmotiv ist, das die Aminosäuresequenz EX₉X₁₀X₁₁AX₁₃X₁₄EIDX₁₈LPNLX₂₃X₂₄X₂₅QWX₂₈AFIX₃₂X₃₃LX₃₅ umfasst;
wobei unabhängig voneinander
X₉ ausgewählt ist aus H, Q, S, T und V;
X₁₀ ausgewählt ist aus I, L, M und V;
X₁₁ ausgewählt ist aus A, D, E, H, K, L, N, Q, R, S, T und Y;
X₁₃ ausgewählt ist aus N und W;
X₁₄ ausgewählt ist aus A, D, E, H, N, Q, R, S und T;
X₁₈ ausgewählt ist aus A, E, G, H, K, L, Q, R, S, T und Y;
X₂₃ ausgewählt ist aus N und T;
X₂₄ ausgewählt ist aus I, L und V;
X₂₅ ausgewählt ist aus A, D, E, H, K, N, Q, R, S und T;
X₂₈ ausgewählt ist aus I, L und V;
X₃₂ ausgewählt ist aus D, E, G, H, N, S und T;
X₃₃ ausgewählt ist aus K und S; und
X₃₅ ausgewählt ist aus A, D, E, H, N, Q, S, T und Y;
[L2] ausgewählt ist aus DDPS und RQPE;
X₄₂ ausgewählt ist aus A und S;
X₄₃ ausgewählt ist aus N und E;
X₄₆ ausgewählt ist aus A, S und C;
X₅₂ ausgewählt ist aus E, N und S;
X₅₃ ausgewählt ist aus D, E und S, vorausgesetzt, dass X₅₃ nicht D ist, wenn X₅₂ N ist; und
X₅₄ ausgewählt ist aus A und S.

2. Polypeptid nach Anspruch 1, das Polypeptid umfassend die Aminosäuresequenz:
AEAKYAK-[*BM*]-[L2]-QSX₄₂X₄₃LLX₄₆EAKKLX₅₂X₅₃X₅₄QAP
wobei unabhängig voneinander
[*BM*], [L2], X₄₂, X₄₃, X₄₆, X₅₂, X₅₃ und X₅₄ sind, wie in Anspruch 1 definiert.

3. Polypeptid nach Anspruch 1 oder 2, wobei
X₅₂ und X₅₃ unabhängig ausgewählt sind aus E und S;
X₅₂ S ist und X₅₃ E ist;
X₅₂ E ist und X₅₃ S ist;
X₅₂ S ist und X₅₃ D ist, oder
X₅₂ N ist und X₅₃ E ist.

4. Polypeptid nach einem der Ansprüche 1 bis 3, umfassend die Aminosäuresequenz: wobei unabhängig voneinander
X₉ ausgewählt ist aus H, Q, S, T und V;
X₁₀ ausgewählt ist aus I, L, M und V;
X₁₁ ausgewählt ist aus A, D, E, H, K, L, N, Q, R, S, T und Y;
X₁₃ ausgewählt ist aus N und W;
X₁₄ ausgewählt ist aus A, D, E, H, N, Q, R, S und T;
X₁₈ ausgewählt ist aus A, E, G, H, K, L, Q, R, S, T und Y;
X₂₃ ausgewählt ist aus N und T;
X₂₄ ausgewählt ist aus I, L und V;
X₂₅ ausgewählt ist aus A, D, E, H, K, N, Q, R, S und T;
X₂₈ ausgewählt ist aus I, L und V;
X₃₂ ausgewählt ist aus D, E, G, H, N, S und T;
X₃₃ ausgewählt ist aus K und S;
X₃₅ ausgewählt ist aus A, D, E, H, N, Q, S, T und Y;
[L2] ausgewählt ist aus DDPS und RQPE;
X₄₂ ausgewählt ist aus A und S;
X₄₃ ausgewählt ist aus N und E;
X₄₆ ausgewählt ist aus A, S und C;
X₅₂ ausgewählt ist aus E, N und S;
X₅₃ ausgewählt ist aus D, E und S, vorausgesetzt, dass X₅₃ nicht D ist, wenn X₅₂ N ist;
und
X₅₄ ausgewählt ist aus A und S.

5. Polypeptid nach einem der Ansprüche 1 bis 4, wobei mindestens eine der folgenden Bedingungen erfüllt ist:
X₉ ist V,
X₁₀ ist L,
X₁₁ ist E,
X₁₃ ist W,
X₁₄ ist D,
X₁₈ ist R,
X₂₃ ist T,
X₂₄ ist I,
X₂₅ ist E,
X₂₈ ist L,
X₃₂ ist N,
X₃₃ ist K,
X₃₅ ist D,
[L2] ist DDPS,
X₄₂ ist S,
X₄₃ ist E,
X₄₆ ist S,
X₅₄ ist S.

6. Polypeptid nach einem der Ansprüche 1 bis 5, wobei *[BM]* eine Aminosäuresequenz, ausgewählt aus der Gruppe bestehend aus den Positionen 1-28 in SEQ ID NOS: 1-248, umfasst, bevorzugt wobei *[BM]* die Aminosäuresequenz, die als Positionen 1-28 in SEQ ID NO: 1 dargestellt ist, umfasst.

7. Polypeptid nach einem der Ansprüche 1 bis 6, ausgewählt aus einem Polypeptid, das die als SEQ ID NO:260, SEQ ID NO:265, SEQ ID NO:266 und SEQ ID NO:267 dargestellte Aminosäuresequenz, umfasst.

8. Verbindung, die in der Lage ist, C5 zu binden, die Verbindung umfassend:
a. mindestens ein C5-bindendes Polypeptid nach einem der vorstehenden Ansprüche;
b. mindestens eine Albumin-Bindungsdomäne des Streptokokkenproteins G; und
c. optional mindestens eine Verknüpfungseinheit zum Verknüpfen der mindestens einen Albumin-Bindungsdomäne mit dem C- oder N-Terminal des mindestens einen C5-bindenden Polypeptids, wobei die Verknüpfungseinheit bevorzugt ein Peptid ist, das die Aminosäuresequenz KVX₆₀GS umfasst, wobei X₆₀ ausgewählt ist aus D, E und A.

9. Verbindung nach Anspruch 8, wobei X₆₀ E ist und die Verbindung ein Polypeptid ist, das die Aminosäuresequenz umfasst, die als SEQ ID NO: 261, SEQ ID NO: 263, SEQ ID NO: 264, SEQ ID NO: 269 oder SEQ ID NO: 270 dargestellt ist.

10. Verbindung nach Anspruch 8, wobei X₆₀ A ist und die Verbindung ein Polypeptid ist, das die Aminosäuresequenz umfasst, die als SEQ ID NO: 262 dargestellt ist.

11. C5-bindendes Polypeptid nach einem der Ansprüche 1-7 oder C5-bindende Verbindung nach einem der Ansprüche 8-10 zur Verwendung in der Therapie.

12. C5-bindendes Polypeptid nach einem der Ansprüche 1-7 oder C5-bindende Verbindung nach einem der Ansprüche 8-10 zur Verwendung in einem Verfahren zur Behandlung und/oder Prophylaxe einer C5-bezogenen Erkrankung.

13. C5-bindendes Polypeptid zur Verwendung nach Anspruch 12 oder C5-bindende Verbindung zur Verwendung nach Anspruch 12, wobei die C5-bezogene Erkrankung eine Erkrankung ist, die ausgewählt ist aus Entzündungskrankheiten; Autoimmunerkrankungen; Infektionserkrankungen; Herz-Kreislauf-Erkrankungen; neurodegenerative Störungen; Transplantatverletzung; Augenerkrankungen; Nierenerkrankungen; Lungenerkrankungen; hämatologische Erkrankungen wie paroxysmale nächtliche Hämoglobinurie (PNH); allergische Erkrankungen und dermatologische Erkrankungen.

14. C5-bindendes Polypeptid zur Verwendung nach einem der Ansprüche 11-13 oder die C5-bindende Verbindung zur Verwendung nach einem der Ansprüche 11-13, wobei das C5-bindende Polypeptid oder die C5-bindende Verbindung intravenös, subkutan, durch Inhalation, nasal, oral, intravitreal oder topisch verabreicht wird.

## Revendications

1. Polypeptide capable de se lier au composant 5 (C5) du complément humain, ledit polypeptide comprenant la séquence d'acides aminés :
[*BM*]-[L2]-QSX₄₂X₄₃LLX₄₆EAKKLX₅₂X₅₃X₅₄Q
dans lequel, indépendamment les uns des autres,
*[BM]* est un motif de liaison à C5 comprenant la séquence d'acides aminés EX₉X₁₀X₁₁AX₁₃X₁₄EIDX₁₈LPNLX₂₃X₂₄X₂₅QWX₂₈AFIX₃₂X₃₃LX₃₅ ;
dans lequel, indépendamment les uns des autres,
X₉ est choisi parmi H, Q, S, T et V ;
X₁₀ est choisi parmi I, L, M et V ;
X₁₁ est choisi parmi A, D, E, H, K, L, N, Q, R, S, T et Y ;
X₁₃ est choisi parmi N et W ;
X₁₄ est choisi parmi A, D, E, H, N, Q, R, S et T ;
X₁₈ est choisi parmi A, E, G, H, K, L, Q, R, S, T et Y ;
X₂₃ est choisi parmi N et T ;
X₂₄ est choisi parmi I, L et V ;
X₂₅ est choisi parmi A, D, E, H, K, N, Q, R, S et T ;
X₂₈ est choisi parmi I, L et V ;
X₃₂ est choisi parmi D, E, G, H, N, S et T ;
X₃₃ est choisi parmi K et S ; et
X₃₅ est choisi parmi A, D, E, H, N, Q, S, T et Y ;
[L2] est choisi parmi DDPS et RQPE ;
X₄₂ est choisi parmi A et S ;
X₄₃ est choisi parmi N et E ;
X₄₆ est choisi parmi A, S et C ;
X₅₂ est choisi parmi E, N et S ;
X₅₃ est choisi parmi D, E et S, à condition que X₅₃ n'est pas D lorsque X₅₂ est N ;
et
X₅₄ est choisi parmi A et S.

2. Polypeptide selon la revendication1, ledit polypeptide comprenant la séquence d'acides aminés :
AEAKYAK-[*BM*]-[L2]-QSX₄₂X₄₃LLX₄₆EAKKLX₅₂X₅₃X₅₄QAP
dans lequel, indépendamment les uns des autres,
[*BM*], [L2], X₄₂, X₄₃, X₄₆, X₅₂, X₅₃ et X₅₄ sont définis selon la revendication 1.

3. Polypeptide selon la revendication 1 ou 2, dans lequel
X₅₂ et X₅₃ sont choisis indépendamment parmi E et S ;
X₅₂ est S et X₅₃ est E ;
X₅₂ est E et X₅₃ est S ;
X₅₂ est S et X₅₃ est D, ou
X₅₂ est N et X₅₃ est E.

4. Polypeptide selon l'une quelconque des revendications 1 à 3 comprenant la séquence d'acides aminés : dans lequel, indépendamment les uns des autres,
X₉ est choisi parmi H, Q, S, T et V ;
X₁₀ est choisi parmi I, L, M et V ;
X₁₁ est choisi parmi A, D, E, H, K, L, N, Q, R, S, T et Y ;
X₁₃ est choisi parmi N et W ;
X₁₄ est choisi parmi A, D, E, H, N, Q, R, S et T ;
X₁₈ est choisi parmi A, E, G, H, K, L, Q, R, S, T et Y ;
X₂₃ est choisi parmi N et T ;
X₂₄ est choisi parmi I, L et V ;
X₂₅ est choisi parmi A, D, E, H, K, N, Q, R, S et T ;
X₂₈ est choisi parmi I, L et V ;
X₃₂ est choisi parmi D, E, G, H, N, S et T ;
X₃₃ est choisi parmi K et S ;
X₃₅ est choisi parmi A, D, E, H, N, Q, S, T et Y ;
[L2] est choisi parmi DDPS et RQPE ;
X₄₂ est choisi parmi A et S ;
X₄₃ est choisi parmi N et E ;
X₄₆ est choisi parmi A, S et C ;
X₅₂ est choisi parmi E, N et S ;
X₅₃ est choisi parmi D, E et S, à condition que X₅₃ n'est pas D lorsque X₅₂ est N ;
et
X₅₄ est choisi parmi A et S.

5. Polypeptide selon l'une quelconque des revendications 1 à 4, dans lequel au moins l'une des conditions suivantes est remplie :
Xg est V,
X₁₀ est L,
X₁₁ est E,
X₁₃ est W,
X₁₄ est D,
X₁₈ est R,
X₂₃ est T,
X₂₄ est I,
X₂₅ est E,
X₂₈ est L,
X₃₂ est N,
X₃₃ est K,
X₃₅ est D,
[L2] est DDPS,
X₄₂ est S,
X₄₃ est E,
X₄₆ est S,
X₅₄ est S.

6. Polypeptide selon l'une quelconque des revendications 1 à 5, dans lequel [*BM*] comprend une séquence d'acides aminés choisi parmi le groupe se composant des positions 1-28 dans les SEQ ID N : 1-248, de préférence dans lequel [*BM*] comprend la séquence d'acides aminés telle que représentée par les positions 1-28 de la SEQ ID N : 1.

7. Polypeptide selon l'une quelconque des revendications 1 à 6, choisi parmi un polypeptide comprenant la séquence d'acides aminés telle que représentée par la SEQ ID N : 260, la SEQ ID N : 265, la SEQ ID N : 266 et la SEQ ID N : 267.

8. Composé capable de se lier à C5, ledit composé comprenant :
a. au moins un polypeptide de liaison à C5 selon l'une quelconque des revendications précédentes ;
b. au moins un domaine de liaison à l'albumine de la protéine streptococcique G ; et
c. facultativement, au moins un fragment de liaison destiné à lier ledit au moins un domaine de liaison à l'albumine à l'extrémité C- ou N-terminale dudit au moins un polypeptide de liaison à C5, ledit fragment de liaison étant de préférence un peptide comprenant la séquence d'acides aminés KVX₆₀GS, dans lequel X₆₀ est choisi parmi D, E et A.

9. Composé selon la revendication 8, dans lequel X₆₀ est E et ledit composé est un polypeptide comprenant la séquence d'acides aminés telle que représentée par la SEQ ID N : 261, la SEQ ID N : 263, la SEQ ID N : 264, la SEQ ID N : 269 ou la SEQ ID N : 270.

10. Composé selon la revendication 8, dans lequel X₆₀ est A et ledit composé est un polypeptide comprenant la séquence d'acides aminés telle que représentée par la SEQ ID N : 262.

11. Polypeptide de liaison à C5 selon l'une quelconque des revendications 1-7 ou composé de liaison à C5 selon l'une quelconque des revendications 8-10 destiné à une utilisation thérapeutique.

12. Polypeptide de liaison à C5 selon l'une quelconque des revendications 1-7 ou composé de liaison à C5 selon l'une quelconque des revendications 8-10 destiné à une utilisation dans une méthode de traitement et/ou en prophylaxie d'une maladie liée à C5.

13. Polypeptide de liaison à C5 destiné à une utilisation selon la revendication 12 ou composé de liaison à C5 destiné à une utilisation selon la revendication 12, dans lequel ladite maladie liée à C5 est une maladie choisie parmi les maladies inflammatoires; maladies auto-immunes ; maladies infectieuses; maladies cardiovasculaires ; maladies neurodégénératives ; lésion du greffon; maladies oculaires; maladies pulmonaires; maladies hématologiques telles que l'hémoglobinurie paroxystique nocturne (HPN) ; maladies allergiques et maladies dermatologiques.

14. Polypeptide de liaison à C5 destiné à une utilisation selon l'une quelconque des revendications 11-13 ou composé de liaison à C5 destiné à une utilisation selon l'une quelconque des revendications 11-13, dans lequel ledit polypeptide ou composé de liaison à C5 est administré par voie intraveineuse, par voie sous-cutanée, par voie inhalée, par voie nasale, par voie orale, par voie intravitréenne, ou par voie topique.


### SEQUENCE LISTING

<110> Swedish Orphan Biovitrum AB
<120> STABLE POLYPEPTIDES BINDING TO HUMAN COMPLEMENT C5
<130> 21070713
<160> 287
<170> PatentIn version 3.5
<210> 1
   <211> 29
   <212> PRT
   <213> Artificial Sequence
<220>
   <223> Modified bacterial sequence
<400> 1
   Glu Val Leu Glu Ala Trp Asp Glu Ile Asp Arg Leu Pro Asn Leu Thr
   1               5                   10                  15
   Ile Glu Gln Trp Leu Ala Phe Ile Asn Lys Leu Asp Asp
               20                  25
<210> 2
   <211> 29
   <212> PRT
   <213> Artificial Sequence
<220>
   <223> Modified bacterial sequence
<400> 2
   Glu Val Leu Glu Ala Trp Asn Glu Ile Asp Arg Leu Pro Asn Leu Thr
   1               5                   10                  15
   Ile Glu Gln Trp Leu Ala Phe Ile Asn Lys Leu Asp Asp
               20                  25
<210> 3
   <211> 29
   <212> PRT
   <213> Artificial Sequence
<220>
   <223> Modified bacterial sequence
<400> 3
   Glu Val Ile Glu Ala Trp Asn Glu Ile Asp Arg Leu Pro Asn Leu Thr
   1               5                   10                  15
   Ile Glu Gln Trp Leu Ala Phe Ile Asn Lys Leu Asp Asp
                       20                  25
<210> 4
   <211> 29
   <212> PRT
   <213> Artificial Sequence
<220>
   <223> Modified bacterial sequence
<400> 4
   Glu Val Leu Glu Ala Trp Asp Glu Ile Asp Arg Leu Pro Asn Leu Thr
   1               5                   10                  15
   Leu Asp Gln Trp Leu Ala Phe Ile Asn Lys Leu Asp Asp
               20                  25
<210> 5
   <211> 29
   <212> PRT
   <213> Artificial Sequence
<220>
   <223> Modified bacterial sequence
<400> 5
   Glu Val Leu Asp Ala Trp Asp Glu Ile Asp Ala Leu Pro Asn Leu Thr
   1               5                   10                  15
   Leu Glu Gln Trp Leu Ala Phe Ile Asn Lys Leu Asp Asp
               20                  25
<210> 6
   <211> 29
   <212> PRT
   <213> Artificial Sequence
<220>
   <223> Modified bacterial sequence
<400> 6
   Glu Val Ile Asp Ala Trp Asp Glu Ile Asp Arg Leu Pro Asn Leu Thr
   1               5                   10                  15
   Leu Asp Gln Trp Leu Ala Phe Ile Asn Lys Leu Asp Asp
               20                  25
<210> 7
   <211> 29
   <212> PRT
   <213> Artificial Sequence
<220>
   <223> Modified bacterial sequence
<400> 7
   Glu Thr Leu Glu Ala Trp Asp Glu Ile Asp Arg Leu Pro Asn Leu Thr
   1 5 10 15
   Ile Glu Gln Trp Leu Ala Phe Ile Asn Lys Leu Asp Asp
   20 25
<210> 8
   <211> 29
   <212> PRT
   <213> Artificial Sequence
<220>
   <223> Modified bacterial sequence
<400> 8
   Glu Val Ile Asp Ala Trp Asn Glu Ile Asp Ala Leu Pro Asn Leu Thr
   1 5 10 15
   Leu Asp Gln Trp Leu Ala Phe Ile Asn Lys Leu Asp Asp
   20 25
<210> 9
   <211> 29
   <212> PRT
   <213> Artificial Sequence
<220>
   <223> Modified bacterial sequence
<400> 9
   Glu Val Leu Asp Ala Trp Asp Glu Ile Asp Lys Leu Pro Asn Leu Thr
   1 5 10 15
   Ile Asp Gln Trp Leu Ala Phe Ile Asn Lys Leu Asp Asp
   20 25
<210> 10
   <211> 29
   <212> PRT
   <213> Artificial Sequence
<220>
   <223> Modified bacterial sequence
<400> 10
   Glu Val Leu Glu Ala Trp Asp Glu Ile Asp His Leu Pro Asn Leu Thr
   1 5 10 15
   Leu Asp Gln Trp Leu Ala Phe Ile Asn Lys Leu Asp Asp
   20 25
<210> 11
   <211> 29
   <212> PRT
   <213> Artificial Sequence
<220>
   <223> Modified bacterial sequence
<400> 11
   Glu Val Leu Glu Ala Trp Asp Glu Ile Asp Ala Leu Pro Asn Leu Thr
   1               5                   10                  15
   Ile Glu Gln Trp Leu Ala Phe Ile Asn Lys Leu Asp Asp
               20                  25
<210> 12
   <211> 29
   <212> PRT
   <213> Artificial Sequence
<220>
   <223> Modified bacterial sequence
<400> 12
   Glu Val Leu Asp Ala Trp Asp Glu Ile Asp Lys Leu Pro Asn Leu Thr
   1               5                   10                  15
   Leu Glu Gln Trp Leu Ala Phe Ile Asn Lys Leu Asp Asp
               20                  25
<210> 13
   <211> 29
   <212> PRT
   <213> Artificial Sequence
<220>
   <223> Modified bacterial sequence
<400> 13
   Glu Thr Ile Thr Ala Trp Asp Glu Ile Asp Lys Leu Pro Asn Leu Thr
   1               5                   10                  15
   Ile Glu Gln Trp Leu Ala Phe Ile Gly Lys Leu Glu Asp
               20                  25
<210> 14
   <211> 29
   <212> PRT
   <213> Artificial Sequence
<220>
   <223> Modified bacterial sequence
<400> 14
   Glu Ser Met Lys Ala Trp Asp Glu Ile Asp Arg Leu Pro Asn Leu Asn
   1               5                   10                  15
   Ile Asn Gln Trp Val Ala Phe Ile Asp Ser Leu Tyr Asp
               20                  25
<210> 15
   <211> 29
   <212> PRT
   <213> Artificial Sequence
<220>
   <223> Modified bacterial sequence
<400> 15
   Glu Ser Ile Glu Ala Trp Thr Glu Ile Asp His Leu Pro Asn Leu Thr
   1               5                   10                  15
   Ile Glu Gln Trp Leu Ala Phe Ile Asn Lys Leu Thr Asp
               20                  25
<210> 16
   <211> 29
   <212> PRT
   <213> Artificial Sequence
<220>
   <223> Modified bacterial sequence
<400> 16
   Glu Val Leu Asp Ala Trp His Glu Ile Asp Thr Leu Pro Asn Leu Thr
   1               5                   10                  15
   Val Arg Gln Trp Leu Ala Phe Ile Ser Lys Leu Glu Asp
               20                  25
<210> 17
   <211> 29
   <212> PRT
   <213> Artificial Sequence
<220>
   <223> Modified bacterial sequence
<400> 17
   Glu His Ile Gln Ala Asn Glu Glu Ile Asp Arg Leu Pro Asn Leu Thr
   1               5                   10                  15
   Ile Lys Gln Trp Leu Ala Phe Ile Asn Lys Leu His Asp
                       20                  25
<210> 18
   <211> 29
   <212> PRT
   <213> Artificial Sequence
<220>
   <223> Modified bacterial sequence
<400> 18
   Glu Val Leu His Ala Trp Ala Glu Ile Asp Ala Leu Pro Asn Leu Thr
   1               5                   10                  15
   Ile Glu Gln Trp Leu Ala Phe Ile Asn Lys Leu Asp Asp
               20                  25
<210> 19
   <211> 29
   <212> PRT
   <213> Artificial Sequence
<220>
   <223> Modified bacterial sequence
<400> 19
   Glu Val Leu Ala Ala Trp Asp Glu Ile Asp Ser Leu Pro Asn Leu Thr
   1               5                   10                  15
   Leu Gln Gln Trp Leu Ala Phe Ile Asn Lys Leu Asp Asp
               20                  25
<210> 20
   <211> 29
   <212> PRT
   <213> Artificial Sequence
<220>
   <223> Modified bacterial sequence
<400> 20
   Glu Val Ile Asp Ala Trp Asn Glu Ile Asp Ala Leu Pro Asn Leu Thr
   1               5                   10                  15
   Leu Glu Gln Trp Leu Ala Phe Ile Asn Lys Leu Asp Asp
               20                  25
<210> 21
   <211> 29
   <212> PRT
   <213> Artificial Sequence
<220>
   <223> Modified bacterial sequence
<400> 21
   Glu Val Leu Asp Ala Trp Asn Glu Ile Asp Ala Leu Pro Asn Leu Thr
   1               5                   10                  15
   Ile Asp Gln Trp Leu Ala Phe Ile Asn Lys Leu Ser Asp
               20                  25
<210> 22
   <211> 29
   <212> PRT
   <213> Artificial Sequence
<220>
   <223> Modified bacterial sequence
<400> 22
   Glu Val Ile Glu Ala Trp Asp Glu Ile Asp Gly Leu Pro Asn Leu Thr
   1               5                   10                  15
   Ile Glu Gln Trp Leu Ala Phe Ile Asn Lys Leu Asp Asp
               20                  25
<210> 23
   <211> 29
   <212> PRT
   <213> Artificial Sequence
<220>
   <223> Modified bacterial sequence
<400> 23
   Glu Val Leu Glu Ala Trp Asp Glu Ile Asp His Leu Pro Asn Leu Thr
   1               5                   10                  15
   Leu Gln Gln Trp Leu Ala Phe Ile Asn Lys Leu Asp Asp
               20                  25
<210> 24
   <211> 29
   <212> PRT
   <213> Artificial Sequence
<220>
   <223> Modified bacterial sequence
<400> 24
   Glu Val Ile Glu Ala Trp Asn Glu Ile Asp Ala Leu Pro Asn Leu Thr
   1               5                   10                  15
   Ile Glu Gln Trp Leu Ala Phe Ile Asn Lys Leu Asp Asp
                       20                  25
<210> 25
   <211> 29
   <212> PRT
   <213> Artificial Sequence
<220>
   <223> Modified bacterial sequence
<400> 25
   Glu Val Ile Ala Ala Trp Asn Glu Ile Asp Arg Leu Pro Asn Leu Thr
   1               5                   10                  15
   Leu Thr Gln Trp Leu Ala Phe Ile Asn Lys Leu Asp Asp
               20                  25
<210> 26
   <211> 29
   <212> PRT
   <213> Artificial Sequence
<220>
   <223> Modified bacterial sequence
<400> 26
   Glu Val Ile Glu Ala Trp Asp Glu Ile Asp Ala Leu Pro Asn Leu Thr
   1               5                   10                  15
   Leu Gln Gln Trp Leu Ala Phe Ile Asn Lys Leu Asp Asp
               20                  25
<210> 27
   <211> 29
   <212> PRT
   <213> Artificial Sequence
<220>
   <223> Modified bacterial sequence
<400> 27
   Glu Val Ile Ala Ala Trp Asp Glu Ile Asp Lys Leu Pro Asn Leu Thr
   1               5                   10                  15
   Ile Glu Gln Trp Leu Ala Phe Ile Asn Lys Leu Asp Asp
               20                  25
<210> 28
   <211> 29
   <212> PRT
   <213> Artificial Sequence
<220>
   <223> Modified bacterial sequence
<400> 28
   Glu Val Ile Ala Ala Trp Asp Glu Ile Asp Lys Leu Pro Asn Leu Thr
   1               5                   10                  15
   Leu Gln Gln Trp Leu Ala Phe Ile Asn Lys Leu Asp Asp
               20                  25
<210> 29
   <211> 29
   <212> PRT
   <213> Artificial Sequence
<220>
   <223> Modified bacterial sequence
<400> 29
   Glu Thr Ile Ala Ala Trp Asp Glu Ile Asp Lys Leu Pro Asn Leu Thr
   1               5                   10                  15
   Ile Glu Gln Trp Leu Ala Phe Ile Asn Lys Leu Asp Asp
               20                  25
<210> 30
   <211> 29
   <212> PRT
   <213> Artificial Sequence
<220>
   <223> Modified bacterial sequence
<400> 30
   Glu Thr Ile Glu Ala Trp Asn Glu Ile Asp Arg Leu Pro Asn Leu Thr
   1 5 10 15
   Ile Glu Gln Trp Leu Ala Phe Ile Asn Lys Leu Asp Asp
   20 25
<210> 31
   <211> 29
   <212> PRT
   <213> Artificial Sequence
<220>
   <223> Modified bacterial sequence
<400> 31
   Glu Val Leu Glu Ala Trp Arg Glu Ile Asp Ala Leu Pro Asn Leu Thr
   1 5 10 15
   Ile Gln Gln Trp Leu Ala Phe Ile Asn Lys Leu Asp Asp
   20 25
<210> 32
   <211> 29
   <212> PRT
   <213> Artificial Sequence
<220>
   <223> Modified bacterial sequence
<400> 32
   Glu Val Ile Glu Ala Trp Asp Glu Ile Asp Gln Leu Pro Asn Leu Thr
   1 5 10 15
   Ile Glu Gln Trp Leu Ala Phe Ile Asn Lys Leu Asp Asp
   20 25
<210> 33
   <211> 29
   <212> PRT
   <213> Artificial Sequence
<220>
   <223> Modified bacterial sequence
<400> 33
   Glu Val Leu Arg Ala Trp Asp Glu Ile Asp His Leu Pro Asn Leu Thr
   1               5                   10                  15
   Leu Glu Gln Trp Leu Ala Phe Ile Asn Lys Leu Asp Asp
               20                  25
<210> 34
   <211> 29
   <212> PRT
   <213> Artificial Sequence
<220>
   <223> Modified bacterial sequence
<400> 34
   Glu Val Leu Glu Ala Trp Asp Glu Ile Asp Arg Leu Pro Asn Leu Thr
   1               5                   10                  15
   Leu Asn Gln Trp Leu Ala Phe Ile Asn Lys Leu Asp Asp
               20                  25
<210> 35
   <211> 29
   <212> PRT
   <213> Artificial Sequence
<220>
   <223> Modified bacterial sequence
<400> 35
   Glu Val Leu Asp Ala Trp Asn Glu Ile Asp His Leu Pro Asn Leu Thr
   1               5                   10                  15
   Ile Glu Gln Trp Leu Ala Phe Ile Asn Lys Leu Asp Asp
               20                  25
<210> 36
   <211> 29
   <212> PRT
   <213> Artificial Sequence
<220>
   <223> Modified bacterial sequence
<400> 36
   Glu Val Ile Asp Ala Trp Asn Glu Ile Asp Lys Leu Pro Asn Leu Thr
   1               5                   10                  15
   Ile Glu Gln Trp Leu Ala Phe Ile Asn Lys Leu Asp Asp
               20                  25
<210> 37
   <211> 29
   <212> PRT
   <213> Artificial Sequence
<220>
   <223> Modified bacterial sequence
<400> 37
   Glu Thr Leu Glu Ala Trp Asp Glu Ile Asp Gln Leu Pro Asn Leu Thr
   1               5                   10                  15
   Leu Gln Gln Trp Leu Ala Phe Ile Asn Lys Leu Asp Asp
               20                  25
<210> 38
   <211> 29
   <212> PRT
   <213> Artificial Sequence
<220>
   <223> Modified bacterial sequence
<400> 38
   Glu Val Ile Glu Ala Trp Asn Glu Ile Asp Ala Leu Pro Asn Leu Thr
   1               5                   10                  15
   Leu Asp Gln Trp Leu Ala Phe Ile Asn Lys Leu Asp Asp
                        20                  25
<210> 39
   <211> 29
   <212> PRT
   <213> Artificial Sequence
<220>
   <223> Modified bacterial sequence
<400> 39
   Glu Val Ile Asp Ala Trp Asn Glu Ile Asp Arg Leu Pro Asn Leu Thr
   1               5                   10                  15
   Leu Gln Gln Trp Leu Ala Phe Ile Asn Lys Leu Asp Asp
               20                  25
<210> 40
   <211> 29
   <212> PRT
   <213> Artificial Sequence
<220>
   <223> Modified bacterial sequence
<400> 40
   Glu Val Ile Asp Ala Trp Asn Glu Ile Asp Gln Leu Pro Asn Leu Thr
   1               5                   10                  15
   Leu Glu Gln Trp Leu Ala Phe Ile Asn Lys Leu Asp Asp
               20                  25
<210> 41
   <211> 29
   <212> PRT
   <213> Artificial Sequence
<220>
   <223> Modified bacterial sequence
<400> 41
   Glu Thr Ile Ala Ala Trp Asp Glu Ile Asp His Leu Pro Asn Leu Thr
   1               5                   10                  15
   Leu Glu Gln Trp Leu Ala Phe Ile Asn Lys Leu Asp Asp
               20                  25
<210> 42
   <211> 29
   <212> PRT
   <213> Artificial Sequence
<220>
   <223> Modified bacterial sequence
<400> 42
   Glu Val Leu Gln Ala Trp Asp Glu Ile Asp His Leu Pro Asn Leu Thr
   1               5                   10                  15
   Ile Gln Gln Trp Leu Ala Phe Ile Asn Lys Leu Ser Asp
               20                  25
<210> 43
   <211> 29
   <212> PRT
   <213> Artificial Sequence
<220>
   <223> Modified bacterial sequence
<400> 43
   Glu Thr Leu His Ala Trp Ala Glu Ile Asp Arg Leu Pro Asn Leu Thr
   1               5                   10                  15
   Ile Glu Gln Trp Leu Ala Phe Ile Asn Lys Leu Asp Asp
               20                  25
<210> 44
   <211> 29
   <212> PRT
   <213> Artificial Sequence
<220>
   <223> Modified bacterial sequence
<400> 44
   Glu Val Leu Glu Ala Trp Asn Glu Ile Asp His Leu Pro Asn Leu Thr
   1               5                   10                  15
   Leu Ala Gln Trp Leu Ala Phe Ile Asn Lys Leu Asp Asp
               20                  25
<210> 45
   <211> 29
   <212> PRT
   <213> Artificial Sequence
<220>
   <223> Modified bacterial sequence
<400> 45
   Glu Val Ile Glu Ala Trp Asp Glu Ile Asp Lys Leu Pro Asn Leu Thr
   1               5                   10                  15
   Ile Ala Gln Trp Leu Ala Phe Ile Asn Lys Leu Asp Asp
                       20                  25
<210> 46
   <211> 29
   <212> PRT
   <213> Artificial Sequence
<220>
   <223> Modified bacterial sequence
<400> 46
   Glu Val Leu Asp Ala Trp Asp Glu Ile Asp His Leu Pro Asn Leu Thr
   1               5                   10                  15
   Leu Gln Gln Trp Leu Ala Phe Ile Asn Lys Leu Asp Asp
               20                  25
<210> 47
   <211> 29
   <212> PRT
   <213> Artificial Sequence
<220>
   <223> Modified bacterial sequence
<400> 47
   Glu Thr Ile Glu Ala Trp Asn Glu Ile Asp Lys Leu Pro Asn Leu Thr
   1               5                   10                  15
   Leu Thr Gln Trp Leu Ala Phe Ile Asn Lys Leu Asp Asp
               20                  25
<210> 48
   <211> 29
   <212> PRT
   <213> Artificial Sequence
<220>
   <223> Modified bacterial sequence
<400> 48
   Glu Val Leu Glu Ala Trp Asn Glu Ile Asp Leu Leu Pro Asn Leu Thr
   1               5                   10                  15
   Ile Glu Gln Trp Leu Ala Phe Ile Asn Lys Leu Asp Asp
               20                  25
<210> 49
   <211> 29
   <212> PRT
   <213> Artificial Sequence
<220>
   <223> Modified bacterial sequence
<400> 49
   Glu Val Ile Glu Ala Trp Asp Glu Ile Asp His Leu Pro Asn Leu Thr
   1               5                   10                  15
   Ile Asp Gln Trp Leu Ala Phe Ile Asn Lys Leu Asp Asp
               20                  25
<210> 50
   <211> 29
   <212> PRT
   <213> Artificial Sequence
<220>
   <223> Modified bacterial sequence
<400> 50
   Glu Val Ile Ser Ala Trp Asn Glu Ile Asp Ala Leu Pro Asn Leu Thr
   1               5                   10                  15
   Leu Gln Gln Trp Leu Ala Phe Ile Asn Lys Leu Asp Asp
               20                  25
<210> 51
   <211> 29
   <212> PRT
   <213> Artificial Sequence
<220>
   <223> Modified bacterial sequence
<400> 51
   Glu Val Ile Ala Ala Trp Asn Glu Ile Asp Lys Leu Pro Asn Leu Thr
   1               5                   10                  15
   Leu Glu Gln Trp Leu Ala Phe Ile Asn Lys Leu Asp Asp
               20                  25
<210> 52
   <211> 29
   <212> PRT
   <213> Artificial Sequence
<220>
   <223> Modified bacterial sequence
<400> 52
   Glu Thr Ile Glu Ala Trp Asn Glu Ile Asp Ser Leu Pro Asn Leu Thr
   1               5                   10                  15
   Leu Asp Gln Trp Leu Ala Phe Ile Asn Lys Leu Asp Asp
                        20                  25
<210> 53
   <211> 29
   <212> PRT
   <213> Artificial Sequence
<220>
   <223> Modified bacterial sequence
<400> 53
   Glu Val Leu Asp Ala Trp Asn Glu Ile Asp Gln Leu Pro Asn Leu Thr
   1               5                   10                  15
   Leu Gln Gln Trp Leu Ala Phe Ile Asn Lys Leu Asp Asp
               20                  25
<210> 54
   <211> 29
   <212> PRT
   <213> Artificial Sequence
<220>
   <223> Modified bacterial sequence
<400> 54
   Glu Val Leu Ala Ala Trp Asn Glu Ile Asp His Leu Pro Asn Leu Thr
   1               5                   10                  15
   Ile Glu Gln Trp Leu Ala Phe Ile Asn Lys Leu Asp Asp
               20                  25
<210> 55
   <211> 29
   <212> PRT
   <213> Artificial Sequence
<220>
   <223> Modified bacterial sequence
<400> 55
   Glu Val Leu Glu Ala Trp Asp Glu Ile Asp His Leu Pro Asn Leu Thr
   1               5                   10                  15
   Ile Thr Gln Trp Leu Ala Phe Ile Asn Lys Leu Asp Asp
               20                  25
<210> 56
   <211> 29
   <212> PRT
   <213> Artificial Sequence
<220>
   <223> Modified bacterial sequence
<400> 56
   Glu Thr Ile Asp Ala Trp Asn Glu Ile Asp His Leu Pro Asn Leu Thr
   1               5                   10                  15
   Ile Glu Gln Trp Leu Ala Phe Ile Asn Lys Leu Asp Asp
               20                  25
<210> 57
   <211> 29
   <212> PRT
   <213> Artificial Sequence
<220>
   <223> Modified bacterial sequence
<400> 57
   Glu Val Ile Glu Ala Trp Asn Glu Ile Asp His Leu Pro Asn Leu Thr
   1               5                   10                  15
   Ile Gln Gln Trp Leu Ala Phe Ile Asn Lys Leu Asp Asp
               20                  25
<210> 58
   <211> 29
   <212> PRT
   <213> Artificial Sequence
<220>
   <223> Modified bacterial sequence
<400> 58
   Glu Val Ile Gln Ala Trp Asn Glu Ile Asp Ala Leu Pro Asn Leu Thr
   1               5                   10                  15
   Ile Ser Gln Trp Leu Ala Phe Ile Asn Lys Leu Asp Asp
               20                  25
<210> 59
   <211> 29
   <212> PRT
   <213> Artificial Sequence
<220>
   <223> Modified bacterial sequence
<400> 59
   Glu Val Ile Ala Ala Trp Asp Glu Ile Asp Ser Leu Pro Asn Leu Thr
   1               5                   10                  15
   Ile Glu Gln Trp Leu Ala Phe Ile Asn Lys Leu Asp Asp
   20 25
<210> 60
   <211> 29
   <212> PRT
   <213> Artificial Sequence
<220>
   <223> Modified bacterial sequence
<400> 60
   Glu His Ile Glu Ala Trp Asn Glu Ile Asp Ala Leu Pro Asn Leu Thr
   1 5 10 15
   Ile Glu Gln Trp Leu Ala Phe Ile Asn Lys Leu Gln Asp
   20 25
<210> 61
   <211> 29
   <212> PRT
   <213> Artificial Sequence
<220>
   <223> Modified bacterial sequence
<400> 61
   Glu Val Leu Glu Ala Trp Asn Glu Ile Asp Lys Leu Pro Asn Leu Thr
   1 5 10 15
   Leu Asp Gln Trp Leu Ala Phe Ile Asn Lys Leu Asp Asp
   20 25
<210> 62
   <211> 29
   <212> PRT
   <213> Artificial Sequence
<220>
   <223> Modified bacterial sequence
<400> 62
   Glu Val Ile Asp Ala Trp Asn Glu Ile Asp His Leu Pro Asn Leu Thr
   1 5 10 15
   Ile Glu Gln Trp Leu Ala Phe Ile Asn Lys Leu Ala Asp
   20 25
<210> 63
   <211> 29
   <212> PRT
   <213> Artificial Sequence
<220>
   <223> Modified bacterial sequence
<400> 63
   Glu Thr Ile Asp Ala Trp Asp Glu Ile Asp Lys Leu Pro Asn Leu Thr
   1               5                   10                  15
   Ile Glu Gln Trp Leu Ala Phe Ile Asn Lys Leu Asp Asp
               20                  25
<210> 64
   <211> 29
   <212> PRT
   <213> Artificial Sequence
<220>
   <223> Modified bacterial sequence
<400> 64
   Glu Val Ile Ala Ala Trp Asp Glu Ile Asp Leu Leu Pro Asn Leu Thr
   1               5                   10                  15
   Leu Gln Gln Trp Leu Ala Phe Ile Asn Lys Leu Ala Asp
               20                  25
<210> 65
   <211> 29
   <212> PRT
   <213> Artificial Sequence
<220>
   <223> Modified bacterial sequence
<400> 65
   Glu Val Ile His Ala Trp Asp Glu Ile Asp Lys Leu Pro Asn Leu Thr
   1               5                   10                  15
   Ile Glu Gln Trp Leu Ala Phe Ile Asn Lys Leu Asp Asp
               20                  25
<210> 66
   <211> 29
   <212> PRT
   <213> Artificial Sequence
<220>
   <223> Modified bacterial sequence
<400> 66
   Glu Val Ile Ala Ala Trp Asn Glu Ile Asp His Leu Pro Asn Leu Thr
   1               5                   10                  15
   Leu Glu Gln Trp Leu Ala Phe Ile Asn Lys Leu Asp Asp
                       20                  25
<210> 67
   <211> 29
   <212> PRT
   <213> Artificial Sequence
<220>
   <223> Modified bacterial sequence
<400> 67
   Glu Thr Leu Asp Ala Trp Asn Glu Ile Asp Lys Leu Pro Asn Leu Thr
   1               5                   10                  15
   Leu Ser Gln Trp Leu Ala Phe Ile Asn Lys Leu Asp Asp
               20                  25
<210> 68
   <211> 29
   <212> PRT
   <213> Artificial Sequence
<220>
   <223> Modified bacterial sequence
<400> 68
   Glu Val Leu Glu Ala Trp Asn Glu Ile Asp Ala Leu Pro Asn Leu Thr
   1               5                   10                  15
   Leu Glu Gln Trp Leu Ala Phe Ile Asn Lys Leu Asp Asp
               20                  25
<210> 69
   <211> 29
   <212> PRT
   <213> Artificial Sequence
<220>
   <223> Modified bacterial sequence
<400> 69
   Glu Val Ile Gln Ala Trp Asp Glu Ile Asp His Leu Pro Asn Leu Thr
   1               5                   10                  15
   Ile Ser Gln Trp Leu Ala Phe Ile Asn Lys Leu Asp Asp
               20                  25
<210> 70
   <211> 29
   <212> PRT
   <213> Artificial Sequence
<220>
   <223> Modified bacterial sequence
<400> 70
   Glu Val Leu Gln Ala Trp Asp Glu Ile Asp Ser Leu Pro Asn Leu Thr
   1               5                   10                  15
   Ile Glu Gln Trp Leu Ala Phe Ile Asn Lys Leu Asp Asp
               20                  25
<210> 71
   <211> 29
   <212> PRT
   <213> Artificial Sequence
<220>
   <223> Modified bacterial sequence
<400> 71
   Glu Thr Leu Glu Ala Trp Asp Glu Ile Asp His Leu Pro Asn Leu Thr
   1               5                   10                  15
   Ile Ala Gln Trp Leu Ala Phe Ile Asn Lys Leu Asp Asp
               20                  25
<210> 72
   <211> 29
   <212> PRT
   <213> Artificial Sequence
<220>
   <223> Modified bacterial sequence
<400> 72
   Glu Thr Ile Asp Ala Trp Asn Glu Ile Asp Arg Leu Pro Asn Leu Thr
   1               5                   10                  15
   Ile Ser Gln Trp Leu Ala Phe Ile Asn Lys Leu Asp Asp
               20                  25
<210> 73
   <211> 29
   <212> PRT
   <213> Artificial Sequence
<220>
   <223> Modified bacterial sequence
<400> 73
   Glu Val Leu Asp Ala Trp His Glu Ile Asp His Leu Pro Asn Leu Thr
   1               5                   10                  15
   Ile Gln Gln Trp Leu Ala Phe Ile Asn Lys Leu Asp Asp
                       20                  25
<210> 74
   <211> 29
   <212> PRT
   <213> Artificial Sequence
<220>
   <223> Modified bacterial sequence
<400> 74
   Glu Gln Ile Arg Ala Trp Asp Glu Ile Asp Lys Leu Pro Asn Leu Thr
   1               5                   10                  15
   Ile Glu Gln Trp Leu Ala Phe Ile Asn Lys Leu Ala Asp
               20                  25
<210> 75
   <211> 29
   <212> PRT
   <213> Artificial Sequence
<220>
   <223> Modified bacterial sequence
<400> 75
   Glu Thr Leu Tyr Ala Trp Asn Glu Ile Asp Lys Leu Pro Asn Leu Thr
   1               5                   10                  15
   Ile Glu Gln Trp Leu Ala Phe Ile Glu Lys Leu Gln Asp
               20                  25
<210> 76
   <211> 29
   <212> PRT
   <213> Artificial Sequence
<220>
   <223> Modified bacterial sequence
<400> 76
   Glu Val Ile Glu Ala Trp Asn Glu Ile Asp Ala Leu Pro Asn Leu Thr
   1               5                   10                  15
   Ile Asp Gln Trp Leu Ala Phe Ile Asn Lys Leu Asp Asp
               20                  25
<210> 77
   <211> 29
   <212> PRT
   <213> Artificial Sequence
<220>
   <223> Modified bacterial sequence
<400> 77
   Glu Val Leu Glu Ala Trp Asn Glu Ile Asp His Leu Pro Asn Leu Thr
   1               5                   10                  15
   Ile Gln Gln Trp Leu Ala Phe Ile Asn Lys Leu Asp Asp
               20                  25
<210> 78
   <211> 29
   <212> PRT
   <213> Artificial Sequence
<220>
   <223> Modified bacterial sequence
<400> 78
   Glu Thr Ile Glu Ala Trp Asp Glu Ile Asp Ala Leu Pro Asn Leu Thr
   1               5                   10                  15
   Ile Glu Gln Trp Leu Ala Phe Ile Asn Lys Leu Asp Asp
               20                  25
<210> 79
   <211> 29
   <212> PRT
   <213> Artificial Sequence
<220>
   <223> Modified bacterial sequence
<400> 79
   Glu Val Ile Glu Ala Trp Asn Glu Ile Asp His Leu Pro Asn Leu Thr
   1 5 10 15
   Leu Gln Gln Trp Leu Ala Phe Ile Asn Lys Leu Asp Asp
   20 25
<210> 80
   <211> 29
   <212> PRT
   <213> Artificial Sequence
<220>
   <223> Modified bacterial sequence
<400> 80
   Glu Val Ile Glu Ala Trp Asn Glu Ile Asp Lys Leu Pro Asn Leu Thr
   1 5 10 15
   Ile Gln Gln Trp Leu Ala Phe Ile Asn Lys Leu Asp Asp
   20 25
<210> 81
   <211> 29
   <212> PRT
   <213> Artificial Sequence
<220>
   <223> Modified bacterial sequence
<400> 81
   Glu Thr Leu Asp Ala Trp Ala Glu Ile Asp His Leu Pro Asn Leu Thr
   1 5 10 15
   Leu Asp Gln Trp Leu Ala Phe Ile Asn Lys Leu Asp Asp
   20 25
<210> 82
   <211> 29
   <212> PRT
   <213> Artificial Sequence
<220>
   <223> Modified bacterial sequence
<400> 82
   Glu His Ile Asp Ala Trp Asn Glu Ile Asp Ala Leu Pro Asn Leu Thr
   1               5                   10                  15
   Leu Ser Gln Trp Leu Ala Phe Ile Asn Lys Leu Asp Asp
               20                  25
<210> 83
   <211> 29
   <212> PRT
   <213> Artificial Sequence
<220>
   <223> Modified bacterial sequence
<400> 83
   Glu Val Leu Asp Ala Trp Asn Glu Ile Asp Lys Leu Pro Asn Leu Thr
   1               5                   10                  15
   Ile Ala Gln Trp Leu Ala Phe Ile Asn Lys Leu Asp Asp
               20                  25
<210> 84
   <211> 29
   <212> PRT
   <213> Artificial Sequence
<220>
   <223> Modified bacterial sequence
<400> 84
   Glu Val Ile Glu Ala Trp Thr Glu Ile Asp Tyr Leu Pro Asn Leu Thr
   1               5                   10                  15
   Leu Gln Gln Trp Leu Ala Phe Ile Asn Lys Leu Asp Asp
               20                  25
<210> 85
   <211> 29
   <212> PRT
   <213> Artificial Sequence
<220>
   <223> Modified bacterial sequence
<400> 85
   Glu Thr Ile Glu Ala Trp Asn Glu Ile Asp His Leu Pro Asn Leu Thr
   1               5                   10                  15
   Ile Ala Gln Trp Leu Ala Phe Ile Asn Lys Leu Asp Asp
               20                  25
<210> 86
   <211> 29
   <212> PRT
   <213> Artificial Sequence
<220>
   <223> Modified bacterial sequence
<400> 86
   Glu Val Ile Gln Ala Trp Asn Glu Ile Asp Lys Leu Pro Asn Leu Thr
   1               5                   10                  15
   Leu Glu Gln Trp Leu Ala Phe Ile Asn Lys Leu Asp Asp
               20                  25
<210> 87
   <211> 29
   <212> PRT
   <213> Artificial Sequence
<220>
   <223> Modified bacterial sequence
<400> 87
   Glu Val Ile Glu Ala Trp Asp Glu Ile Asp His Leu Pro Asn Leu Thr
   1               5                   10                  15
   Ile Glu Gln Trp Leu Ala Phe Ile Asn Lys Leu Asp Asp
                       20                  25
<210> 88
   <211> 29
   <212> PRT
   <213> Artificial Sequence
<220>
   <223> Modified bacterial sequence
<400> 88
   Glu Thr Ile Asp Ala Trp Asn Glu Ile Asp Leu Leu Pro Asn Leu Thr
   1               5                   10                  15
   Ile Glu Gln Trp Leu Ala Phe Ile Asn Lys Leu Asp Asp
               20                  25
<210> 89
   <211> 29
   <212> PRT
   <213> Artificial Sequence
<220>
   <223> Modified bacterial sequence
<400> 89
   Glu His Ile Asp Ala Trp Asn Glu Ile Asp Lys Leu Pro Asn Leu Thr
   1               5                   10                  15
   Leu Asp Gln Trp Leu Ala Phe Ile Asn Lys Leu Asp Asp
               20                  25
<210> 90
   <211> 29
   <212> PRT
   <213> Artificial Sequence
<220>
   <223> Modified bacterial sequence
<400> 90
   Glu Val Val Ala Ala Trp Asn Glu Ile Asp Ala Leu Pro Asn Leu Thr
   1               5                   10                  15
   Ile Glu Gln Trp Leu Ala Phe Ile Asn Lys Leu Asn Asp
               20                  25
<210> 91
   <211> 29
   <212> PRT
   <213> Artificial Sequence
<220>
   <223> Modified bacterial sequence
<400> 91
   Glu Val Ile Glu Ala Trp Asn Glu Ile Asp Ala Leu Pro Asn Leu Thr
   1               5                   10                  15
   Leu Ala Gln Trp Leu Ala Phe Ile Asn Lys Leu Asp Asp
               20                  25
<210> 92
   <211> 29
   <212> PRT
   <213> Artificial Sequence
<220>
   <223> Modified bacterial sequence
<400> 92
   Glu Val Leu Gln Ala Trp Asp Glu Ile Asp Arg Leu Pro Asn Leu Thr
   1               5                   10                  15
   Leu Asp Gln Trp Leu Ala Phe Ile Asn Lys Leu Asp Asp
               20                  25
<210> 93
   <211> 29
   <212> PRT
   <213> Artificial Sequence
<220>
   <223> Modified bacterial sequence
<400> 93
   Glu Val Ile Asp Ala Trp Asp Glu Ile Asp His Leu Pro Asn Leu Thr
   1               5                   10                  15
   Ile Glu Gln Trp Leu Ala Phe Ile Asn Lys Leu Ser Asp
               20                  25
<210> 94
   <211> 29
   <212> PRT
   <213> Artificial Sequence
<220>
   <223> Modified bacterial sequence
<400> 94
   Glu Val Val Glu Ala Trp Asn Glu Ile Asp Gln Leu Pro Asn Leu Thr
   1               5                   10                  15
   Ile Glu Gln Trp Leu Ala Phe Ile Asn Lys Leu Asp Asp
                        20                  25
<210> 95
   <211> 29
   <212> PRT
   <213> Artificial Sequence
<220>
   <223> Modified bacterial sequence
<400> 95
   Glu Val Ile Gln Ala Trp Asn Glu Ile Asp Ala Leu Pro Asn Leu Thr
   1               5                   10                  15
   Ile Glu Gln Trp Leu Ala Phe Ile Asn Lys Leu Asp Asp
               20                  25
<210> 96
   <211> 29
   <212> PRT
   <213> Artificial Sequence
<220>
   <223> Modified bacterial sequence
<400> 96
   Glu Val Ile Gln Ala Trp Asp Glu Ile Asp Lys Leu Pro Asn Leu Thr
   1               5                   10                  15
   Ile Asp Gln Trp Leu Ala Phe Ile Asn Lys Leu Ala Asp
               20                  25
<210> 97
   <211> 29
   <212> PRT
   <213> Artificial Sequence
<220>
   <223> Modified bacterial sequence
<400> 97
   Glu Val Val Ala Ala Trp Asp Glu Ile Asp Ala Leu Pro Asn Leu Thr
   1               5                   10                  15
   Leu Thr Gln Trp Leu Ala Phe Ile Asn Lys Leu Asp Asp
               20                  25
<210> 98
   <211> 29
   <212> PRT
   <213> Artificial Sequence
<220>
   <223> Modified bacterial sequence
<400> 98
   Glu Val Ile Gln Ala Trp Asn Glu Ile Asp Gly Leu Pro Asn Leu Thr
   1               5                   10                  15
   Leu Ser Gln Trp Leu Ala Phe Ile Asn Lys Leu Asp Asp
               20                  25
<210> 99
   <211> 29
   <212> PRT
   <213> Artificial Sequence
<220>
   <223> Modified bacterial sequence
<400> 99
   Glu Thr Ile Glu Ala Trp Asp Glu Ile Asp Ala Leu Pro Asn Leu Thr
   1               5                   10                  15
   Ile Thr Gln Trp Leu Ala Phe Ile Asn Lys Leu Asp Asp
               20                  25
<210> 100
   <211> 29
   <212> PRT
   <213> Artificial Sequence
<220>
   <223> Modified bacterial sequence
<400> 100
   Glu Val Ile Asp Ala Trp Asn Glu Ile Asp His Leu Pro Asn Leu Thr
   1               5                   10                  15
   Ile Gln Gln Trp Leu Ala Phe Ile Asn Lys Leu Ala Asp
               20                  25
<210> 101
   <211> 29
   <212> PRT
   <213> Artificial Sequence
<220>
   <223> Modified bacterial sequence
<400> 101
   Glu Thr Ile Glu Ala Trp Asn Glu Ile Asp Ala Leu Pro Asn Leu Thr
   1               5                   10                  15
   Leu Asp Gln Trp Leu Ala Phe Ile Asn Lys Leu Glu Asp
                       20                  25
<210> 102
   <211> 29
   <212> PRT
   <213> Artificial Sequence
<220>
   <223> Modified bacterial sequence
<400> 102
   Glu His Ile His Ala Trp Asn Glu Ile Asp Glu Leu Pro Asn Leu Thr
   1               5                   10                  15
   Ile Glu Gln Trp Leu Ala Phe Ile Asn Lys Leu Ala Asp
               20                  25
<210> 103
   <211> 29
   <212> PRT
   <213> Artificial Sequence
<220>
   <223> Modified bacterial sequence
<400> 103
   Glu Val Ile Asp Ala Trp Asp Glu Ile Asp His Leu Pro Asn Leu Thr
   1               5                   10                  15
   Ile Asp Gln Trp Leu Ala Phe Ile Asn Lys Leu Ser Asp
               20                  25
<210> 104
   <211> 29
   <212> PRT
   <213> Artificial Sequence
<220>
   <223> Modified bacterial sequence
<400> 104
   Glu Val Ile Asp Ala Asn Asp Glu Ile Asp Ala Leu Pro Asn Leu Thr
   1               5                   10                  15
   Ile Ala Gln Trp Leu Ala Phe Ile Asn Lys Leu His Asp
               20                  25
<210> 105
   <211> 29
   <212> PRT
   <213> Artificial Sequence
<220>
   <223> Modified bacterial sequence
<400> 105
   Glu Thr Ile Glu Ala Trp Asp Glu Ile Asp Lys Leu Pro Asn Leu Thr
   1 5 10 15
   Ile Glu Gln Trp Leu Ala Phe Ile Asn Lys Leu Asp Asp
   20 25
<210> 106
   <211> 29
   <212> PRT
   <213> Artificial Sequence
<220>
   <223> Modified bacterial sequence
<400> 106
   Glu Val Leu Leu Ala Trp Asp Glu Ile Asp His Leu Pro Asn Leu Thr
   1 5 10 15
   Leu Glu Gln Trp Leu Ala Phe Ile Asn Lys Leu Asp Asp
   20 25
<210> 107
   <211> 29
   <212> PRT
   <213> Artificial Sequence
<220>
   <223> Modified bacterial sequence
<400> 107
   Glu His Ile Asp Ala Trp Asn Glu Ile Asp Gly Leu Pro Asn Leu Thr
   1 5 10 15
   Leu Glu Gln Trp Leu Ala Phe Ile Asn Lys Leu Asp Asp
   20 25
<210> 108
   <211> 29
   <212> PRT
   <213> Artificial Sequence
<220>
   <223> Modified bacterial sequence
<400> 108
   Glu Val Ile Glu Ala Trp Ser Glu Ile Asp Ala Leu Pro Asn Leu Thr
   1 5 10 15
   Ile Asp Gln Trp Leu Ala Phe Ile Asn Lys Leu Ala Asp
   20 25
<210> 109
   <211> 29
   <212> PRT
   <213> Artificial Sequence
<220>
   <223> Modified bacterial sequence
<400> 109
   Glu Gln Leu Asn Ala Trp Ala Glu Ile Asp Ala Leu Pro Asn Leu Thr
   1 5 10 15
   Ile Glu Gln Trp Leu Ala Phe Ile Asn Lys Leu Asp Asp
   20 25
<210> 110
   <211> 29
   <212> PRT
   <213> Artificial Sequence
<220>
   <223> Modified bacterial sequence
<400> 110
   Glu Val Ile Asp Ala Trp Asn Glu Ile Asp Ala Leu Pro Asn Leu Thr
   1 5 10 15
   Ile Ala Gln Trp Leu Ala Phe Ile Asn Lys Leu Asp Asp
   20 25
<210> 111
   <211> 29
   <212> PRT
   <213> Artificial Sequence
<220>
   <223> Modified bacterial sequence
<400> 111
   Glu Thr Ile Asp Ala Trp Asn Glu Ile Asp Gln Leu Pro Asn Leu Thr
   1 5 10 15
   Ile Glu Gln Trp Leu Ala Phe Ile Asn Lys Leu Asp Asp
   20 25
<210> 112
   <211> 29
   <212> PRT
   <213> Artificial Sequence
<220>
   <223> Modified bacterial sequence
<400> 112
   Glu Val Ile Glu Ala Trp Asp Glu Ile Asp Lys Leu Pro Asn Leu Thr
   1               5                   10                  15
   Leu Ala Gln Trp Leu Ala Phe Ile Asn Lys Leu Asp Asp
               20                  25
<210> 113
   <211> 29
   <212> PRT
   <213> Artificial Sequence
<220>
   <223> Modified bacterial sequence
<400> 113
   Glu Val Leu Tyr Ala Trp Ala Glu Ile Asp His Leu Pro Asn Leu Thr
   1               5                   10                  15
   Ile Glu Gln Trp Leu Ala Phe Ile Asn Lys Leu Asp Asp
               20                  25
<210> 114
   <211> 29
   <212> PRT
   <213> Artificial Sequence
<220>
   <223> Modified bacterial sequence
<400> 114
   Glu Gln Ile Asp Ala Trp Asn Glu Ile Asp Arg Leu Pro Asn Leu Thr
   1               5                   10                  15
   Ile Gln Gln Trp Leu Ala Phe Ile Asn Lys Leu Asp Asp
               20                  25
<210> 115
   <211> 29
   <212> PRT
   <213> Artificial Sequence
<220>
   <223> Modified bacterial sequence
<400> 115
   Glu Val Leu Ala Ala Trp Asp Glu Ile Asp Arg Leu Pro Asn Leu Thr
   1               5                   10                  15
   Ile Glu Gln Trp Leu Ala Phe Ile Asn Lys Leu Asp Asp
                       20                  25
<210> 116
   <211> 29
   <212> PRT
   <213> Artificial Sequence
<220>
   <223> Modified bacterial sequence
<400> 116
   Glu Val Ile Glu Ala Trp Asp Glu Ile Asp His Leu Pro Asn Leu Thr
   1               5                   10                  15
   Leu His Gln Trp Leu Ala Phe Ile Asn Lys Leu Asp Asp
               20                  25
<210> 117
   <211> 29
   <212> PRT
   <213> Artificial Sequence
<220>
   <223> Modified bacterial sequence
<400> 117
   Glu Val Ile Glu Ala Trp Asn Glu Ile Asp Lys Leu Pro Asn Leu Thr
   1               5                   10                  15
   Leu Gln Gln Trp Leu Ala Phe Ile Asn Lys Leu Asp Asp
               20                  25
<210> 118
   <211> 29
   <212> PRT
   <213> Artificial Sequence
<220>
   <223> Modified bacterial sequence
<400> 118
   Glu Val Ile Asp Ala Asn Asp Glu Ile Asp Ala Leu Pro Asn Leu Thr
   1               5                   10                  15
   Ile Glu Gln Trp Leu Ala Phe Ile Asn Lys Leu His Asp
               20                  25
<210> 119
   <211> 29
   <212> PRT
   <213> Artificial Sequence
<220>
   <223> Modified bacterial sequence
<400> 119
   Glu Val Ile Ala Ala Trp Asp Glu Ile Asp Ala Leu Pro Asn Leu Thr
   1               5                   10                  15
   Ile Glu Gln Trp Leu Ala Phe Ile Asn Lys Leu Asp Asp
               20                  25
<210> 120
   <211> 29
   <212> PRT
   <213> Artificial Sequence
<220>
   <223> Modified bacterial sequence
<400> 120
   Glu Val Ile Glu Ala Trp Thr Glu Ile Asp Gln Leu Pro Asn Leu Thr
   1               5                   10                  15
   Leu Asp Gln Trp Leu Ala Phe Ile Asn Lys Leu Asp Asp
               20                  25
<210> 121
   <211> 29
   <212> PRT
   <213> Artificial Sequence
<220>
   <223> Modified bacterial sequence
<400> 121
   Glu Val Ile Asn Ala Trp Asn Glu Ile Asp Ala Leu Pro Asn Leu Thr
   1               5                   10                  15
   Leu Gln Gln Trp Leu Ala Phe Ile Asn Lys Leu Asp Asp
               20                  25
<210> 122
   <211> 29
   <212> PRT
   <213> Artificial Sequence
<220>
   <223> Modified bacterial sequence
<400> 122
   Glu His Ile Glu Ala Trp Asp Glu Ile Asp His Leu Pro Asn Leu Thr
   1               5                   10                  15
   Ile Asp Gln Trp Leu Ala Phe Ile Asn Lys Leu Ala Asp
                       20                  25
<210> 123
   <211> 29
   <212> PRT
   <213> Artificial Sequence
<220>
   <223> Modified bacterial sequence
<400> 123
   Glu His Leu Glu Ala Trp Arg Glu Ile Asp Ala Leu Pro Asn Leu Thr
   1               5                   10                  15
   Ile Glu Gln Trp Leu Ala Phe Ile Asn Lys Leu Asp Asp
               20                  25
<210> 124
   <211> 29
   <212> PRT
   <213> Artificial Sequence
<220>
   <223> Modified bacterial sequence
<400> 124
   Glu Val Leu Asp Ala Trp Asn Glu Ile Asp Lys Leu Pro Asn Leu Thr
   1               5                   10                  15
   Leu Gln Gln Trp Leu Ala Phe Ile Asn Lys Leu Asp Asp
               20                  25
<210> 125
   <211> 29
   <212> PRT
   <213> Artificial Sequence
<220>
   <223> Modified bacterial sequence
<400> 125
   Glu Val Ile Ala Ala Trp Asp Glu Ile Asp His Leu Pro Asn Leu Thr
   1               5                   10                  15
   Ile Gln Gln Trp Leu Ala Phe Ile Asn Lys Leu Asp Asp
               20                  25
<210> 126
   <211> 29
   <212> PRT
   <213> Artificial Sequence
<220>
   <223> Modified bacterial sequence
<400> 126
   Glu Val Ile Gln Ala Trp Asn Glu Ile Asp Ala Leu Pro Asn Leu Thr
   1               5                   10                  15
   Leu Glu Gln Trp Leu Ala Phe Ile Asn Lys Leu Asp Asp
               20                  25
<210> 127
   <211> 29
   <212> PRT
   <213> Artificial Sequence
<220>
   <223> Modified bacterial sequence
<400> 127
   Glu Val Ile Asp Ala Trp Asn Glu Ile Asp His Leu Pro Asn Leu Thr
   1               5                   10                  15
   Ile Ala Gln Trp Leu Ala Phe Ile Asn Lys Leu Asp Asp
               20                  25
<210> 128
   <211> 29
   <212> PRT
   <213> Artificial Sequence
<220>
   <223> Modified bacterial sequence
<400> 128
   Glu Gln Leu Asp Ala Trp Asp Glu Ile Asp His Leu Pro Asn Leu Thr
   1 5 10 15
   Ile Asp Gln Trp Leu Ala Phe Ile Asn Lys Leu Ser Asp
   20 25
<210> 129
   <211> 29
   <212> PRT
   <213> Artificial Sequence
<220>
   <223> Modified bacterial sequence
<400> 129
   Glu Val Leu Asn Ala Trp Asp Glu Ile Asp Lys Leu Pro Asn Leu Thr
   1 5 10 15
   Ile Glu Gln Trp Leu Ala Phe Ile Asn Lys Leu Asp Asp
   20 25
<210> 130
   <211> 29
   <212> PRT
   <213> Artificial Sequence
<220>
   <223> Modified bacterial sequence
<400> 130
   Glu Val Leu Glu Ala Trp Asn Glu Ile Asp His Leu Pro Asn Leu Thr
   1 5 10 15
   Ile Asp Gln Trp Leu Ala Phe Ile Asn Lys Leu Asp Asp
   20 25
<210> 131
   <211> 29
   <212> PRT
   <213> Artificial Sequence
<220>
   <223> Modified bacterial sequence
<400> 131
   Glu Val Leu Leu Ala Trp Asp Glu Ile Asp Arg Leu Pro Asn Leu Thr
   1 5 10 15
   Ile Asp Gln Trp Leu Ala Phe Ile Asn Lys Leu Ala Asp
   20 25
<210> 132
   <211> 29
   <212> PRT
   <213> Artificial Sequence
<220>
   <223> Modified bacterial sequence
<400> 132
   Glu Val Ile Ala Ala Trp Asn Glu Ile Asp Gln Leu Pro Asn Leu Thr
   1 5 10 15
   Leu Asp Gln Trp Leu Ala Phe Ile Asn Lys Leu Asp Asp
   20 25
<210> 133
   <211> 29
   <212> PRT
   <213> Artificial Sequence
<220>
   <223> Modified bacterial sequence
<400> 133
   Glu Thr Leu Leu Ala Trp Asp Glu Ile Asp Ala Leu Pro Asn Leu Thr
   1 5 10 15
   Ile Glu Gln Trp Leu Ala Phe Ile Asn Lys Leu Asp Asp
   20 25
<210> 134
   <211> 29
   <212> PRT
   <213> Artificial Sequence
<220>
   <223> Modified bacterial sequence
<400> 134
   Glu Val Ile Asp Ala Trp Asn Glu Ile Asp Thr Leu Pro Asn Leu Thr
   1 5 10 15
   Leu Glu Gln Trp Leu Ala Phe Ile Asn Lys Leu Asp Asp
   20 25
<210> 135
   <211> 29
   <212> PRT
   <213> Artificial Sequence
<220>
   <223> Modified bacterial sequence
<400> 135
   Glu Val Leu His Ala Trp Asn Glu Ile Asp His Leu Pro Asn Leu Thr
   1               5                   10                  15
   Leu Asn Gln Trp Leu Ala Phe Ile Asn Lys Leu Gln Asp
               20                  25
<210> 136
   <211> 29
   <212> PRT
   <213> Artificial Sequence
<220>
   <223> Modified bacterial sequence
<400> 136
   Glu Val Ile Gln Ala Trp Asn Glu Ile Asp Ala Leu Pro Asn Leu Thr
   1               5                   10                  15
   Ile Ala Gln Trp Leu Ala Phe Ile Asn Lys Leu Asp Asp
                       20                  25
<210> 137
   <211> 29
   <212> PRT
   <213> Artificial Sequence
<220>
   <223> Modified bacterial sequence
<400> 137
   Glu Thr Val Asp Ala Trp Asn Glu Ile Asp Ala Leu Pro Asn Leu Thr
   1               5                   10                  15
   Ile Glu Gln Trp Leu Ala Phe Ile Asn Lys Leu Asp Asp
               20                  25
<210> 138
   <211> 29
   <212> PRT
   <213> Artificial Sequence
<220>
   <223> Modified bacterial sequence
<400> 138
   Glu Val Ile Gln Ala Trp Asp Glu Ile Asp His Leu Pro Asn Leu Thr
   1 5 10 15
   Ile Asp Gln Trp Leu Ala Phe Ile Asn Lys Leu Asp Asp
   20 25
<210> 139
   <211> 29
   <212> PRT
   <213> Artificial Sequence
<220>
   <223> Modified bacterial sequence
<400> 139
   Glu Val Leu Asp Ala Trp Asn Glu Ile Asp Gln Leu Pro Asn Leu Thr
   1 5 10 15
   Ile Gln Gln Trp Leu Ala Phe Ile Asn Lys Leu Asp Asp
   20 25
<210> 140
   <211> 29
   <212> PRT
   <213> Artificial Sequence
<220>
   <223> Modified bacterial sequence
<400> 140
   Glu Thr Ile Glu Ala Trp Asn Glu Ile Asp Ala Leu Pro Asn Leu Thr
   1 5 10 15
   Leu Asp Gln Trp Leu Ala Phe Ile Asn Lys Leu Asp Asp
   20 25
<210> 141
   <211> 29
   <212> PRT
   <213> Artificial Sequence
<220>
   <223> Modified bacterial sequence
<400> 141
   Glu Val Ile Glu Ala Trp Asp Glu Ile Asp Ala Leu Pro Asn Leu Thr
   1               5                   10                  15
   Ile Asp Gln Trp Leu Ala Phe Ile Asn Lys Leu Asp Asp
               20                  25
<210> 142
   <211> 29
   <212> PRT
   <213> Artificial Sequence
<220>
   <223> Modified bacterial sequence
<400> 142
   Glu Val Ile Glu Ala Trp Asn Glu Ile Asp Gln Leu Pro Asn Leu Thr
   1               5                   10                  15
   Ile Gln Gln Trp Leu Ala Phe Ile Asn Lys Leu Asp Asp
               20                  25
<210> 143
   <211> 29
   <212> PRT
   <213> Artificial Sequence
<220>
   <223> Modified bacterial sequence
<400> 143
   Glu Val Ile Glu Ala Trp Thr Glu Ile Asp His Leu Pro Asn Leu Thr
   1               5                   10                  15
   Ile Glu Gln Trp Leu Ala Phe Ile Asn Lys Leu Asp Asp
                       20                  25
<210> 144
   <211> 29
   <212> PRT
   <213> Artificial Sequence
<220>
   <223> Modified bacterial sequence
<400> 144
   Glu Val Ile Gln Ala Trp Asn Glu Ile Asp His Leu Pro Asn Leu Thr
   1               5                   10                  15
   Leu Gln Gln Trp Leu Ala Phe Ile Asn Lys Leu Glu Asp
               20                  25
<210> 145
   <211> 29
   <212> PRT
   <213> Artificial Sequence
<220>
   <223> Modified bacterial sequence
<400> 145
   Glu Val Ile Gln Ala Asn Asn Glu Ile Asp Gln Leu Pro Asn Leu Thr
   1               5                   10                  15
   Ile Glu Gln Trp Leu Ala Phe Ile Asn Lys Leu His Asp
               20                  25
<210> 146
   <211> 29
   <212> PRT
   <213> Artificial Sequence
<220>
   <223> Modified bacterial sequence
<400> 146
   Glu Val Leu His Ala Trp Ser Glu Ile Asp Lys Leu Pro Asn Leu Thr
   1               5                   10                  15
   Ile Glu Gln Trp Leu Ala Phe Ile Asn Lys Leu Asp Asp
               20                  25
<210> 147
   <211> 29
   <212> PRT
   <213> Artificial Sequence
<220>
   <223> Modified bacterial sequence
<400> 147
   Glu Thr Ile Gln Ala Trp Asp Glu Ile Asp Lys Leu Pro Asn Leu Thr
   1               5                   10                  15
   Leu Asp Gln Trp Leu Ala Phe Ile Asn Lys Leu Ser Asp
               20                  25
<210> 148
   <211> 29
   <212> PRT
   <213> Artificial Sequence
<220>
   <223> Modified bacterial sequence
<400> 148
   Glu Thr Leu Arg Ala Trp Asp Glu Ile Asp Lys Leu Pro Asn Leu Thr
   1               5                   10                  15
   Ile Gln Gln Trp Leu Ala Phe Ile Asn Lys Leu Ala Asp
               20                  25
<210> 149
   <211> 29
   <212> PRT
   <213> Artificial Sequence
<220>
   <223> Modified bacterial sequence
<400> 149
   Glu Val Ile Asp Ala Trp Asn Glu Ile Asp His Leu Pro Asn Leu Thr
   1               5                   10                  15
   Ile Glu Gln Trp Leu Ala Phe Ile Asn Lys Leu Glu Asp
               20                  25
<210> 150
   <211> 29
   <212> PRT
   <213> Artificial Sequence
<220>
   <223> Modified bacterial sequence
<400> 150
   Glu Val Ile Asp Ala Trp Asn Glu Ile Asp His Leu Pro Asn Leu Thr
   1               5                   10                  15
   Leu Gln Gln Trp Leu Ala Phe Ile Asn Lys Leu Ala Asp
                        20                  25
<210> 151
   <211> 29
   <212> PRT
   <213> Artificial Sequence
<220>
   <223> Modified bacterial sequence
<400> 151
   Glu Thr Ile Asp Ala Trp Asn Glu Ile Asp Ala Leu Pro Asn Leu Thr
   1               5                   10                  15
   Leu Asp Gln Trp Leu Ala Phe Ile Asn Lys Leu Asp Asp
               20                  25
<210> 152
   <211> 29
   <212> PRT
   <213> Artificial Sequence
<220>
   <223> Modified bacterial sequence
<400> 152
   Glu Val Ile Glu Ala Trp Asn Glu Ile Asp Gln Leu Pro Asn Leu Thr
   1               5                   10                  15
   Ile Glu Gln Trp Leu Ala Phe Ile Asn Lys Leu Asp Asp
               20                  25
<210> 153
   <211> 29
   <212> PRT
   <213> Artificial Sequence
<220>
   <223> Modified bacterial sequence
<400> 153
   Glu Val Ile Arg Ala Trp Asp Glu Ile Asp Gln Leu Pro Asn Leu Thr
   1               5                   10                  15
   Leu Ser Gln Trp Leu Ala Phe Ile Asn Lys Leu Asp Asp
               20                  25
<210> 154
   <211> 29
   <212> PRT
   <213> Artificial Sequence
<220>
   <223> Modified bacterial sequence
<400> 154
   Glu Val Ile Glu Ala Trp Asn Glu Ile Asp Arg Leu Pro Asn Leu Thr
   1 5 10 15
   Ile His Gln Trp Leu Ala Phe Ile Asn Lys Leu Asp Asp
   20 25
<210> 155
   <211> 29
   <212> PRT
   <213> Artificial Sequence
<220>
   <223> Modified bacterial sequence
<400> 155
   Glu Thr Ile Glu Ala Trp Asn Glu Ile Asp Gln Leu Pro Asn Leu Thr
   1 5 10 15
   Ile Glu Gln Trp Leu Ala Phe Ile Asn Lys Leu Asp Asp
   20 25
<210> 156
   <211> 29
   <212> PRT
   <213> Artificial Sequence
<220>
   <223> Modified bacterial sequence
<400> 156
   Glu Val Leu Thr Ala Trp Ala Glu Ile Asp Ala Leu Pro Asn Leu Thr
   1 5 10 15
   Leu Ser Gln Trp Leu Ala Phe Ile Asn Lys Leu Asp Asp
   20 25
<210> 157
   <211> 29
   <212> PRT
   <213> Artificial Sequence
<220>
   <223> Modified bacterial sequence
<400> 157
   Glu Val Ile Glu Ala Trp Asp Glu Ile Asp Lys Leu Pro Asn Leu Thr
   1 5 10 15
   Val Asp Gln Trp Leu Ala Phe Ile Asn Lys Leu Asp Asp
   20 25
<210> 158
   <211> 29
   <212> PRT
   <213> Artificial Sequence
<220>
   <223> Modified bacterial sequence
<400> 158
   Glu Val Ile Asp Ala Trp Asn Glu Ile Asp His Leu Pro Asn Leu Thr
   1 5 10 15
   Leu Thr Gln Trp Leu Ala Phe Ile Asn Lys Leu Asp Asp
   20 25
<210> 159
   <211> 29
   <212> PRT
   <213> Artificial Sequence
<220>
   <223> Modified bacterial sequence
<400> 159
   Glu Val Ile Glu Ala Trp Asn Glu Ile Asp Gln Leu Pro Asn Leu Thr
   1 5 10 15
   Leu Asp Gln Trp Leu Ala Phe Ile Asn Lys Leu Asp Asp
   20 25
<210> 160
   <211> 29
   <212> PRT
   <213> Artificial Sequence
<220>
   <223> Modified bacterial sequence
<400> 160
   Glu Thr Leu Gln Ala Trp Asp Glu Ile Asp His Leu Pro Asn Leu Thr
   1 5 10 15
   Leu Asn Gln Trp Leu Ala Phe Ile Asn Lys Leu Asp Asp
   20 25
<210> 161
   <211> 29
   <212> PRT
   <213> Artificial Sequence
<220>
   <223> Modified bacterial sequence
<400> 161
   Glu Val Ile Asp Ala Trp Asn Glu Ile Asp His Leu Pro Asn Leu Thr
   1               5                   10                  15
   Ile Glu Gln Trp Leu Ala Phe Ile Asn Lys Leu Asp Asp
               20                  25
<210> 162
   <211> 29
   <212> PRT
   <213> Artificial Sequence
<220>
   <223> Modified bacterial sequence
<400> 162
   Glu Val Ile Glu Ala Trp Asn Glu Ile Asp Leu Leu Pro Asn Leu Thr
   1               5                   10                  15
   Leu Ser Gln Trp Leu Ala Phe Ile Asn Lys Leu Asp Asp
               20                  25
<210> 163
   <211> 29
   <212> PRT
   <213> Artificial Sequence
<220>
   <223> Modified bacterial sequence
<400> 163
   Glu Val Ile Asp Ala Trp Asp Glu Ile Asp Arg Leu Pro Asn Leu Thr
   1               5                   10                  15
   Leu Lys Gln Trp Leu Ala Phe Ile Asn Lys Leu Asp Asp
               20                  25
<210> 164
   <211> 29
   <212> PRT
   <213> Artificial Sequence
<220>
   <223> Modified bacterial sequence
<400> 164
   Glu Thr Leu His Ala Trp Asp Glu Ile Asp Lys Leu Pro Asn Leu Thr
   1               5                   10                  15
   Ile Glu Gln Trp Leu Ala Phe Ile Asn Lys Leu Asp Asp
                       20                  25
<210> 165
   <211> 29
   <212> PRT
   <213> Artificial Sequence
<220>
   <223> Modified bacterial sequence
<400> 165
   Glu Val Ile Lys Ala Trp Asp Glu Ile Asp His Leu Pro Asn Leu Thr
   1               5                   10                  15
   Leu Asn Gln Trp Leu Ala Phe Ile Asn Lys Leu Asp Asp
               20                  25
<210> 166
   <211> 29
   <212> PRT
   <213> Artificial Sequence
<220>
   <223> Modified bacterial sequence
<400> 166
   Glu Val Ile Glu Ala Trp Asn Glu Ile Asp His Leu Pro Asn Leu Thr
   1               5                   10                  15
   Leu Ala Gln Trp Leu Ala Phe Ile Asn Lys Leu Asp Asp
               20                  25
<210> 167
   <211> 29
   <212> PRT
   <213> Artificial Sequence
<220>
   <223> Modified bacterial sequence
<400> 167
   Glu Val Ile Gln Ala Trp Asn Glu Ile Asp His Leu Pro Asn Leu Thr
   1               5                   10                  15
   Ile Asp Gln Trp Leu Ala Phe Ile Thr Lys Leu Glu Asp
               20                  25
<210> 168
   <211> 29
   <212> PRT
   <213> Artificial Sequence
<220>
   <223> Modified bacterial sequence
<400> 168
   Glu Val Ile Glu Ala Trp Asn Glu Ile Asp Arg Leu Pro Asn Leu Thr
   1               5                   10                  15
   Ile Lys Gln Trp Leu Ala Phe Ile Asn Lys Leu Asp Asp
               20                  25
<210> 169
   <211> 29
   <212> PRT
   <213> Artificial Sequence
<220>
   <223> Modified bacterial sequence
<400> 169
   Glu Val Ile Glu Ala Trp Asn Glu Ile Asp Ser Leu Pro Asn Leu Thr
   1               5                   10                  15
   Leu Gln Gln Trp Leu Ala Phe Ile Asn Lys Leu Asp Asp
               20                  25
<210> 170
   <211> 29
   <212> PRT
   <213> Artificial Sequence
<220>
   <223> Modified bacterial sequence
<400> 170
   Glu Thr Ile Asp Ala Trp Asn Glu Ile Asp Lys Leu Pro Asn Leu Thr
   1               5                   10                  15
   Ile Glu Gln Trp Leu Ala Phe Ile Asn Lys Leu Asp Asp
               20                  25
<210> 171
   <211> 29
   <212> PRT
   <213> Artificial Sequence
<220>
   <223> Modified bacterial sequence
<400> 171
   Glu Val Leu Glu Ala Trp Ala Glu Ile Asp Ala Leu Pro Asn Leu Thr
   1               5                   10                  15
   Ile Ala Gln Trp Leu Ala Phe Ile Asn Lys Leu Asp Asp
                        20                  25
<210> 172
   <211> 29
   <212> PRT
   <213> Artificial Sequence
<220>
   <223> Modified bacterial sequence
<400> 172
   Glu Thr Ile Asp Ala Trp Asn Glu Ile Asp Arg Leu Pro Asn Leu Thr
   1               5                   10                  15
   Ile Glu Gln Trp Leu Ala Phe Ile Asn Lys Leu Asp Asp
               20                  25
<210> 173
   <211> 29
   <212> PRT
   <213> Artificial Sequence
<220>
   <223> Modified bacterial sequence
<400> 173
   Glu Thr Leu Lys Ala Trp Asp Glu Ile Asp Arg Leu Pro Asn Leu Thr
   1               5                   10                  15
   Leu Glu Gln Trp Leu Ala Phe Ile Asn Lys Leu Asp Asp
               20                  25
<210> 174
   <211> 29
   <212> PRT
   <213> Artificial Sequence
<220>
   <223> Modified bacterial sequence
<400> 174
   Glu Thr Ile Ala Ala Trp Asn Glu Ile Asp Ala Leu Pro Asn Leu Thr
   1               5                   10                  15
   Leu Gln Gln Trp Leu Ala Phe Ile Asn Lys Leu Asp Asp
               20                  25
<210> 175
   <211> 29
   <212> PRT
   <213> Artificial Sequence
<220>
   <223> Modified bacterial sequence
<400> 175
   Glu Val Leu Gln Ala Trp Asn Glu Ile Asp His Leu Pro Asn Leu Thr
   1               5                   10                  15
   Ile Gln Gln Trp Leu Ala Phe Ile Asn Lys Leu Asp Asp
               20                  25
<210> 176
   <211> 29
   <212> PRT
   <213> Artificial Sequence
<220>
   <223> Modified bacterial sequence
<400> 176
   Glu Val Ile Glu Ala Trp Ser Glu Ile Asp His Leu Pro Asn Leu Thr
   1               5                   10                  15
   Leu Gln Gln Trp Leu Ala Phe Ile Asn Lys Leu Asp Asp
               20                  25
<210> 177
   <211> 29
   <212> PRT
   <213> Artificial Sequence
<220>
   <223> Modified bacterial sequence
<400> 177
   Glu Val Ile Asp Ala Trp Asn Glu Ile Asp Gly Leu Pro Asn Leu Thr
   1               5                   10                  15
   Ile Glu Gln Trp Leu Ala Phe Ile Asn Lys Leu Asp Asp
               20                  25
<210> 178
   <211> 29
   <212> PRT
   <213> Artificial Sequence
<220>
   <223> Modified bacterial sequence
<400> 178
   Glu Val Ile His Ala Trp Asn Glu Ile Asp His Leu Pro Asn Leu Thr
   1               5                   10                  15
   Leu Asn Gln Trp Leu Ala Phe Ile Asn Lys Leu Glu Asp
                       20                  25
<210> 179
   <211> 29
   <212> PRT
   <213> Artificial Sequence
<220>
   <223> Modified bacterial sequence
<400> 179
   Glu Val Leu Asp Ala Trp Asn Glu Ile Asp Ser Leu Pro Asn Leu Thr
   1               5                   10                  15
   Leu Asp Gln Trp Leu Ala Phe Ile Asn Lys Leu Asp Asp
               20                  25
<210> 180
   <211> 29
   <212> PRT
   <213> Artificial Sequence
<220>
   <223> Modified bacterial sequence
<400> 180
   Glu Gln Ile Glu Ala Trp Asn Glu Ile Asp Arg Leu Pro Asn Leu Thr
   1               5                   10                  15
   Leu Glu Gln Trp Leu Ala Phe Ile Asn Lys Leu Asp Asp
               20                  25
<210> 181
   <211> 29
   <212> PRT
   <213> Artificial Sequence
<220>
   <223> Modified bacterial sequence
<400> 181
   Glu Val Val Asp Ala Trp Asn Glu Ile Asp Ala Leu Pro Asn Leu Thr
   1               5                   10                  15
   Leu Gln Gln Trp Leu Ala Phe Ile Asn Lys Leu Asp Asp
               20                  25
<210> 182
   <211> 29
   <212> PRT
   <213> Artificial Sequence
<220>
   <223> Modified bacterial sequence
<400> 182
   Glu Val Ile Glu Ala Trp Asn Glu Ile Asp Lys Leu Pro Asn Leu Thr
   1               5                   10                  15
   Ile Glu Gln Trp Leu Ala Phe Ile Asn Lys Leu Asp Asp
               20                  25
<210> 183
   <211> 29
   <212> PRT
   <213> Artificial Sequence
<220>
   <223> Modified bacterial sequence
<400> 183
   Glu Val Ile Glu Ala Asn Asp Glu Ile Asp Arg Leu Pro Asn Leu Thr
   1               5                   10                  15
   Ile Glu Gln Trp Leu Ala Phe Ile Asn Lys Leu His Asp
               20                  25
<210> 184
   <211> 29
   <212> PRT
   <213> Artificial Sequence
<220>
   <223> Modified bacterial sequence
<400> 184
   Glu Thr Leu Gln Ala Trp Asp Glu Ile Asp Lys Leu Pro Asn Leu Thr
   1               5                   10                  15
   Ile Glu Gln Trp Leu Ala Phe Ile Asn Lys Leu Asp Asp
               20                  25
<210> 185
   <211> 29
   <212> PRT
   <213> Artificial Sequence
<220>
   <223> Modified bacterial sequence
<400> 185
   Glu Val Ile Glu Ala Trp Asp Glu Ile Asp His Leu Pro Asn Leu Thr
   1               5                   10                  15
   Ile Asp Gln Trp Leu Ala Phe Ile Asn Lys Leu Ala Asp
               20                  25
<210> 186
   <211> 29
   <212> PRT
   <213> Artificial Sequence
<220>
   <223> Modified bacterial sequence
<400> 186
   Glu Thr Ile Asp Ala Trp Asn Glu Ile Asp His Leu Pro Asn Leu Thr
   1               5                   10                  15
   Leu Gln Gln Trp Leu Ala Phe Ile Asn Lys Leu Ala Asp
               20                  25
<210> 187
   <211> 29
   <212> PRT
   <213> Artificial Sequence
<220>
   <223> Modified bacterial sequence
<400> 187
   Glu Val Ile Asp Ala Trp Asp Glu Ile Asp Lys Leu Pro Asn Leu Thr
   1               5                   10                  15
   Ile Glu Gln Trp Leu Ala Phe Ile Asn Lys Leu Asp Asp
               20                  25
<210> 188
   <211> 29
   <212> PRT
   <213> Artificial Sequence
<220>
   <223> Modified bacterial sequence
<400> 188
   Glu Val Ile Glu Ala Trp Asn Glu Ile Asp Lys Leu Pro Asn Leu Thr
   1               5                   10                  15
   Leu Ala Gln Trp Leu Ala Phe Ile Asn Lys Leu Asp Asp
               20                  25
<210> 189
   <211> 29
   <212> PRT
   <213> Artificial Sequence
<220>
   <223> Modified bacterial sequence
<400> 189
   Glu Val Leu Gln Ala Trp Asp Glu Ile Asp Lys Leu Pro Asn Leu Thr
   1               5                   10                  15
   Ile Gln Gln Trp Leu Ala Phe Ile Asn Lys Leu Asp Asp
               20                  25
<210> 190
   <211> 29
   <212> PRT
   <213> Artificial Sequence
<220>
   <223> Modified bacterial sequence
<400> 190
   Glu Val Ile Ala Ala Trp Asn Glu Ile Asp Gly Leu Pro Asn Leu Thr
   1 5 10 15
   Leu Gln Gln Trp Leu Ala Phe Ile Asn Lys Leu Asp Asp
   20 25
<210> 191
   <211> 29
   <212> PRT
   <213> Artificial Sequence
<220>
   <223> Modified bacterial sequence
<400> 191
   Glu Thr Leu Asn Ala Trp Asn Glu Ile Asp Ala Leu Pro Asn Leu Thr
   1 5 10 15
   Leu Gln Gln Trp Leu Ala Phe Ile Asn Lys Leu Asp Asp
   20 25
<210> 192
   <211> 29
   <212> PRT
   <213> Artificial Sequence
<220>
   <223> Modified bacterial sequence
<400> 192
   Glu Val Leu Ser Ala Trp Asn Glu Ile Asp Gln Leu Pro Asn Leu Thr
   1 5 10 15
   Leu Glu Gln Trp Leu Ala Phe Ile Asn Lys Leu Asp Asp
   20 25
<210> 193
   <211> 29
   <212> PRT
   <213> Artificial Sequence
<220>
   <223> Modified bacterial sequence
<400> 193
   Glu Thr Leu Glu Ala Trp Asp Glu Ile Asp His Leu Pro Asn Leu Thr
   1               5                   10                  15
   Leu His Gln Trp Leu Ala Phe Ile Asn Lys Leu Asp Asp
               20                  25
<210> 194
   <211> 29
   <212> PRT
   <213> Artificial Sequence
<220>
   <223> Modified bacterial sequence
<400> 194
   Glu Gln Ile Glu Ala Trp Asn Glu Ile Asp His Leu Pro Asn Leu Thr
   1               5                   10                  15
   Leu Gln Gln Trp Leu Ala Phe Ile Asn Lys Leu Ala Asp
               20                  25
<210> 195
   <211> 29
   <212> PRT
   <213> Artificial Sequence
<220>
   <223> Modified bacterial sequence
<400> 195
   Glu Val Val Glu Ala Trp Asp Glu Ile Asp Lys Leu Pro Asn Leu Thr
   1               5                   10                  15
   Ile Glu Gln Trp Leu Ala Phe Ile Asn Lys Leu Asp Asp
               20                  25
<210> 196
   <211> 29
   <212> PRT
   <213> Artificial Sequence
<220>
   <223> Modified bacterial sequence
<400> 196
   Glu Val Leu Glu Ala Trp Asn Glu Ile Asp Glu Leu Pro Asn Leu Thr
   1               5                   10                  15
   Ile Glu Gln Trp Leu Ala Phe Ile Asn Lys Leu Asp Asp
               20                  25
<210> 197
   <211> 29
   <212> PRT
   <213> Artificial Sequence
<220>
   <223> Modified bacterial sequence
<400> 197
   Glu Val Ile Asp Ala Trp Asn Glu Ile Asp Gln Leu Pro Asn Leu Thr
   1 5 10 15
   Leu Gln Gln Trp Leu Ala Phe Ile Asn Lys Leu Asp Asp
   20 25
<210> 198
   <211> 29
   <212> PRT
   <213> Artificial Sequence
<220>
   <223> Modified bacterial sequence
<400> 198
   Glu Thr Ile Asp Ala Trp Asp Glu Ile Asp Lys Leu Pro Asn Leu Thr
   1 5 10 15
   Leu Ser Gln Trp Leu Ala Phe Ile Asn Lys Leu Asp Asp
   20 25
<210> 199
   <211> 29
   <212> PRT
   <213> Artificial Sequence
<220>
   <223> Modified bacterial sequence
<400> 199
   Glu Thr Ile Asp Ala Trp Asn Glu Ile Asp Gln Leu Pro Asn Leu Thr
   1 5 10 15
   Leu Gln Gln Trp Leu Ala Phe Ile Asn Lys Leu Asp Asp
   20 25
<210> 200
   <211> 29
   <212> PRT
   <213> Artificial Sequence
<220>
   <223> Modified bacterial sequence
<400> 200
   Glu Val Ile Gln Ala Trp Asp Glu Ile Asp Ala Leu Pro Asn Leu Thr
   1               5                   10                  15
   Leu Asn Gln Trp Leu Ala Phe Ile Asn Lys Leu Asp Asp
               20                  25
<210> 201
   <211> 29
   <212> PRT
   <213> Artificial Sequence
<220>
   <223> Modified bacterial sequence
<400> 201
   Glu Val Leu Asp Ala Trp Ala Glu Ile Asp Gln Leu Pro Asn Leu Thr
   1               5                   10                  15
   Leu Gln Gln Trp Leu Ala Phe Ile Asn Lys Leu Asp Asp
               20                  25
<210> 202
   <211> 29
   <212> PRT
   <213> Artificial Sequence
<220>
   <223> Modified bacterial sequence
<400> 202
   Glu His Ile Ala Ala Trp Asp Glu Ile Asp Ala Leu Pro Asn Leu Thr
   1               5                   10                  15
   Ile Glu Gln Trp Leu Ala Phe Ile Asn Lys Leu Asp Asp
               20                  25
<210> 203
   <211> 29
   <212> PRT
   <213> Artificial Sequence
<220>
   <223> Modified bacterial sequence
<400> 203
   Glu Val Ile Arg Ala Trp Asp Glu Ile Asp Ala Leu Pro Asn Leu Thr
   1 5 10 15
   Ile Glu Gln Trp Leu Ala Phe Ile Asn Lys Leu Asp Asp
   20 25
<210> 204
   <211> 29
   <212> PRT
   <213> Artificial Sequence
<220>
   <223> Modified bacterial sequence
<400> 204
   Glu Val Ile Asp Ala Trp Asp Glu Ile Asp Ala Leu Pro Asn Leu Thr
   1 5 10 15
   Ile Asp Gln Trp Leu Ala Phe Ile Asn Lys Leu Ala Asp
   20 25
<210> 205
   <211> 29
   <212> PRT
   <213> Artificial Sequence
<220>
   <223> Modified bacterial sequence
<400> 205
   Glu Val Ile Asp Ala Trp Asn Glu Ile Asp Arg Leu Pro Asn Leu Thr
   1 5 10 15
   Ile Gln Gln Trp Leu Ala Phe Ile Asn Lys Leu Asp Asp
   20 25
<210> 206
   <211> 29
   <212> PRT
   <213> Artificial Sequence
<220>
   <223> Modified bacterial sequence
<400> 206
   Glu Val Ile Thr Ala Trp Asn Glu Ile Asp His Leu Pro Asn Leu Thr
   1 5 10 15
   Leu Ser Gln Trp Leu Ala Phe Ile Asn Lys Leu Asp Asp
   20 25
<210> 207
   <211> 29
   <212> PRT
   <213> Artificial Sequence
<220>
   <223> Modified bacterial sequence
<400> 207
   Glu Val Ile Asp Ala Trp Asn Glu Ile Asp Ala Leu Pro Asn Leu Thr
   1 5 10 15
   Ile His Gln Trp Leu Ala Phe Ile Asn Lys Leu Asp Asp
   20 25
<210> 208
   <211> 29
   <212> PRT
   <213> Artificial Sequence
<220>
   <223> Modified bacterial sequence
<400> 208
   Glu Gln Leu Lys Ala Trp Asp Glu Ile Asp Lys Leu Pro Asn Leu Thr
   1 5 10 15
   Ile Glu Gln Trp Leu Ala Phe Ile Glu Lys Leu Gln Asp
   20 25
<210> 209
   <211> 29
   <212> PRT
   <213> Artificial Sequence
<220>
   <223> Modified bacterial sequence
<400> 209
   Glu His Ile Asp Ala Trp Thr Glu Ile Asp His Leu Pro Asn Leu Thr
   1 5 10 15
   Ile Glu Gln Trp Leu Ala Phe Ile Asn Lys Leu Asp Asp
   20 25
<210> 210
   <211> 29
   <212> PRT
   <213> Artificial Sequence
<220>
   <223> Modified bacterial sequence
<400> 210
   Glu Gln Leu Arg Ala Trp Asp Glu Ile Asp Lys Leu Pro Asn Leu Thr
   1               5                   10                  15
   Ile Glu Gln Trp Leu Ala Phe Ile Asn Lys Leu Gln Asp
               20                  25
<210> 211
   <211> 29
   <212> PRT
   <213> Artificial Sequence
<220>
   <223> Modified bacterial sequence
<400> 211
   Glu Val Leu Glu Ala Trp Arg Glu Ile Asp Ser Leu Pro Asn Leu Thr
   1               5                   10                  15
   Ile Ala Gln Trp Leu Ala Phe Ile Asn Lys Leu Asp Asp
               20                  25
<210> 212
   <211> 29
   <212> PRT
   <213> Artificial Sequence
<220>
   <223> Modified bacterial sequence
<400> 212
   Glu Val Ile Gln Ala Trp Asn Glu Ile Asp Lys Leu Pro Asn Leu Thr
   1               5                   10                  15
   Ile Glu Gln Trp Leu Ala Phe Ile Asn Lys Leu Asp Asp
               20                  25
<210> 213
   <211> 29
   <212> PRT
   <213> Artificial Sequence
<220>
   <223> Modified bacterial sequence
<400> 213
   Glu His Val Glu Ala Trp Asn Glu Ile Asp Gln Leu Pro Asn Leu Thr
   1               5                   10                  15
   Ile Glu Gln Trp Leu Ala Phe Ile Asn Lys Leu Ala Asp
                       20                  25
<210> 214
   <211> 29
   <212> PRT
   <213> Artificial Sequence
<220>
   <223> Modified bacterial sequence
<400> 214
   Glu Val Ile Asp Ala Trp Asp Glu Ile Asp Ala Leu Pro Asn Leu Thr
   1               5                   10                  15
   Ile Asp Gln Trp Leu Ala Phe Ile Asn Lys Leu Ser Asp
               20                  25
<210> 215
   <211> 29
   <212> PRT
   <213> Artificial Sequence
<220>
   <223> Modified bacterial sequence
<400> 215
   Glu Val Ile Glu Ala Trp Asn Glu Ile Asp His Leu Pro Asn Leu Thr
   1               5                   10                  15
   Ile Glu Gln Trp Leu Ala Phe Ile Asn Lys Leu Asp Asp
               20                  25
<210> 216
   <211> 29
   <212> PRT
   <213> Artificial Sequence
<220>
   <223> Modified bacterial sequence
<400> 216
   Glu Val Leu Gln Ala Trp Asp Glu Ile Asp Lys Leu Pro Asn Leu Thr
   1 5 10 15
   Ile Glu Gln Trp Leu Ala Phe Ile Asn Lys Leu Ser Asp
   20 25
<210> 217
   <211> 29
   <212> PRT
   <213> Artificial Sequence
<220>
   <223> Modified bacterial sequence
<400> 217
   Glu Val Ile Lys Ala Trp Asn Glu Ile Asp Ser Leu Pro Asn Leu Thr
   1 5 10 15
   Ile Glu Gln Trp Leu Ala Phe Ile Asn Lys Leu Asp Asp
   20 25
<210> 218
   <211> 29
   <212> PRT
   <213> Artificial Sequence
<220>
   <223> Modified bacterial sequence
<400> 218
   Glu Val Leu Glu Ala Trp His Glu Ile Asp Leu Leu Pro Asn Leu Thr
   1 5 10 15
   Ile Gln Gln Trp Leu Ala Phe Ile Asn Lys Leu Asp Asp
   20 25
<210> 219
   <211> 29
   <212> PRT
   <213> Artificial Sequence
<220>
   <223> Modified bacterial sequence
<400> 219
   Glu Val Leu Glu Ala Trp Thr Glu Ile Asp Arg Leu Pro Asn Leu Thr
   1 5 10 15
   Leu Asp Gln Trp Leu Ala Phe Ile Asn Lys Leu Asp Asp
   20 25
<210> 220
   <211> 29
   <212> PRT
   <213> Artificial Sequence
<220>
   <223> Modified bacterial sequence
<400> 220
   Glu Gln Leu Tyr Ala Trp Asn Glu Ile Asp His Leu Pro Asn Leu Thr
   1               5                   10                  15
   Ile Glu Gln Trp Leu Ala Phe Ile Glu Lys Leu Gln Asp
                       20                  25
<210> 221
   <211> 29
   <212> PRT
   <213> Artificial Sequence
<220>
   <223> Modified bacterial sequence
<400> 221
   Glu Val Leu Asn Ala Trp Asp Glu Ile Asp Lys Leu Pro Asn Leu Thr
   1               5                   10                  15
   Ile Lys Gln Trp Leu Ala Phe Ile Asn Lys Leu Asp Asp
               20                  25
<210> 222
   <211> 29
   <212> PRT
   <213> Artificial Sequence
<220>
   <223> Modified bacterial sequence
<400> 222
   Glu Val Ile Arg Ala Trp Asp Glu Ile Asp Lys Leu Pro Asn Leu Thr
   1               5                   10                  15
   Val Glu Gln Trp Leu Ala Phe Ile Asn Lys Leu Asp Asp
               20                  25
<210> 223
   <211> 29
   <212> PRT
   <213> Artificial Sequence
<220>
   <223> Modified bacterial sequence
<400> 223
   Glu Val Val Gln Ala Trp Asp Glu Ile Asp Gln Leu Pro Asn Leu Thr
   1               5                   10                  15
   Leu Glu Gln Trp Leu Ala Phe Ile Asn Lys Leu Asp Asp
               20                  25
<210> 224
   <211> 29
   <212> PRT
   <213> Artificial Sequence
<220>
   <223> Modified bacterial sequence
<400> 224
   Glu Val Ile Arg Ala Trp Asp Glu Ile Asp Gln Leu Pro Asn Leu Thr
   1               5                   10                  15
   Leu Glu Gln Trp Leu Ala Phe Ile Asn Lys Leu Asp Asp
               20                  25
<210> 225
   <211> 29
   <212> PRT
   <213> Artificial Sequence
<220>
   <223> Modified bacterial sequence
<400> 225
   Glu Thr Ile Asp Ala Trp Asn Glu Ile Asp His Leu Pro Asn Leu Thr
   1               5                   10                  15
   Leu Asp Gln Trp Leu Ala Phe Ile Asn Lys Leu Asp Asp
               20                  25
<210> 226
   <211> 29
   <212> PRT
   <213> Artificial Sequence
<220>
   <223> Modified bacterial sequence
<400> 226
   Glu Val Val Ala Ala Trp Thr Glu Ile Asp Leu Leu Pro Asn Leu Thr
   1 5 10 15
   Leu Asp Gln Trp Leu Ala Phe Ile Asn Lys Leu Glu Asp
   20 25
<210> 227
   <211> 29
   <212> PRT
   <213> Artificial Sequence
<220>
   <223> Modified bacterial sequence
<400> 227
   Glu Val Val Ala Ala Trp Asp Glu Ile Asp Ala Leu Pro Asn Leu Thr
   1 5 10 15
   Ile Glu Gln Trp Leu Ala Phe Ile Asn Lys Leu Ser Asp
   20 25
<210> 228
   <211> 29
   <212> PRT
   <213> Artificial Sequence
<220>
   <223> Modified bacterial sequence
<400> 228
   Glu Thr Leu Glu Ala Trp Arg Glu Ile Asp Ser Leu Pro Asn Leu Thr
   1 5 10 15
   Leu Glu Gln Trp Leu Ala Phe Ile Asn Lys Leu Asp Asp
   20 25
<210> 229
   <211> 29
   <212> PRT
   <213> Artificial Sequence
<220>
   <223> Modified bacterial sequence
<400> 229
   Glu Val Ile Lys Ala Trp Asn Glu Ile Asp His Leu Pro Asn Leu Thr
   1               5                   10                  15
   Leu Asp Gln Trp Leu Ala Phe Ile Asn Lys Leu Asp Asp
               20                  25
<210> 230
   <211> 29
   <212> PRT
   <213> Artificial Sequence
<220>
   <223> Modified bacterial sequence
<400> 230
   Glu Val Leu Glu Ala Trp Thr Glu Ile Asp Lys Leu Pro Asn Leu Thr
   1               5                   10                  15
   Ile Asp Gln Trp Leu Ala Phe Ile Asn Lys Leu Asp Asp
               20                  25
<210> 231
   <211> 29
   <212> PRT
   <213> Artificial Sequence
<220>
   <223> Modified bacterial sequence
<400> 231
   Glu Thr Leu Glu Ala Trp Asp Glu Ile Asp Lys Leu Pro Asn Leu Thr
   1               5                   10                  15
   Ile Asp Gln Trp Leu Ala Phe Ile Asn Lys Leu Asp Asp
               20                  25
<210> 232
   <211> 29
   <212> PRT
   <213> Artificial Sequence
<220>
   <223> Modified bacterial sequence
<400> 232
   Glu Val Ile Glu Ala Trp Asn Glu Ile Asp Lys Leu Pro Asn Leu Thr
   1               5                   10                  15
   Ile Asp Gln Trp Leu Ala Phe Ile Asn Lys Leu Asp Asp
               20                  25
<210> 233
   <211> 29
   <212> PRT
   <213> Artificial Sequence
<220>
   <223> Modified bacterial sequence
<400> 233
   Glu Thr Ile Asp Ala Trp Asn Glu Ile Asp Lys Leu Pro Asn Leu Thr
   1               5                   10                  15
   Leu Glu Gln Trp Leu Ala Phe Ile Asn Lys Leu Asp Asp
               20                  25
<210> 234
   <211> 29
   <212> PRT
   <213> Artificial Sequence
<220>
   <223> Modified bacterial sequence
<400> 234
   Glu Thr Leu Asp Ala Trp Asp Glu Ile Asp Ala Leu Pro Asn Leu Thr
   1               5                   10                  15
   Ile Asp Gln Trp Leu Ala Phe Ile Asn Lys Leu Glu Asp
                       20                  25
<210> 235
   <211> 29
   <212> PRT
   <213> Artificial Sequence
<220>
   <223> Modified bacterial sequence
<400> 235
   Glu Val Leu Ser Ala Trp Asn Glu Ile Asp His Leu Pro Asn Leu Thr
   1               5                   10                  15
   Ile Gln Gln Trp Leu Ala Phe Ile Asn Lys Leu Asp Asp
               20                  25
<210> 236
   <211> 29
   <212> PRT
   <213> Artificial Sequence
<220>
   <223> Modified bacterial sequence
<400> 236
   Glu Val Ile Gln Ala Asn Asp Glu Ile Asp Lys Leu Pro Asn Leu Thr
   1               5                   10                  15
   Ile Glu Gln Trp Leu Ala Phe Ile His Lys Leu His Asp
               20                  25
<210> 237
   <211> 29
   <212> PRT
   <213> Artificial Sequence
<220>
   <223> Modified bacterial sequence
<400> 237
   Glu His Leu Asp Ala Trp Asp Glu Ile Asp His Leu Pro Asn Leu Thr
   1               5                   10                  15
   Ile Gln Gln Trp Leu Ala Phe Ile Asn Lys Leu Ala Asp
               20                  25
<210> 238
   <211> 29
   <212> PRT
   <213> Artificial Sequence
<220>
   <223> Modified bacterial sequence
<400> 238
   Glu Val Ile Gln Ala Trp Asn Glu Ile Asp Gln Leu Pro Asn Leu Thr
   1               5                   10                  15
   Ile Glu Gln Trp Leu Ala Phe Ile Asn Lys Leu Asp Asp
               20                  25
<210> 239
   <211> 29
   <212> PRT
   <213> Artificial Sequence
<220>
   <223> Modified bacterial sequence
<400> 239
   Glu Val Ile Glu Ala Trp Asn Glu Ile Asp Tyr Leu Pro Asn Leu Thr
   1 5 10 15
   Ile Ala Gln Trp Ile Ala Phe Ile Asn Lys Leu Asp Asp
   20 25
<210> 240
   <211> 29
   <212> PRT
   <213> Artificial Sequence
<220>
   <223> Modified bacterial sequence
<400> 240
   Glu Thr Ile Gln Ala Trp Asp Glu Ile Asp Arg Leu Pro Asn Leu Thr
   1 5 10 15
   Leu Gln Gln Trp Leu Ala Phe Ile Asn Lys Leu Asp Asp
   20 25
<210> 241
   <211> 29
   <212> PRT
   <213> Artificial Sequence
<220>
   <223> Modified bacterial sequence
<400> 241
   Glu Thr Ile Gln Ala Trp Asp Glu Ile Asp Lys Leu Pro Asn Leu Thr
   1 5 10 15
   Ile Glu Gln Trp Leu Ala Phe Ile Asn Lys Leu Asp Asp
   20 25
<210> 242
   <211> 29
   <212> PRT
   <213> Artificial Sequence
<220>
   <223> Modified bacterial sequence
<400> 242
   Glu Thr Leu Asp Ala Trp Ala Glu Ile Asp His Leu Pro Asn Leu Thr
   1 5 10 15
   Ile Glu Gln Trp Leu Ala Phe Ile Asn Lys Leu Asp Asp
   20 25
<210> 243
   <211> 29
   <212> PRT
   <213> Artificial Sequence
<220>
   <223> Modified bacterial sequence
<400> 243
   Glu Val Ile Glu Ala Trp Asp Glu Ile Asp Lys Leu Pro Asn Leu Thr
   1 5 10 15
   Leu Asn Gln Trp Leu Ala Phe Ile Asn Lys Leu Asp Asp
   20 25
<210> 244
   <211> 29
   <212> PRT
   <213> Artificial Sequence
<220>
   <223> Modified bacterial sequence
<400> 244
   Glu Val Leu Asp Ala Trp Asn Glu Ile Asp Gln Leu Pro Asn Leu Thr
   1 5 10 15
   Ile Glu Gln Trp Leu Ala Phe Ile Asn Lys Leu Asp Asp
   20 25
<210> 245
   <211> 29
   <212> PRT
   <213> Artificial Sequence
<220>
   <223> Modified bacterial sequence
<400> 245
   Glu Val Leu His Ala Trp Asn Glu Ile Asp His Leu Pro Asn Leu Thr
   1 5 10 15
   Ile Glu Gln Trp Leu Ala Phe Ile Glu Lys Leu Glu Asp
   20 25
<210> 246
   <211> 29
   <212> PRT
   <213> Artificial Sequence
<220>
   <223> Modified bacterial sequence
<400> 246
   Glu Val Ile Glu Ala Trp Gln Glu Ile Asp Lys Leu Pro Asn Leu Thr
   1               5                   10                  15
   Ile Asp Gln Trp Leu Ala Phe Ile Asn Lys Leu Asp Asp
               20                  25
<210> 247
   <211> 29
   <212> PRT
   <213> Artificial Sequence
<220>
   <223> Modified bacterial sequence
<400> 247
   Glu Val Val Asp Ala Trp Asn Glu Ile Asp Gln Leu Pro Asn Leu Thr
   1               5                   10                  15
   Ile Glu Gln Trp Leu Ala Phe Ile Asn Lys Leu Asp Asp
               20                  25
<210> 248
   <211> 29
   <212> PRT
   <213> Artificial Sequence
<220>
   <223> Modified bacterial sequence
<400> 248
   Glu Gln Ile Glu Ala Trp Asn Glu Ile Asp Ala Leu Pro Asn Leu Thr
   1               5                   10                  15
   Ile Glu Gln Trp Leu Ala Phe Ile Asn Lys Leu Ala Asp
                       20                  25
<210> 249
   <211> 108
   <212> PRT
   <213> Artificial Sequence
<220>
   <223> Modified bacterial sequence
<400> 249
   Ala Glu Ala Lys Tyr Ala Lys Glu Val Leu Glu Ala Trp Asp Glu Ile
   1               5                   10                  15
   Asp Arg Leu Pro Asn Leu Thr Ile Glu Gln Trp Leu Ala Phe Ile Asn
               20                  25                  30
   Lys Leu Asp Asp Asp Pro Ser Gln Ser Ser Glu Leu Leu Ser Glu Ala
           35                  40                  45
   Lys Lys Leu Asn Asp Ser Gln Ala Pro Lys Val Asp Gly Ser Leu Ala
       50                  55                  60
   Glu Ala Lys Glu Ala Ala Asn Ala Glu Leu Asp Ser Tyr Gly Val Ser
   65                  70                  75                  80
   Asp Phe Tyr Lys Arg Leu Ile Asp Lys Ala Lys Thr Val Glu Gly Val
                   85                  90                  95
   Glu Ala Leu Lys Asp Ala Ile Leu Ala Ala Leu Pro
               100                 105
<210> 250
   <211> 46
   <212> PRT
   <213> Artificial Sequence
<220>
   <223> Modified bacterial sequence
<400> 250
   Leu Ala Glu Ala Lys Glu Ala Ala Asn Ala Glu Leu Asp Ser Tyr Gly
   1               5                   10                  15
   Val Ser Asp Phe Tyr Lys Arg Leu Ile Asp Lys Ala Lys Thr Val Glu
               20                  25                  30
   Gly Val Glu Ala Leu Lys Asp Ala Ile Leu Ala Ala Leu Pro
           35                  40                  45
<210> 251
   <211> 1676
   <212> PRT
   <213> Homo sapiens
<400> 251
   Met Gly Leu Leu Gly Ile Leu Cys Phe Leu Ile Phe Leu Gly Lys Thr
   1               5                   10                  15
   Trp Gly Gln Glu Gln Thr Tyr Val Ile Ser Ala Pro Lys Ile Phe Arg
               20                  25                  30
   Val Gly Ala Ser Glu Asn Ile Val Ile Gln Val Tyr Gly Tyr Thr Glu
           35                  40                  45
   Ala Phe Asp Ala Thr Ile Ser Ile Lys Ser Tyr Pro Asp Lys Lys Phe
       50                  55                  60
   Ser Tyr Ser Ser Gly His Val His Leu Ser Ser Glu Asn Lys Phe Gln
   65                  70                  75                  80
   Asn Ser Ala Ile Leu Thr Ile Gln Pro Lys Gln Leu Pro Gly Gly Gln
                   85                  90                  95
   Asn Pro Val Ser Tyr Val Tyr Leu Glu Val Val Ser Lys His Phe Ser
               100                 105                 110
   Lys Ser Lys Arg Met Pro Ile Thr Tyr Asp Asn Gly Phe Leu Phe Ile
           115                 120                 125
   His Thr Asp Lys Pro Val Tyr Thr Pro Asp Gln Ser Val Lys Val Arg
       130                 135                 140
   Val Tyr Ser Leu Asn Asp Asp Leu Lys Pro Ala Lys Arg Glu Thr Val
   145                 150                 155                 160
   Leu Thr Phe Ile Asp Pro Glu Gly Ser Glu Val Asp Met Val Glu Glu
                   165                 170                 175
   Ile Asp His Ile Gly Ile Ile Ser Phe Pro Asp Phe Lys Ile Pro Ser
               180                 185                 190
   Asn Pro Arg Tyr Gly Met Trp Thr Ile Lys Ala Lys Tyr Lys Glu Asp
           195                 200                 205
   Phe Ser Thr Thr Gly Thr Ala Tyr Phe Glu Val Lys Glu Tyr Val Leu
       210                 215                 220
   Pro His Phe Ser Val Ser Ile Glu Pro Glu Tyr Asn Phe Ile Gly Tyr
   225                 230                 235                 240
   Lys Asn Phe Lys Asn Phe Glu Ile Thr Ile Lys Ala Arg Tyr Phe Tyr
                   245                 250                 255
   Asn Lys Val Val Thr Glu Ala Asp Val Tyr Ile Thr Phe Gly Ile Arg
               260                 265                 270
   Glu Asp Leu Lys Asp Asp Gln Lys Glu Met Met Gln Thr Ala Met Gln
           275                 280                 285
   Asn Thr Met Leu Ile Asn Gly Ile Ala Gln Val Thr Phe Asp Ser Glu
       290                 295                 300
   Thr Ala Val Lys Glu Leu Ser Tyr Tyr Ser Leu Glu Asp Leu Asn Asn
   305                 310                 315                 320
   Lys Tyr Leu Tyr Ile Ala Val Thr Val Ile Glu Ser Thr Gly Gly Phe
                   325                 330                 335
   Ser Glu Glu Ala Glu Ile Pro Gly Ile Lys Tyr Val Leu Ser Pro Tyr
               340                 345                 350
   Lys Leu Asn Leu Val Ala Thr Pro Leu Phe Leu Lys Pro Gly Ile Pro
           355                 360                 365
   Tyr Pro Ile Lys Val Gln Val Lys Asp Ser Leu Asp Gln Leu Val Gly
       370                 375                 380
   Gly Val Pro Val Thr Leu Asn Ala Gln Thr Ile Asp Val Asn Gln Glu
   385                 390                 395                 400
   Thr Ser Asp Leu Asp Pro Ser Lys Ser Val Thr Arg Val Asp Asp Gly
                   405                 410                 415
   Val Ala Ser Phe Val Leu Asn Leu Pro Ser Gly Val Thr Val Leu Glu
               420                 425                 430
   Phe Asn Val Lys Thr Asp Ala Pro Asp Leu Pro Glu Glu Asn Gln Ala
           435                 440                 445
   Arg Glu Gly Tyr Arg Ala Ile Ala Tyr Ser Ser Leu Ser Gln Ser Tyr
       450                 455                 460
   Leu Tyr Ile Asp Trp Thr Asp Asn His Lys Ala Leu Leu Val Gly Glu
   465                 470                 475                 480
   His Leu Asn Ile Ile Val Thr Pro Lys Ser Pro Tyr Ile Asp Lys Ile
                   485                 490                 495
   Thr His Tyr Asn Tyr Leu Ile Leu Ser Lys Gly Lys Ile Ile His Phe
               500                 505                 510
   Gly Thr Arg Glu Lys Phe Ser Asp Ala Ser Tyr Gln Ser Ile Asn Ile
           515                 520                 525
   Pro Val Thr Gln Asn Met Val Pro Ser Ser Arg Leu Leu Val Tyr Tyr
       530                 535                 540
   Ile Val Thr Gly Glu Gln Thr Ala Glu Leu Val Ser Asp Ser Val Trp
   545                 550                 555                 560
   Leu Asn Ile Glu Glu Lys Cys Gly Asn Gln Leu Gln Val His Leu Ser
                   565                 570                 575
   Pro Asp Ala Asp Ala Tyr Ser Pro Gly Gln Thr Val Ser Leu Asn Met
               580                 585                 590
   Ala Thr Gly Met Asp Ser Trp Val Ala Leu Ala Ala Val Asp Ser Ala
           595                 600                 605
   Val Tyr Gly Val Gln Arg Gly Ala Lys Lys Pro Leu Glu Arg Val Phe
       610                 615                 620
   Gln Phe Leu Glu Lys Ser Asp Leu Gly Cys Gly Ala Gly Gly Gly Leu
   625                 630                 635                 640
   Asn Asn Ala Asn Val Phe His Leu Ala Gly Leu Thr Phe Leu Thr Asn
                   645                 650                 655
   Ala Asn Ala Asp Asp Ser Gln Glu Asn Asp Glu Pro Cys Lys Glu Ile
               660                 665                 670
   Leu Arg Pro Arg Arg Thr Leu Gln Lys Lys Ile Glu Glu Ile Ala Ala
           675                 680                 685
   Lys Tyr Lys His Ser Val Val Lys Lys Cys Cys Tyr Asp Gly Ala Cys
       690                 695                 700
   Val Asn Asn Asp Glu Thr Cys Glu Gln Arg Ala Ala Arg Ile Ser Leu
   705                 710                 715                 720
   Gly Pro Arg Cys Ile Lys Ala Phe Thr Glu Cys Cys Val Val Ala Ser
                   725                 730                 735
   Gln Leu Arg Ala Asn Ile Ser His Lys Asp Met Gln Leu Gly Arg Leu
               740                 745                 750
   His Met Lys Thr Leu Leu Pro Val Ser Lys Pro Glu Ile Arg Ser Tyr
           755                 760                 765
   Phe Pro Glu Ser Trp Leu Trp Glu Val His Leu Val Pro Arg Arg Lys
       770                 775                 780
   Gln Leu Gln Phe Ala Leu Pro Asp Ser Leu Thr Thr Trp Glu Ile Gln
   785                 790                 795                 800
   Gly Val Gly Ile Ser Asn Thr Gly Ile Cys Val Ala Asp Thr Val Lys
                   805                 810                 815
   Ala Lys Val Phe Lys Asp Val Phe Leu Glu Met Asn Ile Pro Tyr Ser
               820                 825                 830
   Val Val Arg Gly Glu Gln Ile Gln Leu Lys Gly Thr Val Tyr Asn Tyr
           835                 840                 845
   Arg Thr Ser Gly Met Gln Phe Cys Val Lys Met Ser Ala Val Glu Gly
       850                 855                 860
   Ile Cys Thr Ser Glu Ser Pro Val Ile Asp His Gln Gly Thr Lys Ser
   865                 870                 875                 880
   Ser Lys Cys Val Arg Gln Lys Val Glu Gly Ser Ser Ser His Leu Val
                   885                 890                 895
   Thr Phe Thr Val Leu Pro Leu Glu Ile Gly Leu His Asn Ile Asn Phe
               900                 905                 910
   Ser Leu Glu Thr Trp Phe Gly Lys Glu Ile Leu Val Lys Thr Leu Arg
           915                 920                 925
   Val Val Pro Glu Gly Val Lys Arg Glu Ser Tyr Ser Gly Val Thr Leu
       930                 935                 940
   Asp Pro Arg Gly Ile Tyr Gly Thr Ile Ser Arg Arg Lys Glu Phe Pro
   945                 950                 955                 960
   Tyr Arg Ile Pro Leu Asp Leu Val Pro Lys Thr Glu Ile Lys Arg Ile
                   965                 970                 975
   Leu Ser Val Lys Gly Leu Leu Val Gly Glu Ile Leu Ser Ala Val Leu
   980 985 990
   Ser Gln Glu Gly Ile Asn Ile Leu Thr His Leu Pro Lys Gly Ser Ala
   995 1000 1005
   Glu Ala Glu Leu Met Ser Val Val Pro Val Phe Tyr Val Phe His
   1010 1015 1020
   Tyr Leu Glu Thr Gly Asn His Trp Asn Ile Phe His Ser Asp Pro
   1025 1030 1035
   Leu Ile Glu Lys Gln Lys Leu Lys Lys Lys Leu Lys Glu Gly Met
   1040 1045 1050
   Leu Ser Ile Met Ser Tyr Arg Asn Ala Asp Tyr Ser Tyr Ser Val
   1055 1060 1065
   Trp Lys Gly Gly Ser Ala Ser Thr Trp Leu Thr Ala Phe Ala Leu
   1070 1075 1080
   Arg Val Leu Gly Gln Val Asn Lys Tyr Val Glu Gln Asn Gln Asn
   1085 1090 1095
   Ser Ile Cys Asn Ser Leu Leu Trp Leu Val Glu Asn Tyr Gln Leu
   1100 1105 1110
   Asp Asn Gly Ser Phe Lys Glu Asn Ser Gln Tyr Gln Pro Ile Lys
   1115 1120 1125
   Leu Gln Gly Thr Leu Pro Val Glu Ala Arg Glu Asn Ser Leu Tyr
   1130 1135 1140
   Leu Thr Ala Phe Thr Val Ile Gly Ile Arg Lys Ala Phe Asp Ile
   1145 1150 1155
   Cys Pro Leu Val Lys Ile Asp Thr Ala Leu Ile Lys Ala Asp Asn
   1160 1165 1170
   Phe Leu Leu Glu Asn Thr Leu Pro Ala Gln Ser Thr Phe Thr Leu
   1175 1180 1185
   Ala Ile Ser Ala Tyr Ala Leu Ser Leu Gly Asp Lys Thr His Pro
   1190 1195 1200
   Gln Phe Arg Ser Ile Val Ser Ala Leu Lys Arg Glu Ala Leu Val
   1205 1210 1215
   Lys Gly Asn Pro Pro Ile Tyr Arg Phe Trp Lys Asp Asn Leu Gln
       1220                1225                1230
   His Lys Asp Ser Ser Val Pro Asn Thr Gly Thr Ala Arg Met Val
       1235                1240                1245
   Glu Thr Thr Ala Tyr Ala Leu Leu Thr Ser Leu Asn Leu Lys Asp
       1250                1255                1260
   Ile Asn Tyr Val Asn Pro Val Ile Lys Trp Leu Ser Glu Glu Gln
       1265                1270                1275
   Arg Tyr Gly Gly Gly Phe Tyr Ser Thr Gln Asp Thr Ile Asn Ala
       1280                1285                1290
   Ile Glu Gly Leu Thr Glu Tyr Ser Leu Leu Val Lys Gln Leu Arg
       1295                1300                1305
   Leu Ser Met Asp Ile Asp Val Ser Tyr Lys His Lys Gly Ala Leu
       1310                1315                1320
   His Asn Tyr Lys Met Thr Asp Lys Asn Phe Leu Gly Arg Pro Val
       1325                1330                1335
   Glu Val Leu Leu Asn Asp Asp Leu Ile Val Ser Thr Gly Phe Gly
       1340                1345                1350
   Ser Gly Leu Ala Thr Val His Val Thr Thr Val Val His Lys Thr
       1355                1360                1365
   Ser Thr Ser Glu Glu Val Cys Ser Phe Tyr Leu Lys Ile Asp Thr
       1370                1375                1380
   Gln Asp Ile Glu Ala Ser His Tyr Arg Gly Tyr Gly Asn Ser Asp
       1385                1390                1395
   Tyr Lys Arg Ile Val Ala Cys Ala Ser Tyr Lys Pro Ser Arg Glu
       1400                1405                1410
   Glu Ser Ser Ser Gly Ser Ser His Ala Val Met Asp Ile Ser Leu
       1415                1420                1425
   Pro Thr Gly Ile Ser Ala Asn Glu Glu Asp Leu Lys Ala Leu Val
       1430                1435                1440
   Glu Gly Val Asp Gln Leu Phe Thr Asp Tyr Gln Ile Lys Asp Gly
       1445                1450                1455
   His Val Ile Leu Gln Leu Asn Ser Ile Pro Ser Ser Asp Phe Leu
       1460                1465                1470
   Cys Val Arg Phe Arg Ile Phe Glu Leu Phe Glu Val Gly Phe Leu
       1475                1480                1485
   Ser Pro Ala Thr Phe Thr Val Tyr Glu Tyr His Arg Pro Asp Lys
       1490                1495                1500
   Gln Cys Thr Met Phe Tyr Ser Thr Ser Asn Ile Lys Ile Gln Lys
       1505                1510                1515
   Val Cys Glu Gly Ala Ala Cys Lys Cys Val Glu Ala Asp Cys Gly
       1520                1525                1530
   Gln Met Gln Glu Glu Leu Asp Leu Thr Ile Ser Ala Glu Thr Arg
       1535                1540                1545
   Lys Gln Thr Ala Cys Lys Pro Glu Ile Ala Tyr Ala Tyr Lys Val
       1550                1555                1560
   Ser Ile Thr Ser Ile Thr Val Glu Asn Val Phe Val Lys Tyr Lys
       1565                1570                1575
   Ala Thr Leu Leu Asp Ile Tyr Lys Thr Gly Glu Ala Val Ala Glu
       1580                1585                1590
   Lys Asp Ser Glu Ile Thr Phe Ile Lys Lys Val Thr Cys Thr Asn
       1595                1600                1605
   Ala Glu Leu Val Lys Gly Arg Gln Tyr Leu Ile Met Gly Lys Glu
       1610                1615                1620
   Ala Leu Gln Ile Lys Tyr Asn Phe Ser Phe Arg Tyr Ile Tyr Pro
       1625                1630                1635
   Leu Asp Ser Leu Thr Trp Ile Glu Tyr Trp Pro Arg Asp Thr Thr
       1640                1645                1650
   Cys Ser Ser Cys Gln Ala Phe Leu Ala Asn Leu Asp Glu Phe Ala
       1655                1660                1665
   Glu Asp Ile Phe Leu Asn Gly Cys
       1670                1675
<210> 252
   <211> 108
   <212> PRT
   <213> Artificial Sequence
<220>
   <223> Modified bacterial sequence
<400> 252
   Ala Glu Ala Lys Tyr Ala Lys Glu Val Leu Glu Ala Trp Asp Glu Ile
   1               5                   10                  15
   Asp Arg Leu Pro Asn Leu Thr Ile Glu Gln Trp Leu Ala Phe Ile Asn
               20                  25                  30
   Lys Leu Asp Arg Gln Pro Glu Gln Ser Ser Glu Leu Leu Ser Glu Ala
           35                  40                  45
   Lys Lys Leu Asn Asp Ser Gln Ala Pro Lys Val Asp Gly Ser Leu Ala
       50                  55                  60
   Glu Ala Lys Glu Ala Ala Asn Ala Glu Leu Asp Ser Tyr Gly Val Ser
   65                  70                  75                  80
   Asp Phe Tyr Lys Arg Leu Ile Asp Lys Ala Lys Thr Val Glu Gly Val
                   85                  90                  95
   Glu Ala Leu Lys Asp Ala Ile Leu Ala Ala Leu Pro
               100                 105
<210> 253
   <211> 108
   <212> PRT
   <213> Artificial Sequence
<220>
   <223> Modified bacterial sequence
<400> 253
   Ala Glu Ala Lys Tyr Ala Lys Glu Val Leu Glu Ala Trp Asp Glu Ile
   1               5                   10                  15
   Asp Arg Leu Pro Asn Leu Thr Ile Glu Gln Trp Leu Ala Phe Ile Asn
               20                  25                  30
   Lys Leu Asp Asp Asp Pro Ser Gln Ser Ser Glu Leu Leu Ser Glu Ala
           35                  40                  45
   Lys Lys Leu Ser Glu Ser Gln Ala Pro Lys Val Asp Gly Ser Leu Ala
       50                  55                  60
   Glu Ala Lys Glu Ala Ala Asn Ala Glu Leu Asp Ser Tyr Gly Val Ser
   65                  70                  75                  80
   Asp Phe Tyr Lys Arg Leu Ile Asp Lys Ala Lys Thr Val Glu Gly Val
                   85                  90                  95
   Glu Ala Leu Lys Asp Ala Ile Leu Ala Ala Leu Pro
               100                 105
<210> 254
   <211> 108
   <212> PRT
   <213> Artificial Sequence
<220>
   <223> Modified bacterial sequence
<400> 254
   Ala Glu Ala Lys Tyr Ala Lys Glu Val Leu Glu Ala Trp Asp Glu Ile
   1               5                   10                  15
   Asp Arg Leu Pro Asn Leu Thr Ile Glu Gln Trp Leu Ala Phe Ile Asn
               20                  25                  30
   Lys Leu Asp Asp Asp Pro Ser Gln Ser Ser Glu Leu Leu Ser Glu Ala
           35                  40                  45
   Lys Lys Leu Asn Asp Ser Gln Ala Pro Lys Val Asp Gly Ser Leu Ala
       50                  55                  60
   Glu Ala Lys Val Leu Ala Asn Arg Glu Leu Asp Lys Tyr Gly Val Ser
   65                  70                  75                  80
   Asp Phe Tyr Lys Arg Leu Ile Asn Lys Ala Lys Thr Val Glu Gly Val
                   85                  90                  95
   Glu Ala Leu Lys Leu His Ile Leu Ala Ala Leu Pro
               100                 105
<210> 255
   <211> 108
   <212> PRT
   <213> Artificial Sequence
<220>
   <223> Modified bacterial sequence
<400> 255
   Ala Glu Ala Lys Tyr Ala Lys Glu Val Leu Glu Ala Trp Ser Glu Ile
   1               5                   10                  15
   Asp Arg Leu Pro Asn Leu Thr Ile Glu Gln Trp Leu Ala Phe Ile Asn
               20                  25                  30
   Lys Leu Asp Asp Asp Pro Ser Gln Ser Ser Glu Leu Leu Ser Glu Ala
           35                  40                  45
   Lys Lys Leu Asn Asp Ser Gln Ala Pro Lys Val Asp Gly Ser Leu Ala
       50                  55                  60
   Glu Ala Lys Glu Ala Ala Asn Ala Glu Leu Asp Ser Tyr Gly Val Ser
   65                  70                  75                  80
   Asp Phe Tyr Lys Arg Leu Ile Asp Lys Ala Lys Thr Val Glu Gly Val
                   85                  90                  95
   Glu Ala Leu Lys Asp Ala Ile Leu Ala Ala Leu Pro
               100                 105
<210> 256
   <211> 108
   <212> PRT
   <213> Artificial Sequence
<220>
   <223> Modified bacterial sequence
<400> 256
   Ala Glu Ala Lys Tyr Ala Lys Glu Val Leu Glu Ala Trp Asp Glu Ile
   1               5                   10                  15
   Glu Arg Leu Pro Asn Leu Thr Ile Glu Gln Trp Leu Ala Phe Ile Asn
               20                  25                  30
   Lys Leu Asp Asp Asp Pro Ser Gln Ser Ser Glu Leu Leu Ser Glu Ala
           35                  40                  45
   Lys Lys Leu Asn Asp Ser Gln Ala Pro Lys Val Asp Gly Ser Leu Ala
       50                  55                  60
   Glu Ala Lys Glu Ala Ala Asn Ala Glu Leu Asp Ser Tyr Gly Val Ser
   65                  70                  75                  80
   Asp Phe Tyr Lys Arg Leu Ile Asp Lys Ala Lys Thr Val Glu Gly Val
                   85                  90                  95
   Glu Ala Leu Lys Asp Ala Ile Leu Ala Ala Leu Pro
               100                 105
<210> 257
   <211> 108
   <212> PRT
   <213> Artificial Sequence
<220>
   <223> Modified bacterial sequence
<400> 257
   Ala Glu Ala Lys Tyr Ala Lys Glu Val Leu Glu Ala Trp Asp Glu Ile
   1               5                   10                  15
   Asp Arg Leu Pro Asn Leu Thr Ile Glu Gln Trp Leu Ala Phe Ile Ala
               20                  25                  30
   Lys Leu Asp Asp Asp Pro Ser Gln Ser Ser Glu Leu Leu Ser Glu Ala
           35                  40                  45
   Lys Lys Leu Asn Asp Ser Gln Ala Pro Lys Val Asp Gly Ser Leu Ala
       50                  55                  60
   Glu Ala Lys Glu Ala Ala Asn Ala Glu Leu Asp Ser Tyr Gly Val Ser
   65                  70                  75                  80
   Asp Phe Tyr Lys Arg Leu Ile Asp Lys Ala Lys Thr Val Glu Gly Val
                   85                  90                  95
   Glu Ala Leu Lys Asp Ala Ile Leu Ala Ala Leu Pro
               100                 105
<210> 258
   <211> 108
   <212> PRT
   <213> Artificial Sequence
<220>
   <223> Modified bacterial sequence
<400> 258
   Ala Glu Ala Lys Tyr Ala Lys Glu Val Leu Glu Ala Trp Asp Glu Ile
   1               5                   10                  15
   Asp Arg Leu Pro Asn Leu Thr Ile Glu Gln Trp Leu Ala Phe Ile Asn
               20                  25                  30
   Lys Leu Glu Asp Asp Pro Ser Gln Ser Ser Glu Leu Leu Ser Glu Ala
           35                  40                  45
   Lys Lys Leu Asn Asp Ser Gln Ala Pro Lys Val Asp Gly Ser Leu Ala
       50                  55                  60
   Glu Ala Lys Glu Ala Ala Asn Ala Glu Leu Asp Ser Tyr Gly Val Ser
   65                  70                  75                  80
   Asp Phe Tyr Lys Arg Leu Ile Asp Lys Ala Lys Thr Val Glu Gly Val
                   85                  90                  95
   Glu Ala Leu Lys Asp Ala Ile Leu Ala Ala Leu Pro
               100                 105
<210> 259
   <211> 103
   <212> PRT
   <213> Artificial Sequence
<220>
   <223> Modified bacterial sequence
<400> 259
   Ala Glu Ala Lys Tyr Ala Lys Glu Val Leu Glu Ala Trp Asp Glu Ile
   1 5 10 15
   Asp Arg Leu Pro Asn Leu Thr Ile Glu Gln Trp Leu Ala Phe Ile Asn
   20 25 30
   Lys Leu Asp Asp Asp Pro Ser Gln Ser Ser Glu Leu Leu Ser Glu Ala
   35 40 45
   Lys Lys Leu Asn Asp Ser Gln Ala Pro Leu Ala Glu Ala Lys Glu Ala
   50 55 60
   Ala Asn Ala Glu Leu Asp Ser Tyr Gly Val Ser Asp Phe Tyr Lys Arg
   65 70 75 80
   Leu Ile Asp Lys Ala Lys Thr Val Glu Gly Val Glu Ala Leu Lys Asp
   85 90 95
   Ala Ile Leu Ala Ala Leu Pro
   100
<210> 260
   <211> 57
   <212> PRT
   <213> Artificial Sequence
<220>
   <223> Modified bacterial sequence
<400> 260
   Ala Glu Ala Lys Tyr Ala Lys Glu Val Leu Glu Ala Trp Asp Glu Ile
   1 5 10 15
   Asp Arg Leu Pro Asn Leu Thr Ile Glu Gln Trp Leu Ala Phe Ile Asn
   20 25 30
   Lys Leu Asp Asp Asp Pro Ser Gln Ser Ser Glu Leu Leu Ser Glu Ala
   35 40 45
   Lys Lys Leu Ser Glu Ser Gln Ala Pro
   50 55
<210> 261
   <211> 108
   <212> PRT
   <213> Artificial Sequence
<220>
   <223> Modified bacterial sequence
<400> 261
   Ala Glu Ala Lys Tyr Ala Lys Glu Val Leu Glu Ala Trp Asp Glu Ile
   1               5                   10                  15
   Asp Arg Leu Pro Asn Leu Thr Ile Glu Gln Trp Leu Ala Phe Ile Asn
               20                  25                  30
   Lys Leu Asp Asp Asp Pro Ser Gln Ser Ser Glu Leu Leu Ser Glu Ala
           35                  40                  45
   Lys Lys Leu Ser Glu Ser Gln Ala Pro Lys Val Glu Gly Ser Leu Ala
       50                  55                  60
   Glu Ala Lys Glu Ala Ala Asn Ala Glu Leu Asp Ser Tyr Gly Val Ser
   65                  70                  75                  80
   Asp Phe Tyr Lys Arg Leu Ile Asp Lys Ala Lys Thr Val Glu Gly Val
                   85                  90                  95
   Glu Ala Leu Lys Asp Ala Ile Leu Ala Ala Leu Pro
               100                 105
<210> 262
   <211> 108
   <212> PRT
   <213> Artificial Sequence
<220>
   <223> Modified bacterial sequence
<400> 262
   Ala Glu Ala Lys Tyr Ala Lys Glu Val Leu Glu Ala Trp Asp Glu Ile
   1               5                   10                  15
   Asp Arg Leu Pro Asn Leu Thr Ile Glu Gln Trp Leu Ala Phe Ile Asn
   20 25 30
   Lys Leu Asp Asp Asp Pro Ser Gln Ser Ser Glu Leu Leu Ser Glu Ala
   35 40 45
   Lys Lys Leu Ser Glu Ser Gln Ala Pro Lys Val Ala Gly Ser Leu Ala
   50 55 60
   Glu Ala Lys Glu Ala Ala Asn Ala Glu Leu Asp Ser Tyr Gly Val Ser
   65 70 75 80
   Asp Phe Tyr Lys Arg Leu Ile Asp Lys Ala Lys Thr Val Glu Gly Val
   85 90 95
   Glu Ala Leu Lys Asp Ala Ile Leu Ala Ala Leu Pro
   100 105
<210> 263
   <211> 108
   <212> PRT
   <213> Artificial Sequence
<220>
   <223> Modified bacterial sequence
<400> 263
   Ala Glu Ala Lys Tyr Ala Lys Glu Val Leu Glu Ala Trp Asp Glu Ile
   1 5 10 15
   Asp Arg Leu Pro Asn Leu Thr Ile Glu Gln Trp Leu Ala Phe Ile Asn
   20 25 30
   Lys Leu Asp Asp Asp Pro Ser Gln Ser Ser Glu Leu Leu Ser Glu Ala
   35 40 45
   Lys Lys Leu Glu Ser Ser Gln Ala Pro Lys Val Glu Gly Ser Leu Ala
   50 55 60
   Glu Ala Lys Glu Ala Ala Asn Ala Glu Leu Asp Ser Tyr Gly Val Ser
   65 70 75 80
   Asp Phe Tyr Lys Arg Leu Ile Asp Lys Ala Lys Thr Val Glu Gly Val
   85 90 95
   Glu Ala Leu Lys Asp Ala Ile Leu Ala Ala Leu Pro
   100 105
<210> 264
   <211> 108
   <212> PRT
   <213> Artificial Sequence
<220>
   <223> Modified bacterial sequence
<400> 264
   Ala Glu Ala Lys Tyr Ala Lys Glu Val Leu Glu Ala Trp Asp Glu Ile
   1               5                   10                  15
   Asp Arg Leu Pro Asn Leu Thr Ile Glu Gln Trp Leu Ala Phe Ile Asn
               20                  25                  30
   Lys Leu Asp Arg Gln Pro Glu Gln Ser Ser Glu Leu Leu Ser Glu Ala
           35                  40                  45
   Lys Lys Leu Ser Glu Ser Gln Ala Pro Lys Val Glu Gly Ser Leu Ala
       50                  55                  60
   Glu Ala Lys Glu Ala Ala Asn Ala Glu Leu Asp Ser Tyr Gly Val Ser
   65                  70                  75                  80
   Asp Phe Tyr Lys Arg Leu Ile Asp Lys Ala Lys Thr Val Glu Gly Val
                   85                  90                  95
   Glu Ala Leu Lys Asp Ala Ile Leu Ala Ala Leu Pro
               100                 105
<210> 265
   <211> 57
   <212> PRT
   <213> Artificial Sequence
<220>
   <223> Modified bacterial sequence
<400> 265
   Ala Glu Ala Lys Tyr Ala Lys Glu Val Leu Glu Ala Trp Asp Glu Ile
   1               5                   10                  15
   Asp Arg Leu Pro Asn Leu Thr Ile Glu Gln Trp Leu Ala Phe Ile Asn
               20                  25                  30
   Lys Leu Asp Asp Asp Pro Ser Gln Ser Ser Glu Leu Leu Ser Glu Ala
           35                  40                  45
   Lys Lys Leu Glu Ser Ser Gln Ala Pro
       50                  55
<210> 266
   <211> 57
   <212> PRT
   <213> Artificial Sequence
<220>
   <223> Modified bacterial sequence
<400> 266
   Ala Glu Ala Lys Tyr Ala Lys Glu Val Leu Glu Ala Trp Asp Glu Ile
   1               5                   10                  15
   Asp Arg Leu Pro Asn Leu Thr Ile Glu Gln Trp Leu Ala Phe Ile Asn
               20                  25                  30
   Lys Leu Asp Arg Gln Pro Glu Gln Ser Ser Glu Leu Leu Ser Glu Ala
           35                  40                  45
   Lys Lys Leu Ser Glu Ser Gln Ala Pro
       50                  55
<210> 267
   <211> 58
   <212> PRT
   <213> Artificial Sequence
<220>
   <223> Modified bacterial sequence
<400> 267
   Ala Glu Ala Lys Tyr Ala Lys Glu Val Leu Glu Ala Trp Asp Glu Ile
   1               5                   10                  15
   Asp Arg Leu Pro Asn Leu Thr Ile Glu Gln Trp Leu Ala Phe Ile Asn
               20                  25                  30
   Lys Leu Asp Asp Asp Pro Ser Gln Ser Ser Glu Leu Leu Ser Glu Ala
           35                  40                  45
   Lys Lys Leu Ser Glu Ser Gln Ala Pro Lys
       50                  55
<210> 268
   <211> 108
   <212> PRT
   <213> Artificial Sequence
<220>
   <223> Modified bacterial sequence
<400> 268
   Ala Glu Ala Lys Tyr Ala Lys Glu Val Leu Glu Ala Trp Asp Glu Ile
   1               5                   10                  15
   Asp Arg Leu Pro Asn Leu Thr Ile Glu Gln Trp Leu Ala Phe Ile Asn
               20                  25                  30
   Lys Leu Asp Asp Asp Pro Ser Gln Ser Ser Glu Leu Leu Ser Glu Ala
           35                  40                  45
   Lys Lys Leu Asn Asp Ser Gln Ala Pro Lys Val Glu Gly Ser Leu Ala
       50                  55                  60
   Glu Ala Lys Glu Ala Ala Asn Ala Glu Leu Asp Ser Tyr Gly Val Ser
   65                  70                  75                  80
   Asp Phe Tyr Lys Arg Leu Ile Asp Lys Ala Lys Thr Val Glu Gly Val
                   85                  90                  95
   Glu Ala Leu Lys Asp Ala Ile Leu Ala Ala Leu Pro
               100                 105
<210> 269
   <211> 108
   <212> PRT
   <213> Artificial Sequence
<220>
   <223> Modified bacterial sequence
<400> 269
   Ala Glu Ala Lys Tyr Ala Lys Glu Val Leu Glu Ala Trp Asp Glu Ile
   1               5                   10                  15
   Asp Arg Leu Pro Asn Leu Thr Ile Glu Gln Trp Leu Ala Phe Ile Asn
               20                  25                  30
   Lys Leu Asp Asp Asp Pro Ser Gln Ser Ser Glu Leu Leu Ser Glu Ala
           35                  40                  45
   Lys Lys Leu Asn Glu Ser Gln Ala Pro Lys Val Glu Gly Ser Leu Ala
       50                  55                  60
   Glu Ala Lys Glu Ala Ala Asn Ala Glu Leu Asp Ser Tyr Gly Val Ser
   65                  70                  75                  80
   Asp Phe Tyr Lys Arg Leu Ile Asp Lys Ala Lys Thr Val Glu Gly Val
                   85                  90                  95
   Glu Ala Leu Lys Asp Ala Ile Leu Ala Ala Leu Pro
               100                 105
<210> 270
   <211> 108
   <212> PRT
   <213> Artificial Sequence
<220>
   <223> Modified bacterial sequence
<400> 270
   Ala Glu Ala Lys Tyr Ala Lys Glu Val Leu Glu Ala Trp Asp Glu Ile
   1               5                   10                  15
   Asp Arg Leu Pro Asn Leu Thr Ile Glu Gln Trp Leu Ala Phe Ile Asn
               20                  25                  30
   Lys Leu Asp Asp Asp Pro Ser Gln Ser Ser Glu Leu Leu Ser Glu Ala
           35                  40                  45
   Lys Lys Leu Ser Asp Ser Gln Ala Pro Lys Val Glu Gly Ser Leu Ala
       50                  55                  60
   Glu Ala Lys Glu Ala Ala Asn Ala Glu Leu Asp Ser Tyr Gly Val Ser
   65                  70                  75                  80
   Asp Phe Tyr Lys Arg Leu Ile Asp Lys Ala Lys Thr Val Glu Gly Val
                   85                  90                  95
   Glu Ala Leu Lys Asp Ala Ile Leu Ala Ala Leu Pro
               100                 105
<210> 271
   <211> 62
   <212> PRT
   <213> Artificial Sequence
<220>
   <223> Modified bacterial sequence
<400> 271
   Ala Glu Ala Lys Tyr Ala Lys Glu Val Leu Glu Ala Trp Asp Glu Ile
   1               5                   10                  15
   Asp Arg Leu Pro Asn Leu Thr Ile Glu Gln Trp Leu Ala Phe Ile Asn
               20                  25                  30
   Lys Leu Asp Asp Asp Pro Ser Gln Ser Ser Glu Leu Leu Ser Glu Ala
           35                  40                  45
   Lys Lys Leu Asn Asp Ser Gln Ala Pro Lys Val Asp Gly Ser
       50                  55                  60
<210> 272
   <211> 58
   <212> PRT
   <213> Artificial Sequence
<220>
   <223> Modified bacterial sequence
<400> 272
   Ala Glu Ala Lys Tyr Ala Lys Glu Met Arg Asn Ala Tyr Trp Glu Ile
   1               5                   10                  15
   Ala Leu Leu Pro Asn Leu Thr Asn Gln Gln Lys Arg Ala Phe Ile Arg
               20                  25                  30
   Lys Leu Tyr Asp Asp Pro Ser Gln Ser Ser Glu Leu Leu Ser Glu Ala
           35                  40                  45
   Lys Lys Leu Asn Asp Ser Gln Ala Pro Lys
       50                  55
<210> 273
   <211> 58
   <212> PRT
   <213> Artificial Sequence
<220>
   <223> Modified bacterial sequence
<400> 273
   Ala Glu Ala Lys Tyr Ala Lys Glu Met Arg Asn Ala Tyr Trp Glu Ile
   1               5                   10                  15
   Ala Leu Leu Pro Asn Leu Thr Asn Gln Gln Lys Arg Ala Phe Ile Arg
               20                  25                  30
   Lys Leu Tyr Asp Asp Pro Ser Gln Ser Ser Glu Leu Leu Ser Glu Ala
           35                  40                  45
   Lys Lys Leu Ser Glu Ser Gln Ala Pro Lys
       50                  55
<210> 274
   <211> 58
   <212> PRT
   <213> Artificial Sequence
<220>
   <223> Modified bacterial sequence
<400> 274
   Ala Glu Ala Lys Tyr Ala Lys Glu Met Arg Asn Ala Tyr Trp Glu Ile
   1               5                   10                  15
   Ala Leu Leu Pro Asn Leu Thr Asn Gln Gln Lys Arg Ala Phe Ile Arg
               20                  25                  30
   Lys Leu Tyr Arg Gln Pro Glu Gln Ser Ser Glu Leu Leu Ser Glu Ala
           35                  40                  45
   Lys Lys Leu Ser Glu Ser Gln Ala Pro Lys
       50                  55
<210> 275
   <211> 58
   <212> PRT
   <213> Artificial Sequence
<220>
   <223> Modified bacterial sequence
<400> 275
   Ala Glu Ala Lys Tyr Ala Lys Glu Leu Ile Glu Ala Ala Ala Glu Ile
   1               5                   10                  15
   Asp Ala Leu Pro Asn Leu Thr Arg Arg Gln Trp Asn Ala Phe Ile Lys
               20                  25                  30
   Lys Leu Val Asp Asp Pro Ser Gln Ser Ser Glu Leu Leu Ser Glu Ala
           35                  40                  45
   Lys Lys Leu Asn Asp Ser Gln Ala Pro Lys
       50                  55
<210> 276
   <211> 58
   <212> PRT
   <213> Artificial Sequence
<220>
   <223> Modified bacterial sequence
<400> 276
   Ala Glu Ala Lys Tyr Ala Lys Glu Leu Ile Glu Ala Ala Ala Glu Ile
   1               5                   10                  15
   Asp Ala Leu Pro Asn Leu Thr Arg Arg Gln Trp Asn Ala Phe Ile Lys
               20                  25                  30
   Lys Leu Val Asp Asp Pro Ser Gln Ser Ser Glu Leu Leu Ser Glu Ala
   35 40 45
   Lys Lys Leu Ser Glu Ser Gln Ala Pro Lys
   50 55
<210> 277
   <211> 58
   <212> PRT
   <213> Artificial Sequence
<220>
   <223> Modified bacterial sequence
<400> 277
   Ala Glu Ala Lys Tyr Ala Lys Glu Leu Ile Glu Ala Ala Ala Glu Ile
   1 5 10 15
   Asp Ala Leu Pro Asn Leu Thr Arg Arg Gln Trp Asn Ala Phe Ile Lys
   20 25 30
   Lys Leu Val Arg Gln Pro Glu Gln Ser Ser Glu Leu Leu Ser Glu Ala
   35 40 45
   Lys Lys Leu Ser Glu Ser Gln Ala Pro Lys
   50 55
<210> 278
   <211> 58
   <212> PRT
   <213> Artificial Sequence
<220>
   <223> Modified bacterial sequence
<400> 278
   Ala Glu Ala Lys Tyr Ala Lys Glu Gln Asp Ala Ala Ala His Glu Ile
   1 5 10 15
   Arg Trp Leu Pro Asn Leu Thr Phe Asp Gln Arg Val Ala Phe Ile His
   20 25 30
   Lys Leu Ala Asp Asp Pro Ser Gln Ser Ser Glu Leu Leu Ser Glu Ala
   35 40 45
   Lys Lys Leu Asn Asp Ser Gln Ala Pro Lys
   50 55
<210> 279
   <211> 58
   <212> PRT
   <213> Artificial Sequence
<220>
   <223> Modified bacterial sequence
<400> 279
   Ala Glu Ala Lys Tyr Ala Lys Glu Gln Asp Ala Ala Ala His Glu Ile
   1               5                   10                  15
   Arg Trp Leu Pro Asn Leu Thr Phe Asp Gln Arg Val Ala Phe Ile His
               20                  25                  30
   Lys Leu Ala Asp Asp Pro Ser Gln Ser Ser Glu Leu Leu Ser Glu Ala
           35                  40                  45
   Lys Lys Leu Ser Glu Ser Gln Ala Pro Lys
       50                  55
<210> 280
   <211> 58
   <212> PRT
   <213> Artificial Sequence
<220>
   <223> Modified bacterial sequence
<400> 280
   Ala Glu Ala Lys Tyr Ala Lys Glu Gln Asp Ala Ala Ala His Glu Ile
   1               5                   10                  15
   Arg Trp Leu Pro Asn Leu Thr Phe Asp Gln Arg Val Ala Phe Ile His
               20                  25                  30
   Lys Leu Ala Arg Gln Pro Glu Gln Ser Ser Glu Leu Leu Ser Glu Ala
           35                  40                  45
   Lys Lys Leu Ser Glu Ser Gln Ala Pro Lys
       50                  55
<210> 281
   <211> 58
   <212> PRT
   <213> Artificial Sequence
<220>
   <223> Modified bacterial sequence
<400> 281
   Ala Glu Ala Lys Tyr Ala Lys Glu Asn Leu Phe Ala Gly Trp Glu Ile
   1               5                   10                  15
   Ser Asp Leu Pro Asn Leu Thr Asp Tyr Gln Arg Asn Ala Phe Ile Tyr
               20                  25                  30
   Lys Leu Trp Asp Asp Pro Ser Gln Ser Ser Glu Leu Leu Ser Glu Ala
           35                  40                  45
   Lys Lys Leu Asn Asp Ser Gln Ala Pro Lys
       50                  55
<210> 282
   <211> 58
   <212> PRT
   <213> Artificial Sequence
<220>
   <223> Modified bacterial sequence
<400> 282
   Ala Glu Ala Lys Tyr Ala Lys Glu Asn Leu Phe Ala Gly Trp Glu Ile
   1               5                   10                  15
   Ser Asp Leu Pro Asn Leu Thr Asp Tyr Gln Arg Asn Ala Phe Ile Tyr
               20                  25                  30
   Lys Leu Trp Asp Asp Pro Ser Gln Ser Ser Glu Leu Leu Ser Glu Ala
           35                  40                  45
   Lys Lys Leu Ser Glu Ser Gln Ala Pro Lys
       50                  55
<210> 283
   <211> 58
   <212> PRT
   <213> Artificial Sequence
<220>
   <223> Modified bacterial sequence
<400> 283
   Ala Glu Ala Lys Tyr Ala Lys Glu Asn Leu Phe Ala Gly Trp Glu Ile
   1               5                   10                  15
   Ser Asp Leu Pro Asn Leu Thr Asp Tyr Gln Arg Asn Ala Phe Ile Tyr
               20                  25                  30
   Lys Leu Trp Arg Gln Pro Glu Gln Ser Ser Glu Leu Leu Ser Glu Ala
           35                  40                  45
   Lys Lys Leu Ser Glu Ser Gln Ala Pro Lys
       50                  55
<210> 284
   <211> 58
   <212> PRT
   <213> Artificial Sequence
<220>
   <223> Modified bacterial sequence
<400> 284
   Ala Glu Ala Lys Tyr Ala Lys Glu Asn Leu Phe Ala Gly Trp Glu Ile
   1               5                   10                  15
   Ser Asp Leu Pro Asn Leu Thr Asp Tyr Gln Arg Asn Ala Phe Ile Tyr
               20                  25                  30
   Lys Leu Trp Asp Asp Pro Ser Gln Ser Ser Glu Leu Leu Ser Glu Ala
           35                  40                  45
   Lys Lys Leu Asn Glu Ser Gln Ala Pro Lys
       50                  55
<210> 285
   <211> 58
   <212> PRT
   <213> Artificial Sequence
<220>
   <223> Modified bacterial sequence
<400> 285
   Ala Glu Ala Lys Tyr Ala Lys Glu Asn Leu Phe Ala Gly Trp Glu Ile
   1 5 10 15
   Ser Asp Leu Pro Asn Leu Thr Asp Tyr Gln Arg Asn Ala Phe Ile Tyr
   20 25 30
   Lys Leu Trp Arg Gln Pro Glu Gln Ser Ser Glu Leu Leu Ser Glu Ala
   35 40 45
   Lys Lys Leu Asn Glu Ser Gln Ala Pro Lys
   50 55
<210> 286
   <211> 58
   <212> PRT
   <213> Artificial Sequence
<220>
   <223> Modified bacterial sequence
<400> 286
   Ala Glu Ala Lys Tyr Ala Lys Glu Asn Leu Phe Ala Gly Trp Glu Ile
   1 5 10 15
   Ser Asp Leu Pro Asn Leu Thr Asp Tyr Gln Arg Asn Ala Phe Ile Tyr
   20 25 30
   Lys Leu Trp Asp Asp Pro Ser Gln Ser Ser Glu Leu Leu Ser Glu Ala
   35 40 45
   Lys Lys Leu Ser Asp Ser Gln Ala Pro Lys
   50 55
<210> 287
   <211> 58
   <212> PRT
   <213> Artificial Sequence
<220>
   <223> Modified bacterial sequence
<400> 287
   Ala Glu Ala Lys Tyr Ala Lys Glu Asn Leu Phe Ala Gly Trp Glu Ile
   1               5                   10                  15
   Ser Asp Leu Pro Asn Leu Thr Asp Tyr Gln Arg Asn Ala Phe Ile Tyr
               20                  25                  30
   Lys Leu Trp Arg Gln Pro Glu Gln Ser Ser Glu Leu Leu Ser Glu Ala
           35                  40                  45
   Lys Lys Leu Ser Asp Ser Gln Ala Pro Lys
       50                  55